(19) Europäisches Patentamt
European Patent Office
Office européen des brevets

(11) EP 3 557 260 B1

(12) EUROPEAN PATENT SPECIFICATION

(45) Date of publication and mention of the grant of the patent:
**18.05.2022 Bulletin 2022/20**

(21) Application number: **19176006.5**

(22) Date of filing: **24.12.2013**

(51) International Patent Classification (IPC):
***G01N 33/574*** (2006.01) ***A61K 39/395*** (2006.01)
***A61K 45/00*** (2006.01) ***A61P 1/16*** (2006.01)
***A61P 35/00*** (2006.01) ***G01N 33/48*** (2006.01)
***G01N 33/53*** (2006.01) ***C07K 16/30*** (2006.01)

(52) Cooperative Patent Classification (CPC):
**G01N 33/57438; A61P 1/16; A61P 35/00; C07K 16/303;** A61K 2039/505; A61K 2039/54; A61K 2039/545; C07K 2317/73; G01N 2400/00; G01N 2800/52

# (54) GPC3-TARGETING DRUG WHICH IS ADMINISTERED TO PATIENT RESPONSIVE TO GPC3-TARGETING DRUG THERAPY

GPC3-GERICHTETES ARZNEIMITTEL, DAS EINEM PATIENTEN VERABREICHT WIRD, DER AUF DIE GPC3-GERICHTETE ARZNEIMITTELTHERAPIE REAGIERT

MÉDICAMENT DE CIBLAGE GPC3 ADMINISTRÉ À UN PATIENT EN RÉPONSE À UNE THÉRAPIE MÉDICAMENTEUSE DE CIBLAGE GPC3

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2012 JP 2012280304**

(43) Date of publication of application:
**23.10.2019 Bulletin 2019/43**

(60) Divisional application:
**22167137.3**

(62) Document number(s) of the earlier application(s) in accordance with Art. 76 EPC:
**13864465.3 / 2 937 697**

(73) Proprietors:
- **CHUGAI SEIYAKU KABUSHIKI KAISHA**
  **Tokyo 115-8543 (JP)**
- **F. Hoffmann-La Roche AG**
  **4070 Basel (CH)**

(72) Inventors:
- **OHTOMO, Toshihiko**
  **Chuo-ku, Tokyo 103-8324 (JP)**
- **AMANO, Jun**
  **Gotemba-shi, Shizuoka 412-8513 (JP)**
- **NAKAMURA, Mikiko**
  **Chuo-ku, Tokyo 103-8324 (JP)**
- **CHEN, Ya-Chi**
  **Hoboken, New Jersey 07030 (US)**

(74) Representative: **J A Kemp LLP**
**80 Turnmill Street**
**London EC1M 5QU (GB)**

(56) References cited:
**WO-A1-2012/145469 JP-A- 2007 093 274**
**US-A1- 2008 138 827**

- **MITCHELL HO ET AL: "Glypican-3: A new target for cancer immunotherapy", EUROPEAN JOURNAL OF CANCER, PERGAMON, GB, vol. 47, no. 3, 27 October 2010 (2010-10-27), pages 333-338, XP028129160, ISSN: 0959-8049, DOI: 10.1016/J.EJCA.2010.10.024 [retrieved on 2010-11-01]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

[Sequence Listing]

SEQUENCE LISTING

<110> CHUGAI SEIYAKU KABUSHIKI KAISHA
<120> GPC3-targetted therapeutic agent to be administered to patients who is responsible to GPC3-targetted therapy
<130> PCG-9038WO
<150> JP2012-280304
<151> 2012-12-21
<160> 90
<170> PatentIn version 3.1
<210> 1
<211> 580
<212> PRT
<213> homo sapiens
<400> 1

```
Met Ala Gly Thr Val Arg Thr Ala Cys Leu Val Val Ala Met Leu Leu
1               5                   10                  15
Ser Leu Asp Phe Pro Gly Gln Ala Gln Pro Pro Pro Pro Pro Pro Asp
            20                  25                  30
Ala Thr Cys His Gln Val Arg Ser Phe Phe Gln Arg Leu Gln Pro Gly
        35                  40                  45
Leu Lys Trp Val Pro Glu Thr Pro Val Pro Gly Ser Asp Leu Gln Val
    50                  55                  60
Cys Leu Pro Lys Gly Pro Thr Cys Cys Ser Arg Lys Met Glu Glu Lys
65                  70                  75                  80
Tyr Gln Leu Thr Ala Arg Leu Asn Met Glu Gln Leu Leu Gln Ser Ala
                85                  90                  95
Ser Met Glu Leu Lys Phe Leu Ile Ile Gln Asn Ala Ala Val Phe Gln
            100                 105                 110
Glu Ala Phe Glu Ile Val Val Arg His Ala Lys Asn Tyr Thr Asn Ala
        115                 120                 125
Met Phe Lys Asn Asn Tyr Pro Ser Leu Thr Pro Gln Ala Phe Glu Phe
    130                 135                 140
Val Gly Glu Phe Phe Thr Asp Val Ser Leu Tyr Ile Leu Gly Ser Asp
145                 150                 155                 160
Ile Asn Val Asp Asp Met Val Asn Glu Leu Phe Asp Ser Leu Phe Pro
                165                 170                 175
Val Ile Tyr Thr Gln Leu Met Asn Pro Gly Leu Pro Asp Ser Ala Leu
            180                 185                 190
Asp Ile Asn Glu Cys Leu Arg Gly Ala Arg Arg Asp Leu Lys Val Phe
        195                 200                 205
Gly Asn Phe Pro Lys Leu Ile Met Thr Gln Val Ser Lys Ser Leu Gln
    210                 215                 220
Val Thr Arg Ile Phe Leu Gln Ala Leu Asn Leu Gly Ile Glu Val Ile
225                 230                 235                 240
Asn Thr Thr Asp His Leu Lys Phe Ser Lys Asp Cys Gly Arg Met Leu
                245                 250                 255
Thr Arg Met Trp Tyr Cys Ser Tyr Cys Gln Gly Leu Met Met Val Lys
            260                 265                 270
Pro Cys Gly Gly Tyr Cys Asn Val Val Met Gln Gly Cys Met Ala Gly
        275                 280                 285
Val Val Glu Ile Asp Lys Tyr Trp Arg Glu Tyr Ile Leu Ser Leu Glu
    290                 295                 300
Glu Leu Val Asn Gly Met Tyr Arg Ile Tyr Asp Met Glu Asn Val Leu
305                 310                 315                 320
Leu Gly Leu Phe Ser Thr Ile His Asp Ser Ile Gln Tyr Val Gln Lys
                325                 330                 335
Asn Ala Gly Lys Leu Thr Thr Thr Ile Gly Lys Leu Cys Ala His Ser
            340                 345                 350
Gln Gln Arg Gln Tyr Arg Ser Ala Tyr Tyr Pro Glu Asp Leu Phe Ile
        355                 360                 365
Asp Lys Lys Val Leu Lys Val Ala His Val Glu His Glu Glu Thr Leu
    370                 375                 380
Ser Ser Arg Arg Arg Glu Leu Ile Gln Lys Leu Lys Ser Phe Ile Ser
```

```
385                 390                 395                 400
Phe Tyr Ser Ala Leu Pro Gly Tyr Ile Cys Ser His Ser Pro Val Ala
                405                 410                 415
Glu Asn Asp Thr Leu Cys Trp Asn Gly Gln Glu Leu Val Glu Arg Tyr
            420                 425                 430
Ser Gln Lys Ala Ala Arg Asn Gly Met Lys Asn Gln Phe Asn Leu His
        435                 440                 445
Glu Leu Lys Met Lys Gly Pro Glu Pro Val Val Ser Gln Ile Ile Asp
    450                 455                 460
Lys Leu Lys His Ile Asn Gln Leu Leu Arg Thr Met Ser Met Pro Lys
465                 470                 475                 480
Gly Arg Val Leu Asp Lys Asn Leu Asp Glu Glu Gly Phe Glu Ser Gly
                485                 490                 495
Asp Cys Gly Asp Asp Glu Asp Glu Cys Ile Gly Gly Ser Gly Asp Gly
            500                 505                 510
Met Ile Lys Val Lys Asn Gln Leu Arg Phe Leu Ala Glu Leu Ala Tyr
        515                 520                 525
Asp Leu Asp Val Asp Asp Ala Pro Gly Asn Ser Gln Gln Ala Thr Pro
    530                 535                 540
Lys Asp Asn Glu Ile Ser Thr Phe His Asn Leu Gly Asn Val His Ser
545                 550                 555                 560
Pro Leu Lys Leu Leu Thr Ser Met Ala Ile Ser Val Val Cys Phe Phe
                565                 570                 575
Phe Leu Val His
            580
```

<210> 2
<211> 9
<212> PRT
<213> homo sapiens
<400> 2

```
Phe Val Gly Glu Phe Phe Thr Asp Val
1               5
```

<210> 3
<211> 9
<212> PRT
<213> homo sapiens
<400> 3

```
Glu Tyr Ile Leu Ser Leu Glu Glu Leu
1               5
```

<210> 4
<211> 5
<212> PRT
<213> Mus musculus
<400> 4

```
Asp Tyr Ser Met His
1               5
```

<210> 5
<211> 17
<212> PRT
<213> Mus musculus
<400> 5

Trp Ile Asn Thr Glu Thr Gly Glu Pro Thr Tyr Ala Asp Asp Phe Lys
1 5 10 15
Gly

<210> 6
<211> 2
<212> PRT
<213> Mus musculus
<400> 6

Leu Tyr
1

<210> 7
<211> 16
<212> PRT
<213> Mus musculus
<400> 7

Lys Ser Ser Gln Ser Leu Leu His Ser Asp Gly Lys Thr Phe Leu Asn
1 5 10 15

<210> 8
<211> 7
<212> PRT
<213> Mus musculus
<400> 8

Leu Val Ser Arg Leu Asp Ser
1 5

<210> 9
<211> 9
<212> PRT
<213> Mus musculus
<400> 9

Cys Gln Gly Thr His Phe Pro Arg Thr
1 5

<210> 10
<211> 111
<212> PRT
<213> Mus musculus
<400> 10

```
Gln Ile Gln Leu Glu Gln Ser Gly Pro Glu Leu Lys Lys Pro Gly Glu
1               5                   10                  15
Thr Val Lys Ile Ser Cys Lys Ala Ser Gly Tyr Ile Phe Arg Asp Tyr
            20                  25                  30
Ser Met His Trp Val Lys Gln Ala Pro Gly Lys Gly Leu Lys Trp Met
        35                  40                  45
Gly Trp Ile Asn Thr Glu Thr Gly Glu Pro Thr Tyr Ala Asp Asp Phe
    50                  55                  60
Lys Gly Arg Phe Ala Phe Ser Leu Glu Thr Ser Ala Ser Thr Ala Tyr
65                  70                  75                  80
Leu Gln Ile Asn Asn Leu Lys Asn Glu Asp Thr Ala Thr Tyr Phe Cys
                85                  90                  95
Thr Ser Leu Tyr Trp Gly Gln Gly Thr Leu Val Thr Val Ser Ala
            100                 105                 110
```

<210> 11
<211> 112
<212> PRT
<213> Mus musculus
<400> 11

```
Asp Val Val Met Thr Gln Thr Pro Leu Thr Leu Ser Val Thr Leu Gly
1               5                   10                  15
Gln Pro Ala Ser Ile Ser Cys Lys Ser Ser Gln Ser Leu Leu His Ser
            20                  25                  30
Asp Gly Lys Thr Phe Leu Asn Trp Leu Leu Gln Arg Pro Gly Gln Ser
        35                  40                  45
Pro Lys Arg Leu Ile Tyr Leu Val Ser Arg Leu Asp Ser Gly Val Pro
    50                  55                  60
Asp Arg Phe Thr Gly Ser Gly Ser Gly Thr Asp Phe Thr Leu Lys Ile
65                  70                  75                  80
Ser Arg Val Glu Ala Glu Asp Leu Gly Val Tyr Tyr Cys Cys Gln Gly
                85                  90                  95
Thr His Phe Pro Arg Thr Phe Gly Gly Gly Thr Arg Leu Glu Ile Lys
            100                 105                 110
```

<210> 12
<211> 7
<212> PRT
<213> Mus musculus
<400> 12

```
Thr Tyr Gly Met Gly Val Gly
1               5
```

<210> 13
<211> 16
<212> PRT
<213> Mus musculus
<400> 13

```
Asn Ile Trp Trp His Asp Asp Lys Tyr Tyr Asn Ser Ala Leu Lys Ser
1               5                   10                  15
```

<400> 14
<211> 14
<212> PRT
<213> Mus musculus

<400> 14

Ile Ala Pro Arg Tyr Asn Lys Tyr Glu Gly Phe Phe Ala Phe
1 5 10

<210> 15
<211> 16
<212> PRT
<213> Mus musculus
<400> 15

Arg Ser Ser Gln Ser Ile Val His Ser Asn Gly Asn Thr Tyr Leu Glu
1 5 10 15

<210> 16
<211> 7
<212> PRT
<213> Mus musculus
<400> 16

Lys Val Ser Asn Arg Phe Ser
1 5

<210> 17
<211> 9
<212> PRT
<213> Mus musculus
<400> 17

Phe Gln Gly Ser His Val Pro Trp Thr
1 5

<210> 18
<211> 124
<212> PRT
<213> Mus musculus
<400> 18

Gln Val Thr Leu Lys Glu Ser Gly Pro Gly Ile Leu Gln Pro Ser Gln
1 5 10 15
Thr Leu Ser Leu Thr Cys Ser Phe Ser Gly Phe Ser Leu Ser Thr Tyr
20 25 30
Gly Met Gly Val Gly Trp Ile Arg Gln Pro Ser Gly Lys Gly Leu Glu
35 40 45
Trp Leu Ala Asn Ile Trp Trp His Asp Asp Lys Tyr Tyr Asn Ser Ala
50 55 60
Leu Lys Ser Arg Leu Thr Ile Ser Lys Asp Ile Ser Asn Asn Gln Val
65 70 75 80
Phe Leu Lys Ile Ser Ser Val Asp Thr Ala Asp Thr Ala Thr Tyr Tyr
85 90 95
Cys Ala Gln Ile Ala Pro Arg Tyr Asn Lys Tyr Glu Gly Phe Phe Ala
100 105 110
Phe Trp Gly Gln Gly Thr Leu Val Thr Val Ser Ala
115 120

<210> 19
<211> 112
<212> PRT
<213> Mus musculus

<400> 19

Asp Val Leu Met Thr Gln Thr Pro Leu Ser Leu Pro Val Ser Leu Gly
1               5                   10                  15
Asp Gln Ala Ser Ile Ser Cys Arg Ser Ser Gln Ser Ile Val His Ser
            20                  25                  30
Asn Gly Asn Thr Tyr Leu Glu Trp Tyr Leu Gln Lys Pro Gly Gln Ser
        35                  40                  45
Pro Lys Leu Leu Ile Tyr Lys Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Leu Gly Val Tyr Tyr Cys Phe Gln Gly
                85                  90                  95
Ser His Val Pro Trp Thr Phe Gly Gly Gly Thr Lys Leu Glu Ile Lys
            100                 105                 110

<210> 20
<211> 5
<212> PRT
<213> Mus musculus
<400> 20

Asp Tyr Glu Met His
1               5

<210> 21
<211> 17
<212> PRT
<213> Mus musculus
<400> 21

Ala Leu Asp Pro Lys Thr Gly Asp Thr Ala Tyr Ser Gln Lys Phe Lys
1               5                   10                  15
Gly

<210> 22
<211> 6
<212> PRT
<213> Mus musculus
<400> 22

Phe Tyr Ser Tyr Thr Tyr
1               5

<210> 23
<211> 16
<212> PRT
<213> Mus musculus
<400> 23

Arg Ser Ser Gln Ser Leu Val His Ser Asn Gly Asn Thr Tyr Leu His
1               5                   10                  15

<210> 24
<211> 7
<212> PRT

<213> Mus musculus
<400> 24

```
Lys Val Ser Asn Arg Phe Ser
1               5
```

<210> 25
<211> 9
<212> PRT
<213> Mus musculus
<400> 25

```
Ser Gln Asn Thr His Val Pro Pro Thr
1               5
```

<210> 26
<211> 115
<212> PRT
<213> Mus musculus
<400> 26

```
Gln Val Gln Leu Gln Gln Ser Gly Ala Glu Leu Val Arg Pro Gly Ala
1               5                   10                  15
Ser Val Lys Leu Ser Cys Lys Ala Ser Gly Tyr Thr Phe Thr Asp Tyr
            20                  25                  30
Glu Met His Trp Val Lys Gln Thr Pro Val His Gly Leu Lys Trp Ile
        35                  40                  45
Gly Ala Leu Asp Pro Lys Thr Gly Asp Thr Ala Tyr Ser Gln Lys Phe
    50                  55                  60
Lys Gly Lys Ala Thr Leu Thr Ala Asp Lys Ser Ser Ser Thr Ala Tyr
65                  70                  75                  80
Met Glu Leu Arg Ser Leu Thr Ser Glu Asp Ser Ala Val Tyr Tyr Cys
                85                  90                  95
Thr Arg Phe Tyr Ser Tyr Thr Tyr Trp Gly Gln Gly Thr Leu Val Thr
            100                 105                 110
Val Ser Ala
        115
```

<210> 27
<211> 112
<212> PRT
<213> Mus musculus
<400> 27

```
Asp Val Val Met Thr Gln Thr Pro Leu Ser Leu Pro Val Ser Leu Gly
1               5                   10                  15
Asp Gln Ala Ser Ile Ser Cys Arg Ser Ser Gln Ser Leu Val His Ser
            20                  25                  30
Asn Gly Asn Thr Tyr Leu His Trp Tyr Leu Gln Lys Pro Gly Gln Ser
        35                  40                  45
Pro Lys Leu Leu Ile Tyr Lys Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Leu Gly Val Tyr Phe Cys Ser Gln Asn
                85                  90                  95
Thr His Val Pro Pro Thr Phe Gly Ser Gly Thr Lys Leu Glu Ile Lys
            100                 105                 110
```

<210> 28
<211> 5
<212> PRT
<213> Mus musculus
<400> 28

Ile Asn Ala Met Asn
1               5

<210> 29
<211> 19
<212> PRT
<213> Mus musculus
<400> 29

Arg Ile Arg Ser Glu Ser Asn Asn Tyr Ala Thr Tyr Tyr Gly Asp Ser
1               5                   10                  15
Val Lys Asp

<210> 30
<211> 8
<212> PRT
<213> Mus musculus
<400> 30

Glu Val Thr Thr Ser Phe Ala Tyr
1               5

<210> 31
<211> 16
<212> PRT
<213> Mus musculus
<400> 31

Lys Ser Ser Lys Ser Leu Leu His Ser Asn Gly Asn Thr Tyr Leu Asn
1               5                   10                  15

<210> 32
<211> 7
<212> PRT
<213> Mus musculus
<400> 32

Trp Met Ser Asn Leu Ala Ser
1               5

<210> 33
<211> 9
<212> PRT
<213> Mus musculus
<400> 33

Met Gln His Ile Glu Tyr Pro Phe Thr
1               5

<210> 34
<211> 119

<212> PRT
<213> Mus musculus
<400> 34

```
Glu Val Gln Leu Val Glu Thr Gly Gly Gly Leu Val Gln Pro Glu Gly
1               5                   10                  15
Ser Leu Lys Leu Ser Cys Ala Ala Ser Gly Phe Ser Phe Asn Ile Asn
            20                  25                  30
Ala Met Asn Trp Val Arg Gln Ala Pro Gly Lys Gly Leu Glu Trp Val
        35                  40                  45
Ala Arg Ile Arg Ser Glu Ser Asn Asn Tyr Ala Thr Tyr Tyr Gly Asp
    50                  55                  60
Ser Val Lys Asp Arg Phe Thr Ile Ser Arg Asp Asp Ser Gln Asn Met
65                  70                  75                  80
Leu Tyr Leu Gln Met Asn Asn Leu Lys Thr Glu Asp Thr Ala Ile Tyr
                85                  90                  95
Tyr Cys Val Arg Glu Val Thr Thr Ser Phe Ala Tyr Trp Gly Gln Gly
            100                 105                 110
Thr Leu Val Thr Val Ser Ala
        115
```

<210> 35
<211> 112
<212> PRT
<213> Mus musculus
<400> 35

```
Asp Ile Val Met Thr Gln Ser Ala Pro Ser Val Pro Val Thr Pro Gly
1               5                   10                  15
Glu Ser Val Ser Ile Ser Cys Lys Ser Ser Lys Ser Leu Leu His Ser
            20                  25                  30
Asn Gly Asn Thr Tyr Leu Asn Trp Phe Leu Gln Arg Pro Gly Gln Ser
        35                  40                  45
Pro Gln Leu Leu Ile Tyr Trp Met Ser Asn Leu Ala Ser Gly Val Pro
    50                  55                  60
Asp Arg Phe Ser Gly Ser Gly Ser Gly Thr Ala Phe Thr Leu Arg Ile
65                  70                  75                  80
Ser Arg Val Glu Ala Glu Asp Val Gly Val Tyr Tyr Cys Met Gln His
                85                  90                  95
Ile Glu Tyr Pro Phe Thr Phe Gly Thr Gly Thr Lys Leu Glu Ile Lys
            100                 105                 110
```

<210> 36
<211> 5
<212> PRT
<213> Mus musculus
<400> 36

```
Ala Ser Ala Met Asn
1               5
```

<210> 37
<211> 19
<212> PRT
<213> Mus musculus
<400> 37

Arg Ile Arg Ser Lys Ser Asn Asn Tyr Ala Ile Tyr Tyr Ala Asp Ser
1 5 10 15
Val Lys Asp

<210> 38
<211> 12
<212> PRT
<213> Mus musculus
<400> 38

Asp Pro Gly Tyr Tyr Gly Asn Pro Trp Phe Ala Tyr
1 5 10

<210> 39
<211> 16
<212> PRT
<213> Mus musculus
<400> 39

Arg Ser Ser Lys Ser Leu Leu His Ser Tyr Asp Ile Thr Tyr Leu Tyr
1 5 10 15

<210> 40
<211> 7
<212> PRT
<213> Mus musculus
<400> 40

Gln Met Ser Asn Leu Ala Ser
1 5

<210> 41
<211> 9
<212> PRT
<213> Mus musculus
<400> 41

Ala Gln Asn Leu Glu Leu Pro Pro Thr
1 5

<210> 42
<211> 123
<212> PRT
<213> Mus musculus
<400> 42

```
Glu Val Gln Leu Val Glu Thr Gly Gly Gly Leu Val Gln Pro Lys Gly
1               5                   10                  15
Ser Leu Lys Leu Ser Cys Ala Ala Ser Gly Phe Thr Phe Asn Ala Ser
            20                  25                  30
Ala Met Asn Trp Val Arg Gln Ala Pro Gly Lys Gly Leu Glu Trp Val
        35                  40                  45
Ala Arg Ile Arg Ser Lys Ser Asn Asn Tyr Ala Ile Tyr Tyr Ala Asp
    50                  55                  60
Ser Val Lys Asp Arg Phe Thr Ile Ser Arg Asp Asp Ser Gln Ser Met
65                  70                  75                  80
Leu Tyr Leu Gln Met Asn Asn Leu Lys Thr Glu Asp Thr Ala Met Tyr
                85                  90                  95
Tyr Cys Val Arg Asp Pro Gly Tyr Tyr Gly Asn Pro Trp Phe Ala Tyr
            100                 105                 110
Trp Gly Gln Gly Thr Leu Val Thr Val Ser Ala
        115                 120
```

<210> 43
<211> 112
<212> PRT
<213> Mus musculus
<400> 43

```
Asp Ile Val Met Thr Gln Ala Ala Phe Ser Asn Pro Val Thr Leu Gly
1               5                   10                  15
Thr Ser Ala Ser Ile Ser Cys Arg Ser Ser Lys Ser Leu Leu His Ser
            20                  25                  30
Tyr Asp Ile Thr Tyr Leu Tyr Trp Tyr Leu Gln Lys Pro Gly Gln Ser
        35                  40                  45
Pro Gln Leu Leu Ile Tyr Gln Met Ser Asn Leu Ala Ser Gly Val Pro
    50                  55                  60
Asp Arg Phe Ser Ser Ser Gly Ser Gly Thr Asp Phe Thr Leu Arg Ile
65                  70                  75                  80
Ser Arg Val Glu Ala Glu Asp Val Gly Val Tyr Tyr Cys Ala Gln Asn
                85                  90                  95
Leu Glu Leu Pro Pro Thr Phe Gly Gly Gly Thr Lys Leu Glu Ile Lys
            100                 105                 110
```

<210> 44
<211> 115
<212> PRT
<213> Artificial Sequence
<220>
<223> modified antibody fragment
<400> 44

Gln Val Gln Leu Val Glu Ser Gly Ala Glu Val Lys Lys Pro Gly Ala
1 5 10 15
Ser Val Lys Val Ser Cys Lys Ala Ser Gly Tyr Thr Phe Thr Asp Tyr
20 25 30
Glu Met His Trp Val Arg Gln Ala Pro Gly Gln Gly Leu Glu Trp Met
35 40 45
Gly Ala Leu Asp Pro Lys Thr Gly Asp Thr Ala Tyr Ser Gln Lys Phe
50 55 60
Lys Gly Arg Val Thr Ile Thr Ala Asp Glu Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Phe Tyr Ser Tyr Thr Tyr Trp Gly Gln Gly Thr Leu Val Thr
100 105 110
Val Ser Ser
115

<210> 45
<211> 115
<212> PRT
<213> Artificial Sequence
<220>
<223> modified antibody fragment
<400> 45

Gln Val Gln Leu Val Glu Ser Gly Ala Glu Val Lys Lys Pro Gly Ala
1 5 10 15
Ser Val Lys Val Ser Cys Lys Ala Ser Gly Tyr Thr Phe Thr Asp Tyr
20 25 30
Glu Met His Trp Val Arg Gln Ala Pro Gly Gln Gly Leu Glu Trp Met
35 40 45
Gly Ala Leu Asp Pro Lys Thr Gly Asp Thr Ala Tyr Ser Gln Lys Phe
50 55 60
Lys Gly Arg Val Thr Leu Thr Ala Asp Glu Ser Thr Ser Thr Ala Tyr
65 70 75 80
Met Glu Leu Ser Ser Leu Arg Ser Glu Asp Thr Ala Val Tyr Tyr Cys
85 90 95
Thr Arg Phe Tyr Ser Tyr Thr Tyr Trp Gly Gln Gly Thr Leu Val Thr
100 105 110
Val Ser Ser
115

<210> 46
<211> 115
<212> PRT
<213> Artificial Sequence
<220>
<223> modified antibody fragment
<400> 46

```
Gln Val Gln Leu Val Glu Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Glu Met His Trp Val Arg Gln Ala Pro Gly Gln Gly Leu Glu Trp Met
        35                  40                  45
Gly Ala Leu Asp Pro Lys Thr Gly Asp Thr Ala Tyr Ser Gln Lys Phe
    50                  55                  60
Lys Gly Arg Val Thr Leu Thr Ala Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Thr Arg Phe Tyr Ser Tyr Thr Tyr Trp Gly Gln Gly Thr Leu Val Thr
            100                 105                 110
Val Ser Ser
        115
```

<210> 47
<211> 115
<212> PRT
<213> Artificial Sequence
<220>
<223> modified antibody fragment
<400> 47

```
Gln Val Gln Leu Val Glu Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Glu Met His Trp Val Arg Gln Ala Pro Gly Gln Gly Leu Glu Trp Met
        35                  40                  45
Gly Ala Leu Asp Pro Lys Thr Gly Asp Thr Ala Tyr Ser Gln Lys Phe
    50                  55                  60
Lys Gly Arg Val Thr Leu Thr Ala Asp Lys Ser Thr Ser Thr Ala Tyr
65                  70                  75                  80
Met Glu Leu Ser Ser Leu Thr Ser Glu Asp Thr Ala Val Tyr Tyr Cys
                85                  90                  95
Thr Arg Phe Tyr Ser Tyr Thr Tyr Trp Gly Gln Gly Thr Leu Val Thr
            100                 105                 110
Val Ser Ser
        115
```

<210> 48
<211> 115
<212> PRT
<213> Artificial Sequence
<220>
<223> modified antibody fragment
<400> 48

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Glu Met His Trp Val Arg Gln Ala Pro Gly Gln Gly Leu Glu Trp Met
        35                  40                  45
Gly Ala Leu Asp Pro Lys Thr Gly Asp Thr Ala Tyr Ser Gln Lys Phe
    50                  55                  60
Lys Gly Arg Val Thr Leu Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Thr Arg Phe Tyr Ser Tyr Thr Tyr Trp Gly Gln Gly Thr Leu Val Thr


                100                 105                 110
    Val Ser Ser
            115
```

<210> 49
<211> 115
<212> PRT
<213> Artificial Sequence
<220>
<223> modified antibody fragment
<400> 49

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Glu Met His Trp Val Arg Gln Ala Pro Gly Gln Gly Leu Glu Trp Met
        35                  40                  45
Gly Ala Leu Asp Pro Lys Thr Gly Asp Thr Ala Tyr Ser Gln Lys Phe
    50                  55                  60
Lys Gly Arg Val Thr Leu Thr Ala Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Thr Arg Phe Tyr Ser Tyr Thr Tyr Trp Gly Gln Gly Thr Leu Val Thr
            100                 105                 110
Val Ser Ser
        115
```

<210> 50
<211> 115
<212> PRT
<213> Artificial Sequence
<220>
<223> modified antibody fragment
<400> 50

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Glu Met His Trp Val Arg Gln Ala Pro Gly Gln Gly Leu Glu Trp Met
        35                  40                  45
Gly Ala Leu Asp Pro Lys Thr Gly Asp Thr Ala Tyr Ser Gln Lys Phe
    50                  55                  60
Lys Gly Arg Val Thr Leu Thr Ala Asp Lys Ser Thr Ser Thr Ala Tyr
65                  70                  75                  80
Met Glu Leu Ser Ser Leu Thr Ser Glu Asp Thr Ala Val Tyr Tyr Cys
                85                  90                  95
Thr Arg Phe Tyr Ser Tyr Thr Tyr Trp Gly Gln Gly Thr Leu Val Thr
            100                 105                 110
Val Ser Ser
        115
```

<210> 51
<211> 112
<212> PRT
<213> Artificial Sequence
<220>
<223> modified antibody fragment
<400> 51

```
Asp Val Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Val His Ser
            20                  25                  30
Asn Gly Asn Thr Tyr Leu His Trp Tyr Leu Gln Lys Pro Gly Gln Ser
        35                  40                  45
Pro Gln Leu Leu Ile Tyr Lys Val Ser Asn Arg Phe Ser Gly Val Pro


    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Asn
                85                  90                  95
Thr His Val Pro Pro Thr Phe Gly Gln Gly Thr Lys Leu Glu Ile Lys
            100                 105                 110
```

<210> 52
<211> 112
<212> PRT
<213> Artificial Sequence
<220>
<223> modified antibody fragment
<400> 52

```
Asp Val Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Val His Ser
            20                  25                  30
Asn Ala Asn Thr Tyr Leu His Trp Tyr Leu Gln Lys Pro Gly Gln Ser
        35                  40                  45
Pro Gln Leu Leu Ile Tyr Lys Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Asn
                85                  90                  95
Thr His Val Pro Pro Thr Phe Gly Gln Gly Thr Lys Leu Glu Ile Lys
            100                 105                 110
```

<210> 53
<211> 112
<212> PRT
<213> Artificial Sequence
<220>
<223> modified antibody fragment
<400> 53

```
Asp Val Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Val His Ser
            20                  25                  30
Asn Asp Asn Thr Tyr Leu His Trp Tyr Leu Gln Lys Pro Gly Gln Ser
        35                  40                  45
Pro Gln Leu Leu Ile Tyr Lys Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Asn
                85                  90                  95
Thr His Val Pro Pro Thr Phe Gly Gln Gly Thr Lys Leu Glu Ile Lys
            100                 105                 110
```

<210> 54
<211> 112
<212> PRT
<213> Artificial Sequence
<220>
<223> modified antibody fragment
<400> 54

```
Asp Val Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Val His Ser
            20                  25                  30
Asn Glu Asn Thr Tyr Leu His Trp Tyr Leu Gln Lys Pro Gly Gln Ser
        35                  40                  45
Pro Gln Leu Leu Ile Tyr Lys Val Ser Asn Arg Phe Ser Gly Val Pro
```

```
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Asn
                85                  90                  95
Thr His Val Pro Pro Thr Phe Gly Gln Gly Thr Lys Leu Glu Ile Lys
            100                 105                 110
```

<210> 55
<211> 112
<212> PRT
<213> Artificial Sequence
<220>
<223> modified antibody fragment
<400> 55

```
Asp Val Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Val His Ser
            20                  25                  30
Asn Phe Asn Thr Tyr Leu His Trp Tyr Leu Gln Lys Pro Gly Gln Ser
        35                  40                  45
Pro Gln Leu Leu Ile Tyr Lys Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Asn
                85                  90                  95
Thr His Val Pro Pro Thr Phe Gly Gln Gly Thr Lys Leu Glu Ile Lys
            100                 105                 110
```

<210> 56
<211> 112
<212> PRT
<213> Artificial Sequence
<220>
<223> modified antibody fragment
<400> 56

```
Asp Val Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Val His Ser
            20                  25                  30
Asn His Asn Thr Tyr Leu His Trp Tyr Leu Gln Lys Pro Gly Gln Ser
        35                  40                  45
Pro Gln Leu Leu Ile Tyr Lys Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Asn
                85                  90                  95
Thr His Val Pro Pro Thr Phe Gly Gln Gly Thr Lys Leu Glu Ile Lys
            100                 105                 110
```

<210> 57
<211> 112
<212> PRT
<213> Artificial Sequence

<220>
<223> modified antibody fragment
<400> 57

```
Asp Val Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Val His Ser
            20                  25                  30
Asn Asn Asn Thr Tyr Leu His Trp Tyr Leu Gln Lys Pro Gly Gln Ser
        35                  40                  45
Pro Gln Leu Leu Ile Tyr Lys Val Ser Asn Arg Phe Ser Gly Val Pro


    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Asn
                85                  90                  95
Thr His Val Pro Pro Thr Phe Gly Gln Gly Thr Lys Leu Glu Ile Lys
            100                 105                 110
```

<210> 58
<211> 112
<212> PRT
<213> Artificial Sequence
<220>
<223> modified antibody fragment
<400> 58

```
Asp Val Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Val His Ser
            20                  25                  30
Asn Thr Asn Thr Tyr Leu His Trp Tyr Leu Gln Lys Pro Gly Gln Ser
        35                  40                  45
Pro Gln Leu Leu Ile Tyr Lys Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Asn
                85                  90                  95
Thr His Val Pro Pro Thr Phe Gly Gln Gly Thr Lys Leu Glu Ile Lys
            100                 105                 110
```

<210> 59
<211> 112
<212> PRT
<213> Artificial Sequence
<220>
<223> modified antibody fragment
<400> 59

```
Asp Val Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Val His Ser
            20                  25                  30
Asn Gln Asn Thr Tyr Leu His Trp Tyr Leu Gln Lys Pro Gly Gln Ser
        35                  40                  45
Pro Gln Leu Leu Ile Tyr Lys Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Asn
                85                  90                  95
Thr His Val Pro Pro Thr Phe Gly Gln Gly Thr Lys Leu Glu Ile Lys
            100                 105                 110
```

<210> 60
<211> 112
<212> PRT
<213> Artificial Sequence
<220>
<223> modified antibody fragment
<400> 60

```
Asp Val Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Val His Ser
            20                  25                  30
Asn Ile Asn Thr Tyr Leu His Trp Tyr Leu Gln Lys Pro Gly Gln Ser
        35                  40                  45
Pro Gln Leu Leu Ile Tyr Lys Val Ser Asn Arg Phe Ser Gly Val Pro

    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Asn
                85                  90                  95
Thr His Val Pro Pro Thr Phe Gly Gln Gly Thr Lys Leu Glu Ile Lys
            100                 105                 110
```

<210> 61
<211> 112
<212> PRT
<213> Artificial Sequence
<220>
<223> modified antibody fragment
<400> 61

```
Asp Val Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Val His Ser
            20                  25                  30
Asn Lys Asn Thr Tyr Leu His Trp Tyr Leu Gln Lys Pro Gly Gln Ser
        35                  40                  45
Pro Gln Leu Leu Ile Tyr Lys Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Asn
                85                  90                  95
Thr His Val Pro Pro Thr Phe Gly Gln Gly Thr Lys Leu Glu Ile Lys
            100                 105                 110
```

<210> 62
<211> 112
<212> PRT
<213> Artificial Sequence
<220>
<223> modified antibody fragment
<400> 62

```
Asp Val Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Val His Ser
            20                  25                  30
Asn Leu Asn Thr Tyr Leu His Trp Tyr Leu Gln Lys Pro Gly Gln Ser
        35                  40                  45
Pro Gln Leu Leu Ile Tyr Lys Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Asn
                85                  90                  95
Thr His Val Pro Pro Thr Phe Gly Gln Gly Thr Lys Leu Glu Ile Lys
            100                 105                 110
```

<210> 63
<211> 112
<212> PRT
<213> Artificial Sequence
<220>
<223> modified antibody fragment
<400> 63

```
Asp Val Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Val His Ser
            20                  25                  30
Asn Ser Asn Thr Tyr Leu His Trp Tyr Leu Gln Lys Pro Gly Gln Ser
        35                  40                  45
Pro Gln Leu Leu Ile Tyr Lys Val Ser Asn Arg Phe Ser Gly Val Pro
```

```
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Asn
                85                  90                  95
Thr His Val Pro Pro Thr Phe Gly Gln Gly Thr Lys Leu Glu Ile Lys
            100                 105                 110
```

<210> 64
<211> 112
<212> PRT
<213> Artificial Sequence
<220>
<223> modified antibody fragment
<400> 64

```
Asp Val Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Val His Ser
            20                  25                  30
Asn Trp Asn Thr Tyr Leu His Trp Tyr Leu Gln Lys Pro Gly Gln Ser
        35                  40                  45
Pro Gln Leu Leu Ile Tyr Lys Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Asn
                85                  90                  95
Thr His Val Pro Pro Thr Phe Gly Gln Gly Thr Lys Leu Glu Ile Lys
            100                 105                 110
```

<210> 65
<211> 112
<212> PRT
<213> Artificial Sequence
<220>
<223> modified antibody fragment
<400> 65

```
Asp Val Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Val His Ser
            20                  25                  30
Asn Tyr Asn Thr Tyr Leu His Trp Tyr Leu Gln Lys Pro Gly Gln Ser
        35                  40                  45
Pro Gln Leu Leu Ile Tyr Lys Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Asn
                85                  90                  95
Thr His Val Pro Pro Thr Phe Gly Gln Gly Thr Lys Leu Glu Ile Lys
            100                 105                 110
```

<210> 66
<211> 112
<212> PRT
<213> Artificial Sequence

<220>
<223> modified antibody fragment
<400> 66

```
Asp Val Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Val His Ser
            20                  25                  30
Asn Arg Asn Thr Tyr Leu His Trp Tyr Leu Gln Lys Pro Gly Gln Ser
        35                  40                  45
Pro Gln Leu Leu Ile Tyr Lys Val Ser Asn Arg Phe Ser Gly Val Pro


    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Asn
                85                  90                  95
Thr His Val Pro Pro Thr Phe Gly Gln Gly Thr Lys Leu Glu Ile Lys
            100                 105                 110
```

<210> 67
<211> 115
<212> PRT
<213> Artificial Sequence
<220>
<223> modified antibody fragment
<400> 67

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Glu Met His Trp Ile Arg Gln Pro Pro Gly Lys Gly Leu Glu Trp Ile
        35                  40                  45
Gly Ala Ile Asn Pro Lys Thr Gly Asp Thr Ala Tyr Ser Gln Lys Phe
    50                  55                  60
Lys Gly Arg Val Thr Leu Thr Ala Asp Lys Ser Thr Ser Thr Ala Tyr
65                  70                  75                  80
Met Glu Leu Ser Ser Leu Thr Ser Glu Asp Thr Ala Val Tyr Tyr Cys
                85                  90                  95
Thr Arg Phe Tyr Ser Tyr Thr Tyr Trp Gly Arg Gly Thr Leu Val Thr
            100                 105                 110
```

Val Ser Ser 115
<210> 68
<211> 112
<212> PRT
<213> Artificial Sequence
<220>
<223> modified antibody fragment
<400> 68

```
Asp Ile Val Met Thr Gln Ser Pro Leu Ser Leu Pro Val Thr Pro Gly
1               5                   10                  15
Gln Pro Ala Ser Ile Ser Cys Arg Ala Ser Arg Ser Leu Val His Ser
            20                  25                  30
Asn Arg Asn Thr Tyr Leu His Trp Tyr Gln Gln Lys Pro Gly Gln Ala
        35                  40                  45
Pro Arg Leu Leu Ile Tyr Lys Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Asn
                85                  90                  95
Thr His Val Pro Pro Thr Phe Gly Arg Gly Thr Lys Leu Glu Ile Lys
            100                 105                 110
```

<210> 69
<211> 115
<212> PRT
<213> Artificial Sequence
<220>
<223> modified antibody fragment
<400> 69

```
Gln Val Gln Leu Val Gln Ser Gly Ala Glu Val Lys Lys Pro Gly Ala
1               5                   10                  15
Ser Val Thr Val Ser Cys Lys Ala Ser Gly Tyr Thr Phe Thr Asp Tyr
            20                  25                  30
Glu Met His Trp Ile Arg Gln Pro Pro Gly Glu Gly Leu Glu Trp Ile

        35                  40                  45
Gly Ala Ile Asp Pro Lys Thr Gly Asp Thr Ala Tyr Ser Gln Ser Phe
    50                  55                  60
Gln Asp Arg Val Thr Leu Thr Ala Asp Lys Ser Thr Ser Thr Ala Tyr
65                  70                  75                  80
Met Glu Leu Ser Ser Leu Thr Ser Glu Asp Thr Ala Val Tyr Tyr Cys
                85                  90                  95
Thr Arg Phe Tyr Ser Tyr Thr Tyr Trp Gly Gln Gly Thr Leu Val Thr
            100                 105                 110
Val Ser Ser
        115
```

<210> 70
<211> 112
<212> PRT
<213> Artificial Sequence
<220>
<223> modified antibody fragment
<400> 70

```
Asp Ile Val Met Thr Gln Ser Pro Leu Ser Leu Pro Val Thr Pro Gly
1               5                   10                  15
Glu Pro Ala Ser Ile Ser Cys Arg Ala Ser Glu Ser Leu Val His Ser
            20                  25                  30
Asn Arg Asn Thr Tyr Leu His Trp Tyr Gln Gln Lys Pro Gly Gln Ala
        35                  40                  45
Pro Arg Leu Leu Ile Tyr Lys Val Ser Asn Arg Phe Ser Gly Val Pro
    50                  55                  60
Asp Arg Phe Ser Gly Ser Gly Ser Gly Thr Asp Phe Thr Leu Thr Ile
65                  70                  75                  80
Ser Ser Leu Gln Ala Glu Asp Val Gly Val Tyr Tyr Cys Ser Gln Asn
                85                  90                  95
Thr His Val Pro Pro Thr Phe Gly Gln Gly Thr Lys Leu Glu Ile Lys
            100                 105                 110
```

<210> 71
<211> 115
<212> PRT
<213> Artificial Sequence
<220>
<223> modified antibody fragment
<400> 71

```
Gln Val Gln Leu Val Gln Ser Gly Ala Glu Val Lys Lys Pro Gly Ala
1               5                   10                  15
Ser Val Thr Val Ser Cys Lys Ala Ser Gly Tyr Thr Phe Thr Asp Tyr
            20                  25                  30
Glu Met His Trp Ile Arg Gln Pro Pro Gly Glu Gly Leu Glu Trp Ile
        35                  40                  45
Gly Ala Ile Asp Pro Lys Thr Gly Asp Thr Ala Tyr Ser Glu Ser Phe
    50                  55                  60
Gln Asp Arg Val Thr Leu Thr Ala Asp Lys Ser Thr Ser Thr Ala Tyr
65                  70                  75                  80
Met Glu Leu Ser Ser Leu Thr Ser Glu Asp Thr Ala Val Tyr Tyr Cys
                85                  90                  95
Thr Arg Phe Tyr Ser Tyr Thr Tyr Trp Gly Gln Gly Thr Leu Val Thr
            100                 105                 110
Val Ser Ser
        115
```

<210> 72
<211> 112
<212> PRT
<213> Artificial Sequence
<220>
<223> modified antibody fragment
<400> 72

```
Asp Ile Val Met Thr Gln Ser Pro Leu Ser Leu Pro Val Thr Pro Gly
```

```
1               5                   10                  15
Glu Pro Ala Ser Ile Ser Cys Gln Ala Ser Glu Ser Leu Val His Ser
            20                  25                  30
Asn Arg Asn Thr Tyr Leu His Trp Tyr Leu Gln Lys Pro Gly Gln Ser
        35                  40                  45
Pro Gln Leu Leu Ile Tyr Lys Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Asn
                85                  90                  95
Thr His Val Pro Pro Thr Phe Gly Gln Gly Thr Lys Leu Glu Ile Lys
            100                 105                 110
```

<210> 73
<211> 112
<212> PRT
<213> Artificial Sequence
<220>
<223> modified antibody fragment
<400> 73

```
Asp Ile Val Met Thr Gln Ser Pro Leu Ser Leu Pro Val Thr Pro Gly
1               5                   10                  15
Glu Pro Ala Ser Ile Ser Cys Gln Ala Ser Glu Ser Leu Val His Ser
            20                  25                  30
Asn Arg Asn Thr Tyr Leu His Trp Tyr Leu Gln Lys Pro Gly Gln Ser
        35                  40                  45
Pro Gln Leu Leu Ile Tyr Lys Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Asn
                85                  90                  95
Thr His Val Pro Pro Thr Phe Gly Gln Gly Thr Lys Val Glu Ile Glu
            100                 105                 110
```

<210> 74
<211> 330
<212> PRT
<213> homo sapiens
<400> 74

Ala Ser Thr Lys Gly Pro Ser Val Phe Pro Leu Ala Pro Ser Ser Lys
1 5 10 15
Ser Thr Ser Gly Gly Thr Ala Ala Leu Gly Cys Leu Val Lys Asp Tyr
20 25 30
Phe Pro Glu Pro Val Thr Val Ser Trp Asn Ser Gly Ala Leu Thr Ser
35 40 45
Gly Val His Thr Phe Pro Ala Val Leu Gln Ser Ser Gly Leu Tyr Ser
50 55 60
Leu Ser Ser Val Val Thr Val Pro Ser Ser Ser Leu Gly Thr Gln Thr
65 70 75 80
Tyr Ile Cys Asn Val Asn His Lys Pro Ser Asn Thr Lys Val Asp Lys
85 90 95
Lys Val Glu Pro Lys Ser Cys Asp Lys Thr His Thr Cys Pro Pro Cys
100 105 110
Pro Ala Pro Glu Leu Leu Gly Gly Pro Ser Val Phe Leu Phe Pro Pro
115 120 125
Lys Pro Lys Asp Thr Leu Met Ile Ser Arg Thr Pro Glu Val Thr Cys
130 135 140
Val Val Val Asp Val Ser His Glu Asp Pro Glu Val Lys Phe Asn Trp
145 150 155 160
Tyr Val Asp Gly Val Glu Val His Asn Ala Lys Thr Lys Pro Arg Glu
165 170 175
Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205
Lys Ala Leu Pro Ala Pro Ile Glu Lys Thr Ile Ser Lys Ala Lys Gly
210 215 220
Gln Pro Arg Glu Pro Gln Val Tyr Thr Leu Pro Pro Ser Arg Asp Glu
225 230 235 240
Leu Thr Lys Asn Gln Val Ser Leu Thr Cys Leu Val Lys Gly Phe Tyr
245 250 255
Pro Ser Asp Ile Ala Val Glu Trp Glu Ser Asn Gly Gln Pro Glu Asn
260 265 270
Asn Tyr Lys Thr Thr Pro Pro Val Leu Asp Ser Asp Gly Ser Phe Phe
275 280 285
Leu Tyr Ser Lys Leu Thr Val Asp Lys Ser Arg Trp Gln Gln Gly Asn
290 295 300
Val Phe Ser Cys Ser Val Met His Glu Ala Leu His Asn His Tyr Thr
305 310 315 320
Gln Lys Ser Leu Ser Leu Ser Pro Gly Lys
325 330

<210> 75
<211> 326
<212> PRT
<213> homo sapiens
<400> 75

Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Asn Phe Gly Thr Gln Thr
65                  70                  75                  80
Tyr Thr Cys Asn Val Asp His Lys Pro Ser Asn Thr Lys Val Asp Lys
                85                  90                  95
Thr Val Glu Arg Lys Cys Cys Val Glu Cys Pro Pro Cys Pro Ala Pro
            100                 105                 110
Pro Val Ala Gly Pro Ser Val Phe Leu Phe Pro Pro Lys Pro Lys Asp
        115                 120                 125
Thr Leu Met Ile Ser Arg Thr Pro Glu Val Thr Cys Val Val Val Asp
    130                 135                 140
Val Ser His Glu Asp Pro Glu Val Gln Phe Asn Trp Tyr Val Asp Gly
145                 150                 155                 160
Val Glu Val His Asn Ala Lys Thr Lys Pro Arg Glu Glu Gln Phe Asn
                165                 170                 175
Ser Thr Phe Arg Val Val Ser Val Leu Thr Val Val His Gln Asp Trp
            180                 185                 190
Leu Asn Gly Lys Glu Tyr Lys Cys Lys Val Ser Asn Lys Gly Leu Pro
        195                 200                 205
Ala Pro Ile Glu Lys Thr Ile Ser Lys Thr Lys Gly Gln Pro Arg Glu
    210                 215                 220
Pro Gln Val Tyr Thr Leu Pro Pro Ser Arg Glu Glu Met Thr Lys Asn
225                 230                 235                 240
Gln Val Ser Leu Thr Cys Leu Val Lys Gly Phe Tyr Pro Ser Asp Ile
                245                 250                 255
Ala Val Glu Trp Glu Ser Asn Gly Gln Pro Glu Asn Asn Tyr Lys Thr
            260                 265                 270
Thr Pro Pro Met Leu Asp Ser Asp Gly Ser Phe Phe Leu Tyr Ser Lys
        275                 280                 285
Leu Thr Val Asp Lys Ser Arg Trp Gln Gln Gly Asn Val Phe Ser Cys
    290                 295                 300
Ser Val Met His Glu Ala Leu His Asn His Tyr Thr Gln Lys Ser Leu
305                 310                 315                 320

Ser Leu Ser Pro Gly Lys
                325

<210> 76
<211> 377
<212> PRT
<213> homo sapiens
<400> 76

```
Ala Ser Thr Lys Gly Pro Ser Val Phe Pro Leu Ala Pro Cys Ser Arg
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Thr Cys Asn Val Asn His Lys Pro Ser Asn Thr Lys Val Asp Lys
                85                  90                  95
Arg Val Glu Leu Lys Thr Pro Leu Gly Asp Thr Thr His Thr Cys Pro
            100                 105                 110
Arg Cys Pro Glu Pro Lys Ser Cys Asp Thr Pro Pro Pro Cys Pro Arg
        115                 120                 125
Cys Pro Glu Pro Lys Ser Cys Asp Thr Pro Pro Pro Cys Pro Arg Cys
    130                 135                 140
Pro Glu Pro Lys Ser Cys Asp Thr Pro Pro Pro Cys Pro Arg Cys Pro
145                 150                 155                 160
Ala Pro Glu Leu Leu Gly Gly Pro Ser Val Phe Leu Phe Pro Pro Lys
                165                 170                 175
Pro Lys Asp Thr Leu Met Ile Ser Arg Thr Pro Glu Val Thr Cys Val
            180                 185                 190
Val Val Asp Val Ser His Glu Asp Pro Glu Val Gln Phe Lys Trp Tyr
        195                 200                 205
Val Asp Gly Val Glu Val His Asn Ala Lys Thr Lys Pro Arg Glu Glu
    210                 215                 220
Gln Tyr Asn Ser Thr Phe Arg Val Val Ser Val Leu Thr Val Leu His
225                 230                 235                 240
Gln Asp Trp Leu Asn Gly Lys Glu Tyr Lys Cys Lys Val Ser Asn Lys
                245                 250                 255
Ala Leu Pro Ala Pro Ile Glu Lys Thr Ile Ser Lys Thr Lys Gly Gln
            260                 265                 270
Pro Arg Glu Pro Gln Val Tyr Thr Leu Pro Pro Ser Arg Glu Glu Met
        275                 280                 285
Thr Lys Asn Gln Val Ser Leu Thr Cys Leu Val Lys Gly Phe Tyr Pro
    290                 295                 300
Ser Asp Ile Ala Val Glu Trp Glu Ser Ser Gly Gln Pro Glu Asn Asn
305                 310                 315                 320
Tyr Asn Thr Thr Pro Pro Met Leu Asp Ser Asp Gly Ser Phe Phe Leu
                325                 330                 335
Tyr Ser Lys Leu Thr Val Asp Lys Ser Arg Trp Gln Gln Gly Asn Ile
            340                 345                 350
Phe Ser Cys Ser Val Met His Glu Ala Leu His Asn Arg Phe Thr Gln
        355                 360                 365
Lys Ser Leu Ser Leu Ser Pro Gly Lys
    370                 375
```

<210> 77
<211> 327
<212> PRT
<213> homo sapiens
<400> 77

```
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
```

```
                20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Lys Thr
65                  70                  75                  80
Tyr Thr Cys Asn Val Asp His Lys Pro Ser Asn Thr Lys Val Asp Lys
                85                  90                  95
Arg Val Glu Ser Lys Tyr Gly Pro Pro Cys Pro Ser Cys Pro Ala Pro
            100                 105                 110
Glu Phe Leu Gly Gly Pro Ser Val Phe Leu Phe Pro Pro Lys Pro Lys
        115                 120                 125
Asp Thr Leu Met Ile Ser Arg Thr Pro Glu Val Thr Cys Val Val Val
    130                 135                 140
Asp Val Ser Gln Glu Asp Pro Glu Val Gln Phe Asn Trp Tyr Val Asp
145                 150                 155                 160
Gly Val Glu Val His Asn Ala Lys Thr Lys Pro Arg Glu Glu Gln Phe
                165                 170                 175
Asn Ser Thr Tyr Arg Val Val Ser Val Leu Thr Val Leu His Gln Asp
            180                 185                 190
Trp Leu Asn Gly Lys Glu Tyr Lys Cys Lys Val Ser Asn Lys Gly Leu
        195                 200                 205
Pro Ser Ser Ile Glu Lys Thr Ile Ser Lys Ala Lys Gly Gln Pro Arg
    210                 215                 220
Glu Pro Gln Val Tyr Thr Leu Pro Pro Ser Gln Glu Glu Met Thr Lys
225                 230                 235                 240
Asn Gln Val Ser Leu Thr Cys Leu Val Lys Gly Phe Tyr Pro Ser Asp
                245                 250                 255
Ile Ala Val Glu Trp Glu Ser Asn Gly Gln Pro Glu Asn Asn Tyr Lys
            260                 265                 270
Thr Thr Pro Pro Val Leu Asp Ser Asp Gly Ser Phe Phe Leu Tyr Ser
        275                 280                 285
Arg Leu Thr Val Asp Lys Ser Arg Trp Gln Glu Gly Asn Val Phe Ser
    290                 295                 300
Cys Ser Val Met His Glu Ala Leu His Asn His Tyr Thr Gln Lys Ser
305                 310                 315                 320
Leu Ser Leu Ser Leu Gly Lys
                325
```

<210> 78
<211> 374
<212> PRT
<213> homo sapiens
<400> 78

```
Met Trp Phe Leu Thr Thr Leu Leu Leu Trp Val Pro Val Asp Gly Gln
1               5                   10                  15
Val Asp Thr Thr Lys Ala Val Ile Thr Leu Gln Pro Pro Trp Val Ser
            20                  25                  30
Val Phe Gln Glu Glu Thr Val Thr Leu His Cys Glu Val Leu His Leu
        35                  40                  45
Pro Gly Ser Ser Ser Thr Gln Trp Phe Leu Asn Gly Thr Ala Thr Gln
    50                  55                  60
Thr Ser Thr Pro Ser Tyr Arg Ile Thr Ser Ala Ser Val Asn Asp Ser
65                  70                  75                  80
Gly Glu Tyr Arg Cys Gln Arg Gly Leu Ser Gly Arg Ser Asp Pro Ile
                85                  90                  95
Gln Leu Glu Ile His Arg Gly Trp Leu Leu Leu Gln Val Ser Ser Arg
            100                 105                 110
Val Phe Thr Glu Gly Glu Pro Leu Ala Leu Arg Cys His Ala Trp Lys
        115                 120                 125
Asp Lys Leu Val Tyr Asn Val Leu Tyr Tyr Arg Asn Gly Lys Ala Phe
    130                 135                 140
Lys Phe Phe His Trp Asn Ser Asn Leu Thr Ile Leu Lys Thr Asn Ile


145                 150                 155                 160
Ser His Asn Gly Thr Tyr His Cys Ser Gly Met Gly Lys His Arg Tyr
                165                 170                 175
Thr Ser Ala Gly Ile Ser Val Thr Val Lys Glu Leu Phe Pro Ala Pro
            180                 185                 190
Val Leu Asn Ala Ser Val Thr Ser Pro Leu Leu Glu Gly Asn Leu Val
        195                 200                 205
Thr Leu Ser Cys Glu Thr Lys Leu Leu Leu Gln Arg Pro Gly Leu Gln
    210                 215                 220
Leu Tyr Phe Ser Phe Tyr Met Gly Ser Lys Thr Leu Arg Gly Arg Asn
225                 230                 235                 240
Thr Ser Ser Glu Tyr Gln Ile Leu Thr Ala Arg Arg Glu Asp Ser Gly
                245                 250                 255
Leu Tyr Trp Cys Glu Ala Ala Thr Glu Asp Gly Asn Val Leu Lys Arg
            260                 265                 270
Ser Pro Glu Leu Glu Leu Gln Val Leu Gly Leu Gln Leu Pro Thr Pro
        275                 280                 285
Val Trp Phe His Val Leu Phe Tyr Leu Ala Val Gly Ile Met Phe Leu
    290                 295                 300
Val Asn Thr Val Leu Trp Val Thr Ile Arg Lys Glu Leu Lys Arg Lys
305                 310                 315                 320
Lys Lys Trp Asp Leu Glu Ile Ser Leu Asp Ser Gly His Glu Lys Lys
                325                 330                 335
Val Ile Ser Ser Leu Gln Glu Asp Arg His Leu Glu Glu Glu Leu Lys
            340                 345                 350
Cys Gln Glu Gln Lys Glu Glu Gln Leu Gln Glu Gly Val His Arg Lys
        355                 360                 365
Glu Pro Gln Gly Ala Thr
    370
```

<210> 79
<211> 316
<212> PRT
<213> homo sapiens
<400> 79

```
Met Thr Met Glu Thr Gln Met Ser Gln Asn Val Cys Pro Arg Asn Leu
1               5                   10                  15
Trp Leu Leu Gln Pro Leu Thr Val Leu Leu Leu Leu Ala Ser Ala Asp
            20                  25                  30
Ser Gln Ala Ala Pro Pro Lys Ala Val Leu Lys Leu Glu Pro Pro Trp
        35                  40                  45
Ile Asn Val Leu Gln Glu Asp Ser Val Thr Leu Thr Cys Gln Gly Ala
    50                  55                  60
Arg Ser Pro Glu Ser Asp Ser Ile Gln Trp Phe His Asn Gly Asn Leu
65                  70                  75                  80
Ile Pro Thr His Thr Gln Pro Ser Tyr Arg Phe Lys Ala Asn Asn Asn
                85                  90                  95
Asp Ser Gly Glu Tyr Thr Cys Gln Thr Gly Gln Thr Ser Leu Ser Asp
            100                 105                 110
Pro Val His Leu Thr Val Leu Ser Glu Trp Leu Val Leu Gln Thr Pro
        115                 120                 125
His Leu Glu Phe Gln Glu Gly Glu Thr Ile Met Leu Arg Cys His Ser
    130                 135                 140
Trp Lys Asp Lys Pro Leu Val Lys Val Thr Phe Phe Gln Asn Gly Lys
145                 150                 155                 160
Ser Gln Lys Phe Ser His Leu Asp Pro Thr Phe Ser Ile Pro Gln Ala
                165                 170                 175
Asn His Ser His Ser Gly Asp Tyr His Cys Thr Gly Asn Ile Gly Tyr
            180                 185                 190
Thr Leu Phe Ser Ser Lys Pro Val Thr Ile Thr Val Gln Val Pro Ser
        195                 200                 205
Met Gly Ser Ser Ser Pro Met Gly Val Ile Val Ala Val Val Ile Ala
    210                 215                 220
Thr Ala Val Ala Ala Ile Val Ala Ala Val Val Ala Leu Ile Tyr Cys


225                 230                 235                 240
Arg Lys Lys Arg Ile Ser Ala Asn Ser Thr Asp Pro Val Lys Ala Ala
                245                 250                 255
Gln Phe Glu Pro Pro Gly Arg Gln Met Ile Ala Ile Arg Lys Arg Gln
            260                 265                 270
Leu Glu Glu Thr Asn Asn Asp Tyr Glu Thr Ala Asp Gly Gly Tyr Met
        275                 280                 285
Thr Leu Asn Pro Arg Ala Pro Thr Asp Asp Asp Lys Asn Ile Tyr Leu
    290                 295                 300
Thr Leu Pro Pro Asn Asp His Val Asn Ser Asn Asn
305                 310                 315
```

<210> 80
<211> 291
<212> PRT
<213> homo sapiens
<400> 80

```
Met Gly Ile Leu Ser Phe Leu Pro Val Leu Ala Thr Glu Ser Asp Trp
1               5                   10                  15
Ala Asp Cys Lys Ser Pro Gln Pro Trp Gly His Met Leu Leu Trp Thr
            20                  25                  30
Ala Val Leu Phe Leu Ala Pro Val Ala Gly Thr Pro Ala Ala Pro Pro
        35                  40                  45
Lys Ala Val Leu Lys Leu Glu Pro Gln Trp Ile Asn Val Leu Gln Glu
    50                  55                  60
Asp Ser Val Thr Leu Thr Cys Arg Gly Thr His Ser Pro Glu Ser Asp
65                  70                  75                  80
Ser Ile Gln Trp Phe His Asn Gly Asn Leu Ile Pro Thr His Thr Gln
                85                  90                  95
Pro Ser Tyr Arg Phe Lys Ala Asn Asn Asn Asp Ser Gly Glu Tyr Thr
            100                 105                 110
Cys Gln Thr Gly Gln Thr Ser Leu Ser Asp Pro Val His Leu Thr Val
        115                 120                 125
Leu Ser Glu Trp Leu Val Leu Gln Thr Pro His Leu Glu Phe Gln Glu
    130                 135                 140
Gly Glu Thr Ile Val Leu Arg Cys His Ser Trp Lys Asp Lys Pro Leu
145                 150                 155                 160
Val Lys Val Thr Phe Phe Gln Asn Gly Lys Ser Lys Lys Phe Ser Arg
                165                 170                 175
Ser Asp Pro Asn Phe Ser Ile Pro Gln Ala Asn His Ser His Ser Gly
            180                 185                 190
Asp Tyr His Cys Thr Gly Asn Ile Gly Tyr Thr Leu Tyr Ser Ser Lys
        195                 200                 205
Pro Val Thr Ile Thr Val Gln Ala Pro Ser Ser Ser Pro Met Gly Ile
    210                 215                 220
Ile Val Ala Val Val Thr Gly Ile Ala Val Ala Ala Ile Val Ala Ala
225                 230                 235                 240
Val Val Ala Leu Ile Tyr Cys Arg Lys Lys Arg Ile Ser Ala Asn Pro
                245                 250                 255
Thr Asn Pro Asp Glu Ala Asp Lys Val Gly Ala Glu Asn Thr Ile Thr
            260                 265                 270
Tyr Ser Leu Leu Met His Pro Asp Ala Leu Glu Glu Pro Asp Asp Gln
        275                 280                 285
Asn Arg Ile
    290
```

<210> 81
<211> 254
<212> PRT
<213> homo sapiens
<400> 81

```
Met Trp Gln Leu Leu Leu Pro Thr Ala Leu Leu Leu Leu Val Ser Ala
1               5                   10                  15
Gly Met Arg Thr Glu Asp Leu Pro Lys Ala Val Val Phe Leu Glu Pro
            20                  25                  30
```

```
Gln Trp Tyr Arg Val Leu Glu Lys Asp Ser Val Thr Leu Lys Cys Gln
        35                  40                  45
Gly Ala Tyr Ser Pro Glu Asp Asn Ser Thr Gln Trp Phe His Asn Glu
    50                  55                  60
Ser Leu Ile Ser Ser Gln Ala Ser Ser Tyr Phe Ile Asp Ala Ala Thr
65                  70                  75                  80
Val Asp Asp Ser Gly Glu Tyr Arg Cys Gln Thr Asn Leu Ser Thr Leu
                85                  90                  95
Ser Asp Pro Val Gln Leu Glu Val His Ile Gly Trp Leu Leu Leu Gln
            100                 105                 110
Ala Pro Arg Trp Val Phe Lys Glu Glu Asp Pro Ile His Leu Arg Cys
        115                 120                 125
His Ser Trp Lys Asn Thr Ala Leu His Lys Val Thr Tyr Leu Gln Asn
    130                 135                 140
Gly Lys Gly Arg Lys Tyr Phe His His Asn Ser Asp Phe Tyr Ile Pro
145                 150                 155                 160
Lys Ala Thr Leu Lys Asp Ser Gly Ser Tyr Phe Cys Arg Gly Leu Val
                165                 170                 175
Gly Ser Lys Asn Val Ser Ser Glu Thr Val Asn Ile Thr Ile Thr Gln
            180                 185                 190
Gly Leu Ser Val Ser Thr Ile Ser Ser Phe Phe Pro Pro Gly Tyr Gln
        195                 200                 205
Val Ser Phe Cys Leu Val Met Val Leu Leu Phe Ala Val Asp Thr Gly
    210                 215                 220
Leu Tyr Phe Ser Val Lys Thr Asn Ile Arg Ser Ser Thr Arg Asp Trp
225                 230                 235                 240
Lys Asp His Lys Phe Lys Trp Arg Lys Asp Pro Gln Asp Lys
                245                 250
```

<210> 82
<211> 233
<212> PRT
<213> homo sapiens
<400> 82

```
Met Trp Gln Leu Leu Leu Pro Thr Ala Leu Leu Leu Leu Val Ser Ala
1               5                   10                  15
Gly Met Arg Thr Glu Asp Leu Pro Lys Ala Val Val Phe Leu Glu Pro
            20                  25                  30
Gln Trp Tyr Ser Val Leu Glu Lys Asp Ser Val Thr Leu Lys Cys Gln
        35                  40                  45
Gly Ala Tyr Ser Pro Glu Asp Asn Ser Thr Gln Trp Phe His Asn Glu
    50                  55                  60
Ser Leu Ile Ser Ser Gln Ala Ser Ser Tyr Phe Ile Asp Ala Ala Thr
65                  70                  75                  80
Val Asn Asp Ser Gly Glu Tyr Arg Cys Gln Thr Asn Leu Ser Thr Leu
                85                  90                  95
Ser Asp Pro Val Gln Leu Glu Val His Ile Gly Trp Leu Leu Leu Gln
            100                 105                 110
Ala Pro Arg Trp Val Phe Lys Glu Glu Asp Pro Ile His Leu Arg Cys
        115                 120                 125
His Ser Trp Lys Asn Thr Ala Leu His Lys Val Thr Tyr Leu Gln Asn
    130                 135                 140
Gly Lys Asp Arg Lys Tyr Phe His His Asn Ser Asp Phe His Ile Pro
145                 150                 155                 160
Lys Ala Thr Leu Lys Asp Ser Gly Ser Tyr Phe Cys Arg Gly Leu Val
                165                 170                 175
Gly Ser Lys Asn Val Ser Ser Glu Thr Val Asn Ile Thr Ile Thr Gln
            180                 185                 190
Gly Leu Ala Val Ser Thr Ile Ser Ser Phe Ser Pro Pro Gly Tyr Gln
        195                 200                 205
Val Ser Phe Cys Leu Val Met Val Leu Leu Phe Ala Val Asp Thr Gly
    210                 215                 220
Leu Tyr Phe Ser Val Lys Thr Asn Ile
225                 230
```

<210> 83
<211> 140
<212> PRT
<213> Mus musculus
<400> 83

```
Met Glu Trp Ile Trp Ile Phe Leu Phe Ile Leu Ser Gly Thr Ala Gly
1               5                   10                  15
Val Gln Ser Gln Val Gln Leu Gln Gln Ser Gly Ala Glu Leu Ala Arg
            20                  25                  30
Pro Gly Ala Ser Val Lys Leu Ser Cys Arg Ala Ser Gly Tyr Thr Phe
        35                  40                  45
Thr Ser Tyr Gly Ile Ser Trp Met Met Gln Arg Thr Gly Gln Gly Leu
    50                  55                  60
Glu Trp Ile Gly Glu Ile Tyr Pro Arg Ser Gly Ile Thr Tyr Tyr Asn
65                  70                  75                  80
Glu Lys Phe Lys Gly Lys Ala Thr Leu Thr Ala Asp Lys Ser Ser Ser
                85                  90                  95
Thr Ala Tyr Met Gln Leu Ser Ser Leu Thr Ser Glu Asp Ser Ala Val
            100                 105                 110
Tyr Phe Cys Ala Arg Asp Val Ser Asp Gly Tyr Leu Phe Pro Tyr Trp
        115                 120                 125
Gly Gln Gly Thr Leu Val Thr Val Ser Ala Ala Lys
    130                 135                 140
```

<210> 84
<211> 129
<212> PRT
<213> Mus musculus

<400> 84

```
Met Ser Val Pro Thr Gln Val Leu Gly Leu Leu Leu Leu Trp Leu Thr
1               5                   10                  15
Gly Ala Arg Cys Asp Ile Gln Met Thr Gln Ser Pro Ala Ser Leu Ser
            20                  25                  30
Ala Ser Val Gly Glu Thr Val Thr Ile Thr Cys Arg Thr Ser Glu Asn
        35                  40                  45
Ile Tyr Ser Tyr Leu Ala Trp Tyr Gln Gln Lys Gln Gly Lys Ser Pro
    50                  55                  60
Gln Leu Leu Val Tyr Asn Ala Lys Thr Leu Pro Glu Gly Val Pro Ser
65                  70                  75                  80
Arg Phe Ser Gly Ser Gly Ser Gly Thr Gln Phe Ser Leu Lys Ile Asn
                85                  90                  95
Ser Leu Gln Pro Glu Asp Phe Gly Ser Tyr Tyr Cys Gln His His Tyr
            100                 105                 110
Gly Thr Pro Pro Thr Phe Gly Gly Gly Thr Lys Leu Glu Ile Lys Arg
        115                 120                 125
Ala
```

<210> 85
<211> 139
<212> PRT
<213> Mus musculus
<400> 86

```
Met Asn Phe Gly Leu Ser Leu Ile Phe Leu Ala Leu Ile Leu Lys Gly
1               5                   10                  15
Val Gln Cys Glu Val Gln Leu Val Glu Ser Gly Gly Asp Val Val Arg
            20                  25                  30
Pro Gly Gly Ser Leu Lys Leu Ser Cys Ala Ala Ser Gly Phe Thr Phe
        35                  40                  45
Ser Ser Tyr Gly Met Ser Trp Val Arg Gln Leu Pro Asp Lys Arg Leu
    50                  55                  60
Glu Trp Val Ala Ser Val Gly Asn Gly Gly Ser Tyr Arg Tyr Tyr Pro
65                  70                  75                  80
Glu Asn Leu Lys Gly Arg Phe Thr Ile Ser Arg Asp Asn Thr Lys Asn
                85                  90                  95

Thr Leu Tyr Leu Gln Ile Ser Gly Leu Lys Ser Glu Asp Thr Ala Ile
            100                 105                 110
Tyr Tyr Cys Ala Arg Arg Gly Ala Phe Pro Tyr Phe Asp Val Trp Gly
        115                 120                 125
Ala Gly Thr Thr Val Thr Val Ser Ser Ala Lys
    130                 135
```

<210> 86
<211> 129
<212> PRT
<213> Mus musculus
<400> 86

Met Ser Val Pro Thr Gln Val Leu Gly Leu Leu Leu Leu Trp Leu Thr
1 5 10 15
Gly Ala Arg Cys Asp Ile Gln Met Thr Gln Ser Pro Ala Ser Leu Ser
20 25 30
Ala Ser Val Gly Glu Thr Val Thr Ile Thr Cys Arg Thr Ser Glu Asn
35 40 45
Ile Tyr Ser Tyr Leu Ala Trp Tyr Gln Gln Lys Gln Gly Lys Ser Pro
50 55 60
Gln Leu Leu Val Tyr Asn Ala Lys Thr Leu Pro Glu Gly Val Pro Ser
65 70 75 80
Arg Phe Ser Gly Ser Gly Ser Gly Thr Gln Phe Ser Leu Lys Ile Asn
85 90 95
Ser Leu Gln Pro Glu Asp Phe Gly Ser Tyr Tyr Cys Gln His His Tyr
100 105 110
Gly Thr Pro Pro Thr Phe Gly Gly Gly Thr Lys Leu Glu Ile Lys Arg
115 120 125
Ala

<210> 87
<211> 136
<212> PRT
<213> Mus musculus
<400> 87

Met Arg Val Leu Ile Leu Leu Trp Leu Phe Thr Ala Phe Pro Gly Ile
1 5 10 15
Leu Ser Asp Val Gln Leu Gln Glu Ser Gly Pro Gly Leu Val Lys Pro
20 25 30
Ser Gln Ser Leu Ser Leu Thr Cys Thr Val Thr Gly Tyr Ser Ile Thr
35 40 45
Ser Asp Ser Ala Trp Asn Trp Ile Arg Gln Phe Pro Gly Asn Lys Leu
50 55 60
Glu Trp Met Ala Tyr Ile Met Tyr Ser Gly Ile Thr Ser Tyr Asn Pro
65 70 75 80
Ser Leu Lys Ser Arg Ile Ser Ile Thr Arg Asp Thr Ala Lys Asn Gln
85 90 95
Phe Phe Leu Gln Leu Asn Ser Val Thr Thr Glu Asp Ser Ala Thr Tyr
100 105 110
Tyr Cys Ser Arg Gly Tyr Trp Tyr Phe Asp Val Trp Gly Ala Gly Thr
115 120 125
Thr Val Thr Val Ser Ser Ala Lys
130 135

<210> 88
<211> 129
<212> PRT
<213> Mus musculus
<400> 88

Met Asp Phe Gln Val Gln Ile Phe Ser Phe Leu Leu Ile Ser Ala Ser
1 5 10 15
Val Ile Met Ser Arg Gly Gln Ile Val Leu Thr Gln Ser Pro Ala Ile
20 25 30
Met Ser Ala Ser Leu Gly Glu Glu Ile Thr Leu Thr Cys Ser Ala Ser
35 40 45

```
Ser Ser Val Ser Tyr Met His Trp Tyr Gln Gln Lys Ser Gly Thr Ser
    50                  55                  60
Pro Lys Leu Leu Ile Tyr Ser Thr Ser Ile Leu Ala Ser Gly Val Pro
65                  70                  75                  80
Ser Arg Phe Ser Gly Ser Gly Ser Gly Thr Phe Tyr Ser Leu Thr Ile
                85                  90                  95
Ser Ser Val Glu Ala Glu Asp Ala Ala Asp Tyr Tyr Cys Leu Gln Trp
            100                 105                 110
Ile Thr Tyr Arg Thr Phe Gly Gly Gly Thr Lys Leu Glu Ile Lys Arg
        115                 120                 125
Ala
```

<210> 89
<211> 138
<212> PRT
<213> Mus musculus
<400> 89

```
Met Cys Trp Ser Cys Ile Ile Leu Phe Leu Leu Ala Thr Ala Ala Arg
1               5                   10                  15
Val His Ser Gln Val Gln Leu Gln Gln Ser Gly Ala Glu Leu Val Gly
            20                  25                  30
Pro Gly Ala Ser Val Lys Ile Ser Cys Lys Ala Phe Gly Tyr Thr Phe
        35                  40                  45
Thr Asn His His Ile Asn Trp Val Lys Gln Arg Pro Gly Gln Gly Leu
    50                  55                  60
Asp Trp Ile Gly Tyr Ile Asn Pro Tyr Asn Asp Tyr Thr Asn Tyr Asn
65                  70                  75                  80
Gln Lys Phe Lys Gly Lys Ala Thr Leu Thr Val Asp Lys Ser Ser Ser
                85                  90                  95
Thr Ala Tyr Met Glu Leu Ser Ser Leu Thr Ser Glu Asp Ser Ala Val
            100                 105                 110
Tyr Tyr Cys Ala Arg Ser Asp Pro Ala Trp Phe Ala Tyr Trp Gly Gln
        115                 120                 125
Gly Thr Leu Val Thr Val Ser Ala Ala Lys
    130                 135
```

<210> 90
<211> 129
<212> PRT
<213> Mus musculus
<400> 90

```
Met Arg Pro Ser Ile Gln Phe Leu Gly Leu Leu Leu Phe Trp Leu His
1               5                   10                  15
Gly Ala Gln Cys Asp Ile Gln Met Thr Gln Ser Pro Ser Ser Leu Ser
            20                  25                  30
Ala Ser Leu Gly Gly Lys Val Thr Ile Thr Cys Lys Ala Ser Gln Asp
        35                  40                  45
Ile Asn Lys Asn Ile Ala Trp Tyr Gln His Lys Pro Gly Lys Gly Pro
    50                  55                  60
Arg Leu Leu Ile Trp Tyr Thr Tyr Thr Leu Gln Pro Gly Ile Pro Ser
65                  70                  75                  80
Arg Phe Ser Gly Ser Gly Ser Gly Arg Asp Tyr Ser Phe Ser Ile Ser
                85                  90                  95
Asn Leu Glu Pro Glu Asp Ile Ala Thr Tyr Tyr Cys Leu Gln Tyr Asp
            100                 105                 110
Asn Leu Pro Phe Thr Phe Gly Thr Gly Thr Lys Leu Glu Ile Lys Arg
        115                 120                 125
Ala
```

## Description

[Technical Field]

The present invention provides a method for determining the efficacy of GPC3-targeting drug therapy for cancer in a patient or determining the continuation of GPC3-targeting drug therapy for a patient. The present invention also relates to a GPC3-targeting drug for use in a method of treating GPC3-expressing cancer where the GPC-3 targeting drug is to be further administered to a patient for the efficacy of the GPC3-targeting drug therapy has been determined or the continuation of the GPC3-targeting drug therapy has been determined. The GPC3-targeting drug comprises an ant-GPC3 antibody.

[Background Art]

Hepatocellular cancer is reportedly the fifth leading cause of cancer deaths worldwide, accounting for approximately 600,000 deaths each year (Non Patent Literature 1). Most patients with hepatocellular cancer die within 1 year after being diagnosed with the disease. Unfortunately, hepatocellular cancer cases are frequently diagnosed at a late stage which rarely responds to curative therapies. Still, medical treatments including chemotherapy, chemoembolization, ablation, and proton beam therapy are insufficiently effective for such patients. Many patients exhibit recurrence of the disease with vascular invasion and multiple intrahepatic metastases, which rapidly progresses to the advanced stage. Their 5-year survival rates are only 7% (Non Patent Literature 2). Patients with hepatocellular cancer amenable to the resection of local foci have relatively good prognosis, though their 5-year survival rates still remain at a level of 15% and 39% (Non Patent Literature 3). Thus, there has been a demand in the art for novel therapy for such a malignant disease hepatocellular cancer.

Hepatocellular cancer is reportedly responsible for more than 90% of primary liver cancer cases in Japan. Medical methods for treating such hepatocellular cancer include, for example, chemotherapy-based transcatheter arterial embolization (TAE) therapy, which involves inducing the selective necrosis of the hepatocellular cancer by the injection of a mixture of an oil-based contrast medium (Lipiodol), an anticancer agent, and an obstructing substance (Gelfoam) into the hepatic artery (which serves as a nutrient supply pathway to the tumor) resulting in the obstruction of the nutrient artery. In addition, invasive approaches are used, such as percutaneous ethanol injection, percutaneous microwave coagulation therapy, and radiofrequency ablation. Also, clinical trials have been conducted on systemic chemotherapy using chemotherapeutic agents such as fluorouracil (5-FU), uracil-tegafur (UFT), mitomycin C (MMC), mitoxantrone (DHAD), adriamycin (ADR), epirubicin (EPI), and cisplatin (CDDP) either alone or in combination with interferon (IFN) (Non Patent Literature 4) .

Meanwhile, an orally active form of sorafenib (Nexavar, BAY43-9006) has been approved, which is more advantageously effective than the chemotherapeutic agents described above in such a way that this agent blocks the growth of cancer cells by inhibiting Raf kinase in the Raf/MEK/ERK signal transduction while the agent exerts antiangiogenic effects by targeting VEGFR-2, VEGFR-3, and PDGFR-β tyrosine kinases. The efficacy of sorafenib has been studied in two phase-III multicenter placebocontrolled trials (Sorafenib HCC Assessment Randomized Protocol (SHARP) trial and Asia-Pacific trial) targeting advanced hepatocellular cancer. Sorafenib was confirmed to prolong survival durations, with HR of 0.68, in both of these trials. In the SHARP trial, sorafenib prolonged the survival duration to 10.7 months versus 7.9 months with the placebo. In the Asian trial, this agent prolonged the survival duration to 6.5 months versus 4.2 months with the placebo. The agent, however, had a low objective response rate and showed no prolongation of a time to symptomatic progression, though the agent prolonged a time to tumor progression (5.5 months versus 2.8 months in the European and American trial and 2.8 months versus 1.4 months in the Asian trial) on the images. The Asian cohorts exhibited a short duration of life prolongation, which is probably because their treatments were started at a slightly later stage during the disease process in the Asian region compared with Europe and the United States (Non Patent Literatures 5 and 6).

As liver cancer progresses, its specific symptoms associated with liver dysfunction are generally observed, such as anorexia, weight loss, general malaise, palpable right hypochondrial mass, right hypochondrial pain, sense of abdominal fullness, fever, and jaundice. The chemotherapeutic agents (e.g., sorafenib), however, have complications to be overcome, including their inherent adverse reactions such as diarrhea or constipation, anemia, suppression of the immune system to cause infection or sepsis (with lethal severity), hemorrhage, cardiac toxicity, hepatic toxicity, renal toxicity, anorexia, and weight loss.

Although particular early-stage symptoms are not initially observed in liver cancer, its specific symptoms associated with liver dysfunction, such as anorexia, weight loss, general malaise, palpable right hypochondrial mass, right hypochondrial pain, sense of abdominal fullness, fever, and jaundice, are generally observed with progression of the liver cancer. According to clinical observation, such symptoms are enhanced by use of the chemotherapeutic agents. For example, anorexia in a patient with detectable liver cancer cells and symptoms such as weight loss associated with or independent of the anorexia may be more enhanced by the administration of the chemotherapeutic agents to the patient than without the use of the chemotherapeutic agents. In some cases, the use of the chemotherapeutic agents must be discontinued for the patient having such symptoms. These enhanced symptoms are impediments to treatments with the chemotherapeutic agents. Thus, there has been a demand for the establishment of excellent therapy from the viewpoint of, for example, improving therapeutic effects or improving QOL of patients to be treated.

Glypican 3 (GPC3) is frequently expressed at a high level in liver cancer and as such, seems to be useful in the identification of its functions in liver cancer or as a therapeutic or diagnostic target of liver cancer.

Under the circumstances described above, drugs are under development with GPC3 as a therapeutic target of liver cancer. A liver cancer drug comprising an anti-GPC3 antibody as an active ingredient has been developed, the antibody having antibody-dependent cellular cytotoxicity (hereinafter, referred to as "ADCC") activity and/or complement-dependent cytotoxicity (hereinafter, referred to as "CDC") activity against cells expressing GPC3 (Patent Literature 1). Also, a GPC3-targeting drug comprising a humanized anti-GPC3 antibody having ADCC activity and CDC activity as an active ingredient has been developed (Patent Literature 2). Further GPC3-targeting drugs have been developed, which comprise a humanized anti-GPC3 antibody with enhanced ADCC activity (Patent Literatures 3 and 4) or an anti-GPC3 antibody having ADCC activity and CDC activity as well as improved plasma dynamics (Patent Literature 5). These anti-GPC3 antibodies in combination therapy with the chemotherapeutic agents such as sorafenib have been found to attenuate the adverse reactions, for example, brought about by the sole therapy of the chemotherapeutic agents (e.g., sorafenib) and also found to exhibit synergistic effects based on these agents (Patent Literature 6). Accordingly, excellent methods for treating liver cancer are in the process of being established using GPC3-targeting drugs as the nucleus from the viewpoint of, for example, improving therapeutic effects or improving QOL of patients to be treated.

Meanwhile, GPC3-targeting methods for diagnosing liver cancer are also under development. GPC3 is known to be expressed on cell surface and processed, at the particular site, by convertase, phospholipase D, Notum or unspecified mechanism (Non Patent Literature 7 and 8) during or after expression on cell surface. By use of such a phenomenon, a diagnostic agent or a diagnostic method for liver cancer has been developed, which involves an antibody capable of binding to an epitope in a soluble form of GPC3 secreted into the plasma of a patient after processing (Patent Literature 7). Also, a diagnostic agent or a diagnostic method for liver cancer has been developed, which involves an antibody capable of binding to an epitope in an anchored form of GPC3 still existing on cell surface after processing in a tissue preparation or the like isolated from a patient (Patent Literature 8). These diagnostic agents or diagnostic methods, however, are means for detecting the presence of liver cancer in a patient to be tested. Neither a method for determining the efficacy of GPC3-targeting drug therapy for a patient treated with the GPC3-targeting drug therapy nor a method for determining the continuation of GPC3-targeting drug therapy for the patient has been known yet.

References cited herein are as listed below. It should be noted that none of these literatures are admitted to be the prior art to the present invention.

[Citation List]

[Patent Literature]

[Patent Literature 1] WO2003/000883
[Patent Literature 2] WO2006/006693
[Patent Literature 3] WO2006/046751
[Patent Literature 4] WO2007/047291
[Patent Literature 5] WO2009/041062
[Patent Literature 6] WO2009/122667
[Patent Literature 7] WO2004/038420
[Patent Literature 8] WO2009/116659


[Non Patent Literature]

[Non Patent Literature 1] Llovet JM, Burroughs A, Bruix J; Lancet (2003), 362, 1907-17
[Non Patent Literature 2] Bosch FX, Ribes J, Cleries R; Gastroenterology (2004), 127, S5-16
[Non Patent Literature 3] Takenaka K, Kawahara N, Yamamoto K, Kajiyama K, Maeda T, Itasaka H, Shirabe K, Nishizaki T, Yanaga K, Sugimachi K; Arch Surg (1996), 131, 71-6
[Non Patent Literature 4] Yeo W, Mok TS, Zee B, Leung TW, Lai PB, Lau WY, Koh J, Mo FK, Yu SC, Chan AT, Hui P, Ma B, Lam KC, Ho WM, Wong HT, Tang A, Johnson PJ; J Natl Cancer Inst (2005), 97, 1532-8
[Non Patent Literature 5] Llovet J, Ricci S, Mazzaferro V, Hilgard P, Gane E, et al. Sorafenib in advanced hepatocellular carcinoma. New Eng. J. Med. (2008) 359, 378-90
[Non Patent Literature 6] Cheng AL, Chen Z, Tsao CJ, Qin S, Kim JS, et al. Efficacy and safety of sorefanib in patients in the Asia-Pacific region with advanced hepatocellular carcinoma: a phase III randomized, doubleblind, placebo- controlled trial. Lancet Oncol. (2009) 10, 25-34
[Non Patent Literature 7] De Cat B, Muyldermans S-Y, Coomans C, Degeest G, Vanderschueren B, et al. Processing by proprotein convertases is required for glypican-3 modulation of cell survival, Wnt signaling, and gastrulation movements. J. Cell. Biol. (2003) 163, 625-635
[Non Patent Literature 8] Traister A, Shi W and Filmus J. Mammalian Notum induces the release of glypicans and other GPI-anchored proteins from the cell surface. Biochem. J. (2008) 410, 503-511

The following documents are also referred to:

- US2008/0138827 A1 which is concerned with an agent for predicting and judging the recurrence after treating liver cancer;
- JP2007/093274 A which is concerned with a diagnostic method for cancer and a therapeutic agent therefor;
- WO2012/145469 A1 which is concerned with human monoclonal antibodies specific for glypican-3 and use thereof; and
- Mitchell et al (2010) European Journal of Cancer, 47(3): 333-338, which is concerned with Glypican-3: A new target for cancer immunotherapy.

[Summary of Invention]

[Technical Problem]

The present invention has been made in light of the situations as described above, and an object of the present invention is to provide a method for determining the efficacy of GPC3-targeting drug therapy for a patient treated with the GPC3-targeting drug therapy or determining the continuation of GPC3-targeting drug therapy for the patient. Another object of the present invention is to provide a GPC3-targeting drug or a preparation which is to be further administered to a patient for which the efficacy of the GPC3-targeting drug therapy has been determined or the continuation of the GPC3-targeting drug therapy has been determined.

[Solution to Problem]

The present inventors have conducted diligent studies under the situations as described above and consequently created a method comprising monitoring a concentration of free GPC3 in a biological sample isolated from a patient treated with GPC3-targeting drug therapy, wherein when the concentration of free GPC3 is a above predetermined value or when the concentration of free GPC3 has been increased as a result of receiving the GPC3-targeting drug therapy, the efficacy of the GPC3-targeting drug therapy is determined or the continuation of the GPC3-targeting drug therapy is determined. The present inventors have also created a GPC3-targeting drug for use in a method of treating GPC3-expressing cancer which is to be further administered to a patient for which the efficacy of the GPC3-targeting drug therapy has been determined or the continuation of the GPC3-targeting drug therapy has been determined. It has been expected from the previous findings that the concentration of free GPC3 detected in plasma is decreased over time with the continuation of the treatment, if the GPC3-targeting drug therapy has efficacy. Surprisingly, the present inventors have found that the concentration of free GPC3 is stabilized or increased, rather than decreased, in plasma isolated from a patient with stable disease that may respond to the GPC3-targeting drug therapy. The GPC3-targeting drug is an anti-GPC3 antibody.
More specifically, the present invention provides the following aspects:

[1] a method for determining the efficacy of GPC3-targeting drug therapy for GPC3-expressing cancer in a patient or determining the continuation of GPC3-targeting drug therapy for a patient, comprising monitoring a concentration of free GPC3 in a biological sample isolated from the patient before the start of GPC3-targeting drug therapy and/or the patient treated with the GPC3-targeting drug therapy, wherein the GPC3-targeing drug comprises an anti-GPC3 antibody, wherein the biological sample is blood, plasma, or serum, and wherein when the concentration of free GPC3 is above a predetermined value, the GPC3-targeting drug therapy is determined to be effective or the GPC3-targeting drug therapy is determined to be continued.

[2] The method according to [1], wherein:

(a) the predetermined value of free GPC3 is within the range from 0.1 ng/mL to 100 ng/mL;
(b) the concentration of free GPC3 is measured using an immunological method;
(c) the concentration of free GPC3 is larger than that in a biological sample isolated before the start of the GPC3-targeting drug therapy from the patient;
(d) the patient shows high expression of GPC3 in an immunohistochemical staining score;
(e) the cancer is cancer having high expression of GPC31iver cancer; and/or
(f) the GPC3-targeting drug is administered to achieve a blood trough level of 200 μg/ml or higher in the cancer patient.

[3] The method according to [1] or 2, wherein:

(A) the anti-GPC3 antibody has antibody-dependent cellular cytotoxicity (ADCC) activity and/or complement-dependent cytotoxicity (CDC) activity;
(B) the anti-GPC3 antibody is an anti-GPC3 chimeric antibody or a humanized anti-GPC3 antibody comprising any of the following (1) to (5):

(1) heavy chain CDR1, heavy chain CDR2, and heavy chain CDR3 represented by SEQ ID NOs: 4, 5, and 6, respectively, and light chain CDR1, light chain CDR2, and light chain CDR3 represented by SEQ ID NOs: 7, 8, and 9, respectively;
(2) heavy chain CDR1, heavy chain CDR2, and heavy chain CDR3 represented by SEQ ID NOs: 12, 13, and 14, respectively, and light chain CDR1, light chain CDR2, and light chain CDR3 represented by SEQ ID NOs: 15, 16, and 17, respectively;
(3) heavy chain CDR1, heavy chain CDR2, and heavy chainCDR3 represented by SEQ ID NOs: 20, 21, and 22, respectively, and light chain CDR1, light chain CDR2, and light chain CDR3 represented by SEQ ID NOs: 23, 24, and 25, respectively;
(4) heavy chain CDR1, heavy chain CDR2, and heavy chain CDR3 represented by SEQ ID NOs: 28, 29, and 30, respectively, and light chain CDR1, light chain CDR2, and light chain CDR3 represented by SEQ ID NOs: 31, 32, and 33, respectively; and
(5) heavy chain CDR1, heavy chain CDR2, and heavy chain CDR3 represented by SEQ ID NOs: 36, 37, and 38, respectively, and light chain CDR1, light chain CDR2, and light chain CDR3 represented by SEQ ID NOs: 39, 40, and 41, respectively; and/or

(C) the anti-GPC3 antibody comprises any of the following (1) to (6):

(1) a heavy chain variable region selected from the group of heavy chain variable regions represented by SEQ ID NOs: 44, 45, 46, 47, 48, 49, and 50 and a light chain variable region represented by SEQ ID NO: 51;
(2) a heavy chain variable region selected from the group of heavy chain variable regions represented by SEQ ID NOs: 44, 45, 46, 47, 48, 49, and 50 and a light chain variable region selected from the group of light chain variable regions represented by SEQ ID NOs: 52, 53, 54, 55, 56, 57, 58, 59, 60, 61, 62, 63, 64, 65, and 66;
(3) a heavy chain variable region represented by SEQ ID NO:67 and a light chain variable region represented by SEQ ID NO: 68;
(4) a heavy chain variable region represented by SEQ ID NO: 69 and a light chain variable region represented by SEQ ID NO: 70;
(5) a heavy chain variable region represented by SEQ ID NO: 71 and a light chain variable region represented by SEQ ID NO: 72; and
(6) a heavy chain variable region represented by SEQ ID NO: 71 and a light chain variable region represented by SEQ ID NO: 73.

[4] The method according to any one of [1] to [3], wherein the GPC3-targeting drug comprises an anti-GPC3 antibody conjugated with a cytotoxic substance.

[5] The method according to [1] or [2], the cancer is cancer selected from breast cancer, uterine cervix cancer, colon cancer, uterine body cancer, head and neck cancer, lung cancer, malignant glioma, malignant lymphoma, ovary cancer, pancreatic cancer, prostatic cancer, renal cancer, skin cancer, gastric cancer, testicle cancer, thyroid cancer, urothelial cancer.

[6]. A GPC3-targeting drug for use in a method of treating GPC3-expressing cancer, where in the method the method comprises:

(a) performing the method of any one of [1] to [6]; and
(b) administering the GPC3-targeting drug to the patient for which the GPC3-targeting drug has been determined to be effective or that the GPC3-targeting drug has been determined to be continued,

wherein the GPC3-targeting drug comprises an anti-GPC3 antibody.

[Effect of Invention]

According to the present invention, whether GPC3-targeting drug therapy has efficacy or whether GPC3-targeting drug therapy should be continued can be determined conveniently and accurately. This can improve the effects of the GPC3-targeting drug therapy and improve QOL of a patient to be treated. As a result, the better treatment of cancer is achieved.The invention is applied to GPC3-targeting drugs that comprise an anti-GPC3 antibody, with the biological sample employed in the invention being selected from blood, plasma, or serum.

[Brief Description of Drawings]

[FIG. 1A] FIG. 1A is a diagram showing the histochemical staining images of tissues evaluated as having high expression in a staining score of GPC3-IHC (staining method 1). The numeral shown in the upper part of each staining image represents a patient number.
[FIG. 1B] FIG. 1B is a diagram showing the histochemical staining images of tissues evaluated as being negative or having low expression in a staining score of GPC3-IHC (staining method 1). The numeral shown in the upper part of each staining image represents a patient number.
[FIG. 2] FIG. 2 is a diagram showing the durations of GC33 administration to 20 cases. Each cycle involves four doses of GC33 (administered once a week).
[FIG. 3] FIG. 3 is a diagram showing a difference in progression-free survival duration among a patient group from which samples divided into two groups (with a total score of 7 or higher and with a total score lower than 7) according to a staining method based on epitope retrieval using autoclaving were isolated. The solid line represents the progression-free survival duration of the group with a total score of 7 or higher (9 cases). The broken line represents the progression-free survival duration of the group with a total score lower than 7 (7 cases). The hazard ratio of the group with a total score of 7 or higher to the group with a total score lower than 7 was 0.376 (95% confidence interval: 0.116-1.227, p = 0.0852).
[FIG. 4A] FIG. 4A is a diagram showing the correlation between the concentration of free GPC3 detected in serum and the GPC3-IHC score of tumor tissues, in a group evaluated as having high expression of GPC3. The ordinate shows the serum concentration (ng/mL) of free GPC3. The abscissa shows the number of lapsed days (day) after the start of GPC3-targeting drug therapy.
[FIG. 4B] FIG. 4B is a diagram showing the correlation between the concentration of free GPC3 detected in serum and the GPC3-IHC score of tumor tissues, in a group evaluated as having low expression of GPC3 or being negative. The ordinate shows the serum concentration (ng/mL) of free GPC3. The abscissa shows the number of lapsed days (day) after the start of GPC3-targeting drug therapy.
[FIG. 5A] FIG. 5A is a diagram showing the correlation between the concentration of free GPC3 in serum isolated from serum collected from patients before the start of GPC3-targeting drug therapy and the progression-free survival duration of the patients. The ordinate shows a survival rate. The abscissa shows progression-free survival duration (day) after the start of GPC3-targeting drug therapy. The solid line represents the progression-free survival duration of a group having a measurable level of free GPC3 (6 cases). The broken line represents the progression-free survival duration of a group having a GPC3 level below the measurement limit (0.4 ng/mL) (14 cases).
[FIG. 5B] FIG. 5B is a diagram showing the correlation between the concentration of free GPC3 in serum isolated from serum collected from patients during a test period (including before and after the start of GPC3-targeting drug therapy) and the progression-free survival duration of the patients. The ordinate shows a survival rate. The abscissa shows progression-free survival duration (day) after the start of GPC3-targeting drug therapy. The solid line represents the progression-free survival duration of a group having a measurable level of free GPC3 (9 cases) in serum isolated from serum collected from the patients before or during GPC3-targeting drug therapy. The broken line represents the progression-free survival duration of a group having a GPC3 level below the measurement limit (0.4 ng/mL) (both before and after the start of GPC3-targeting drug therapy) (11 cases) in serum isolated from serum collected from the patients treated with the therapy.

[FIG. 6A] FIG. 6A is a diagram showing the correlation between the concentration of free GPC3 in serum isolated from serum collected from patients before the start of GPC3-targeting drug therapy and the progression-free survival duration of the patients. The ordinate shows a survival rate. The abscissa shows progression-free survival duration (day) after the start of GPC3-targeting drug therapy. The solid line represents the progression-free survival duration of a group having a measurable level of free GPC3 (8 cases). The broken line represents the progression-free survival duration of a group having a GPC3 level below the measurement limit (0.4 ng/mL) (19 cases). The hazard ratio of the group with a detectable level of GPC3 to the group with a GPC3 level below the detection limit was 0.265 (95% confidence interval: 0.077-0.914, p = 0.0219).

[FIG. 6B] FIG. 6B is a diagram showing the correlation between the concentration of free GPC3 in serum isolated from serum collected from patients during a test period (including before and after the start of GPC3-targeting drug therapy) and the progression-free survival duration of the patients. The ordinate shows a survival rate. The abscissa shows progression-free survival duration (day) after the start of GPC3-targeting drug therapy. The solid line represents the progression-free survival duration of a group having a measurable level of free GPC3 (13 cases) in serum isolated from serum collected from the patients before or during GPC3-targeting drug therapy. The broken line represents the progression-free survival duration of a group having a GPC3 level below the measurement limit (0.4 ng/mL) (both before and after the start of GPC3-targeting drug therapy) (14 cases) in serum isolated from serum collected from the patients treated with the therapy. The hazard ratio of the group with a detectable level of GPC3 to the group with a GPC3 level below the detection limit was 0.283 (95% confidence interval: 0.112-0.715, p = 0.0038).

[FIG. 7A] FIG. 7A is a diagram showing the correlation between the serum concentration of free GPC3 isolated from serum collected from patients before the start of GPC3-targeting drug therapy and the progression-free survival duration of the patients in a group with the serum concentration of free GPC3 lower than the median value (1129.7 pg/mL). The solid line represents the progression-free survival duration of a placebo group (34 cases). The broken line represents the progression-free survival duration of a group with a putative trough level of GC33 lower than the median value (low-GC33-exposed group: 19 cases). The dotted line represents the progression-free survival duration of a group with a putative trough level of GC33 equal to or higher than the median value (high-GC33-exposed group: 34 cases). The median value of the progression-free survival duration was 83 days for the placebo group, 43.5 days for the low-GC33-exposed group, and 124 days for the high-GC33-exposed group. The hazard ratio of the high-GC33-exposed group to the placebo group was 0.803 (p = 0.397), whereas the hazard ratio of the high-GC33-exposed group to the low-GC33-exposed group was 0.425 (p = 0.010).

[FIG. 7B] FIG. 7B is a diagram showing the correlation between the serum concentration of free GPC3 isolated from serum collected from patients before the start of GPC3-targeting drug therapy and the progression-free survival duration of the patients in a group with the serum concentration of free GPC3 equal to or higher than the median value (1129.7 pg/mL). The solid line represents the progression-free survival duration of a placebo group (24 cases). The broken line represents the progression-free survival duration of a low-GC33-exposed group (40 cases). The dotted line represents the progression-free survival duration of a high-GC33-exposed group (24 cases). The median value of the progression-free survival duration was 44 days for the placebo group, 46.5 days for the low-GC33-exposed group, and 87 days for the high-GC33-exposed group. The hazard ratio of the high-GC33-exposed group to the placebo group was 0.510 (p = 0.036), whereas the hazard ratio of the high-GC33-exposed group to the low-GC33-exposed group was 0.572 (p = 0.056).

[FIG. 7C] FIG. 7C is a diagram showing the correlation between the serum concentration of free GPC3 isolated from serum collected from patients before the start of GPC3-targeting drug therapy and the overall survival duration of the patients in a group with the serum concentration of free GPC3 lower than the median value (1129.7 pg/mL). The solid line represents the overall survival duration of a placebo group (34 cases). The broken line represents the overall survival duration of a low-GC33-exposed group (19 cases). The dotted line represents the overall survival duration of a high-GC33-exposed group (34 cases). The median value of the overall survival duration was 203 days for the placebo group, 86 days for the low-GC33-exposed group, and 295 days for the high-GC33-exposed group. The hazard ratio of the high-GC33-exposed group to the placebo group was 0.590 (p = 0.200), whereas the hazard ratio of the high-GC33-exposed group to the low-GC33-exposed group was 0.329 (p = 0.008).

[FIG. 7D] FIG. 7D is a diagram showing the correlation between the serum concentration of free GPC3 isolated from serum collected from patients before the start of GPC3-targeting drug therapy and the overall survival duration of the patients in a group with the serum concentration of free GPC3 equal to or higher than the median value (1129.7 pg/mL). The solid line represents the overall survival duration of a placebo group (24 cases). The broken line represents the overall survival duration of a low-GC33-exposed group (40 cases). The dotted line represents the overall survival duration of a high-GC33-exposed group (24 cases). The median value of the overall survival duration was 121 days for the placebo group, 177 days for the low-GC33-exposed group, and 308 days for the high-GC33-exposed group. The hazard ratio of the high-GC33-exposed group to the placebo group was 0.303 (p = 0.005), whereas the hazard ratio of the high-GC33-exposed group to the low-GC33-exposed group was 0.280 (p = 0.002).

[FIG. 7E] FIG. 7E is a diagram showing the correlation between the serum concentration of free GPC3 isolated from serum collected from patients before the start of GPC3-targeting drug therapy and the progression-free survival duration of the patients in a group with the serum concentration of free GPC3 higher than 175 pg/mL. The solid line represents the progression-free survival duration of a placebo group (51 cases). The broken line represents the progression-free survival duration of a low-GC33-exposed group (56 cases). The dotted line represents the progression-free survival duration of a high-GC33-exposed group (47 cases). The median value of the progression-free survival duration was 51 days for the placebo group, 45 days for the low-GC33-exposed group, and 124 days for the high-GC33-exposed group. The hazard ratio of the high-GC33-exposed group to the placebo group was 0.597 (p = 0.0184), whereas the hazard ratio of the high-GC33-exposed group to the low-GC33-exposed group was 0.439 (p = 0.0003).

[FIG. 7F] FIG. 7F is a diagram showing the correlation between the serum concentration of free GPC3 isolated from serum collected from patients before the start of GPC3-targeting drug therapy and the overall survival duration of the patients in a group with the serum concentration of free GPC3 higher than 175 pg/mL. The solid line represents the overall survival duration of a placebo group (51 cases). The broken line represents the overall survival duration of a low-GC33-exposed group (56 cases). The dotted line represents the overall survival duration of a high-GC33-exposed group (47 cases). The median value of the overall survival duration was 203 days for the placebo group, 141 days for the low-GC33-exposed group, and 308 days for the high-GC33-exposed group. The hazard ratio of the high-GC33-exposed group to the placebo group was 0.402 (p = 0.0037), whereas the hazard ratio of the high-GC33-exposed group to the low-GC33-exposed group was 0.238 (p = <0.0001).

[FIG. 8A] FIG. 8A is a diagram showing the correlation between the serum concentration of free GPC3 isolated from serum collected from patients before the start of GPC3-targeting drug therapy and the progression-free survival duration of the patients in a group with the serum concentration of free GPC3 lower than the median value (1161.5 pg/mL). The solid line represents the progression-free survival duration of a placebo group (31 cases). The broken line represents the progression-free survival duration of a low-GC33-exposed group (20 cases). The dotted line represents the progression-free survival duration of a high-GC33-exposed group (36 cases). The median value of the progression-free survival duration was 82 days for the placebo group, 43 days for the low-GC33-exposed group, and 124 days for the high-GC33-exposed group. The hazard ratio of the high-GC33-exposed group to the placebo group was 0.713 (p = 0.197), whereas the hazard ratio of the high-GC33-exposed group to the low-GC33-exposed group was 0.392 (p = 0.004).

[FIG. 8B] FIG. 8B is a diagram showing the correlation between the serum concentration of free GPC3 isolated from serum collected from patients before the start of GPC3-targeting drug therapy and the progression-free survival duration of the patients in a group with the serum concentration of free GPC3 equal to or higher than the median value (1161.5 pg/mL). The solid line represents the progression-free survival duration of a placebo group (27 cases). The broken line represents the progression-free survival duration of a low-GC33-exposed group (39 cases). The dotted line represents the progression-free survival duration of a high-GC33-exposed group (22 cases). The median value of the progression-free survival duration was 45 days for the placebo group, 47 days for the low-GC33-exposed group, and 87 days for the high-GC33-exposed group. The hazard ratio of the high-GC33-exposed group to the placebo group was 0.588 (p = 0.092), whereas the hazard ratio of the high-GC33-exposed group to the low-GC33-exposed group was 0.626 (p = 0.116).

[FIG. 8C] FIG. 8C is a diagram showing the correlation between the serum concentration of free GPC3 isolated from serum collected from patients before the start of GPC3-targeting drug therapy and the overall survival duration of the patients in a group with the serum concentration of free GPC3 lower than the median value (1161.5 pg/mL). The solid line represents the overall survival duration of a placebo group (31 cases). The broken line represents the overall survival duration of a low-GC33-exposed group (20 cases). The dotted line represents the overall survival duration of a high-GC33-exposed group (36 cases). The median value of the overall survival duration was 203 days for the placebo group, 86 days for the low-GC33-exposed group, and 295 days for the high-GC33-exposed group. The hazard ratio of the high-GC33-exposed group to the placebo group was 0.508 (p = 0.100), whereas the hazard ratio of the high-GC33-exposed group to the low-GC33-exposed group was 0.287 (p = 0.002).

[FIG. 8D] FIG. 8D is a diagram showing the correlation between the serum concentration of free GPC3 isolated from serum collected from patients before the start of GPC3-targeting drug therapy and the overall survival duration of the patients in a group with the serum concentration of free GPC3 equal to or higher than the median value (1161.5 pg/mL). The solid line represents the overall survival duration of a placebo group (27 cases). The broken line represents the overall survival duration of a low-GC33-exposed group (39 cases). The dotted line represents the overall survival duration of a high-GC33-exposed group (22 cases). The median value of the overall survival duration was 176 days for the placebo group, 177 days for the low-GC33-exposed group, and 291 days for the high-GC33-exposed group. The hazard ratio of the high-GC33-exposed group to the placebo group was 0.300 (p = 0.022), whereas the hazard ratio of the high-GC33-exposed group to the low-GC33-exposed group was 0.324 (p = 0.005).

[FIG. 8E] FIG. 8E is a diagram showing the correlation between the serum concentration of free GPC3 isolated from serum collected from patients before the start of GPC3-targeting drug therapy and the progression-free survival duration of the patients in a group with the serum concentration of free GPC3 higher than 259.7 pg/mL. The solid line represents the progression-free survival duration of a placebo group (50 cases). The broken line represents the progression-free survival duration of a low-GC33-exposed group (55 cases). The dotted line represents the progression-free survival duration of a high-GC33-exposed group (47 cases). The median value of the progression-free survival duration was 46.5 days for the placebo group, 45.5 days for the low-GC33-exposed group, and 124 days for the high-GC33-exposed group. The hazard ratio of the high-GC33-exposed group to the placebo group was 0.567 (p = 0.010), whereas the hazard ratio of the high-GC33-exposed group to the low-GC33-exposed group was 0.467 (p = 0.0009).

[FIG. 8F] FIG. 8F is a diagram showing the correlation between the serum concentration of free GPC3 isolated from serum collected from patients before the start of GPC3-targeting drug therapy and the overall survival duration of the patients in a group with the serum concentration of free GPC3 higher than 259.7 pg/mL. The solid line represents the overall survival duration of a placebo group (50 cases). The broken line represents the overall survival duration of a low-GC33-exposed group (55 cases). The dotted line represents the overall survival duration of a high-GC33-exposed group (47 cases). The median value of the overall survival duration was 185 days for the placebo group, 156 days for the low-GC33-exposed group, and 308 days for the high-GC33-exposed group. The hazard ratio of the high-GC33-exposed group to the placebo group was 0.414 (p = 0.0043), whereas the hazard ratio of the high-GC33-exposed group to the low-GC33-exposed group was 0.304 (p = <0.0001).

[Deleted]

[Description of Embodiments]

Definition

Chemical terms and technical terms used in relation to the present invention have meanings generally understood by those skilled in the art, unless otherwise defined herein.

Indefinite article

In the present invention, the indefinite articles "a" and "an" refer to one or two or more (i.e., at least one) object(s) grammatically represented by the indefinite articles. For example, "a factor" means one factor or two or more factors.

Amino acid

Each amino acid is indicated herein by single-letter code or three-letter code, or both, as represented by, for example, Ala/A, Leu/L, Arg/R, Lys/K, Asn/N, Met/M, Asp/D, Phe/F, Cys/C, Pro/P, Gln/Q, Ser/S, Glu/E, Thr/T, Gly/G, Trp/W, His/H, Tyr/Y, Ile/I, and Val/V.

Amino acid modification

An amino acid in the amino acid sequence of an antigen-binding molecule can be modified by an appropriately adopted method known in the art such as site-directed mutagenesis (Kunkel et al., Proc. Natl. Acad. Sci. USA (1985) 82, 488-492) or overlap extension PCR. Also, a plurality of methods known in the art can be adopted as methods for modifying an amino acid to substitute the amino acid by an amino acid other than natural one (Annu. Rev. Biophys. Biomol. Struct. (2006) 35, 225-249; and Proc. Natl. Acad. Sci. U.S.A. (2003) 100 (11), 6353-6357). For example, a tRNA-containing cellfree translation system (Clover Direct (Protein Express, an R & D oriented company)) comprising a non-natural amino acid bound with an amber suppressor tRNA complementary to UAG codon (amber codon), which is a stop codon, is also preferably used.

The term "and/or" used herein to represent amino acid modification sites is meant to include every combination appropriately represented by "and" and "or". Specifically, for example, the phrase "amino acids 43, 52, and/or 105 are substituted" includes the following variations of amino acid modification:

(a) position 43, (b) position 52, (c) position 105, (d) positions 43 and 52, (e) positions 43 and 105, (f) positions 52 and 105, and (g) positions 43, 52, and 105.

EU numbering and Kabat numbering

According to a method used in the present invention, amino acid positions assigned to antibody CDRs and FRs are defined by the Kabat method (Sequences of Proteins of Immunological Interest, National Institute of Health, Bethesda, Md., 1987 and 1991). When the antigen-binding molecule described herein is an antibody or an antigen-binding fragment, amino acids in variable and constant regions are indicated according to the Kabat numbering and the EU numbering conforming to the Kabat amino acid positions, respectively.

Biological sample

The term "biological sample" refers to a sample of a tissue or a fluid isolated from a subject. Examples of such samples include plasma, serum, spinal fluid, lymph, external sections of skin, respiratory tract, intestinal tract, and genitourinary tract, tear, saliva, sputum, milk, whole blood or any blood fraction, blood derivatives, blood cells, tumor, nervous tissues, organs or any type of tissue, any sample obtained by lavage (e.g., samples derived from the bronchi), and samples of components constituting cell cultures *in vitro.* In the present invention, the biological sample is blood, plasma, or serum

The concentration of free GPC3 is measured in a biological sample isolated from a patient. The concentration of free GPC3 may be measured in, for example, a whole blood sample or a blood fraction (e.g., serum or plasma) sample (also referred to as a whole blood sample, a serum sample, or a plasma sample, respectively, herein). In a non-limiting aspect, the concentration of free GPC3 in a whole blood sample, a serum sample, or a plasma sample from a patient can be measured using, for example, commercially available Human Glypican-3 ELISA kit (BioMosaics Inc.) or Enzyme-linked Immunosorbent Assay Kit For Glypican 3 (GPC3) (USCN Life Science Inc.) and the whole blood sample, the serum sample, or the plasma sample treated with EDTA.

The term "isolated" refers to causing "artificial" change from a natural state, i.e., shifting and/or removing a naturally occurring substance from its original environment. In the present invention, the term "isolated" means that, for example, a polynucleotide or a polypeptide present in an organism is unisolated, whereas the same polynucleotide or polypeptide thereas is isolated when separated from a material present with the polynucleotide or the polypeptide in a natural state. A polynucleotide or a polypeptide introduced into an organism by transformation, genetic manipulation, or any other recombination method is in an isolated state even when present in the organism (regardless of being alive or dead).

Free GPC3

In the present invention, the term "free GPC3" refers to GPC3 unanchored to GPC3-expressing cells and includes fragments of secretory GPC3 that can be easily dissociated from GPC3 anchored to GPC3-expressing cells under particular conditions *in vivo* or *in vitro.* In a non-limiting aspect, examples of the "free GPC3" can include a polypeptide from the amino terminus to position 358 in GPC3 consisting of the polypeptide defined by SEQ ID NO: 1, a polypeptide from the amino terminus to position 374 in GPC3 consisting of the polypeptide defined by SEQ ID NO: 1, a GPC3 polypeptide liberated by the degradation of a GPI anchor present at the carboxy terminus, and their fragments (Patent Literature 7). Those skilled in the art can appropriately select an approach known in the art for determining the structure of free GPC3. In a non-limiting aspect, a method therefor that may be appropriately used involves, for example, directly detecting free GPC3 present in the serum or the plasma of a patient or a model animal by the method described in Patent Literature 7 and analyzing its structure or involves, for example, allowing an enzyme dissociating free GPC3, such as convertase, phospholipase D, or Notum, to act on GPC3 expressed in cells cultured *in vitro,* detecting the resulting free GPC3, and analyzing its structure (e.g., J. Cell. Biol. (2003) 163 (3), 625-635).

Method for measuring concentration of free GPC3

The concentration of free GPC3 can be measured by one or more methods selected from the group consisting of the following: spectroscopic methods such as nuclear magnetic resonance (NMR) and mass spectrometry (MS); and SELDI(-TOF), MALDI(-TOF), 1D gel-based analysis, 2D gel-based analysis, liquid chromatography (e.g., highpressure liquid chromatography (HPLC) or low-pressure liquid chromatography (LPLC)), thin-layer chromatography, and LC-MS-based techniques. Examples of appropriate LCMS techniques can include ICAT(R) (Applied Biosystems, Inc.) and iTRAQ(R) (Applied Biosystems, Inc.). Also, a method which involves detecting a further fragment of free GPC3 further digested with an appropriate enzyme may be appropriately adopted.

The assay of free GPC3 can be carried out by a direct or indirect detection method. Free GPC3 can be detected directly or indirectly via the interaction of a ligand or a ligand group with, for example, an enzyme, a bond, a receptor or a transport protein, an antibody, a peptide, an aptamer or an oligonucleotide, or an arbitrary synthetic chemical receptor or compound capable of specifically binding to free GPC3. The ligand may be modified with a detectable label such as a luminescent label, a fluorescent label, or a radioactive label, and/or an affinity tag.

Immunological method

Examples of preferred methods for assaying free GPC3 can include immunological methods using an antibody capable of binding to an epitope present in GPC3. Examples of the immunological methods include enzyme immunoassay (ELISA or EIA), fluorescence immunoassay (FIA), radioimmunoassay (RIA), luminescence immunoassay (LIA), immunoenzymatic technique, fluorescent antibody technique, immunochromatography, immunoturbidimetry, latex turbidimetry, and latex agglutination assay. In the immunological method of the present invention, free GPC3 may be assayed by procedures of manual operation or using an apparatus such as an analyzer.

The immunological method of the present invention can be performed according to, for example, a method known in the art such as sandwich technique. For example, a primary antibody immobilized on a carrier, a biological sample, and a secondary antibody modified with a labeling material are reacted simultaneously or in order. This reaction forms a complex of the primary antibody immobilized on a carrier, free GPC3, and the secondary antibody modified with a labeling material. The labeling material conjugated with the secondary antibody contained in this complex can be quantified to thereby measure the amount (concentration) of the free GPC3 contained in the biological sample.

In the case of, for example, the enzyme immunoassay, a primary antibody-immobilized microplate, serially diluted biological samples, a secondary antibody modified with an enzyme such as HRP, a washing buffer, and a solution containing a substrate reactive with the enzyme such as HRP are preferably used. In a non-limiting aspect of assay, the enzyme modifying the secondary antibody is reacted under the optimum conditions thereof with the substrate. The amount of the resulting enzymatic reaction product can be measured by an optical method or the like. In the case of the fluorescence immunoassay, a primary antibody-immobilized optical waveguide, serially diluted biological samples, a secondary antibody modified with a fluorescent material, and a washing buffer may be preferably used. In a non-limiting aspect of assay, the fluorescent material modifying the secondary antibody can be irradiated with excitation light to thereby emit fluorescence, the intensity of which is then measured.

The radioimmunoassay involves measuring the amount of radiation from a radioactive substance. The luminescence immunoassay involves measuring luminescence intensity derived from a luminescent reaction system. For example, the immunoturbidimetry, the latex turbidimetry, or the latex agglutination assay involves measuring transmitted light or scattering light by an endpoint or rate method. The immunochromatography, for example, which is based on visual observation, involves visually measuring the color of the labeling material appearing on a test line. Alternatively, an instrument such as an analyzer may be appropriately used instead of this visual measurement.

In the immunological method of the present invention, the primary antibody for immobilization on a carrier can be adsorbed or bound to the carrier by a method such as physical adsorption, chemical binding, or a combination thereof. A method known in the art may be appropriately used for immobilizing the antibody by physical adsorption. Examples of the method include a method which involves contacting the antibody with the carrier by mixing in a solution such as a buffer solution, and a method which involves contacting the antibody dissolved in a buffer or the like with the carrier. Alternatively, the antibody may be immobilized onto the carrier by chemical binding. Examples of the method include a method which involves contacting the antibody and the carrier by mixing with a divalent cross-linking reagent such as glutaraldehyde, carbodiimide, imide ester, or maleimide to react the reagent with amino groups, carboxyl groups, thiol groups, aldehyde groups, or hydroxy groups in both the antibody and the carrier. Such immobilization may require treatment for suppressing nonspecific reaction or the natural aggregation or the like of the antibody-immobilized carrier. In such a case, the aftertreatment of the immobilization can be performed by a method known in the art. Examples of the method include a method which involves coating the surface or inner wall of the antibody-immobilized carrier by contacting with, for example, a protein (e.g., bovine serum albumin (BSA), casein, gelatin, egg albumin, or a salt thereof), a surfactant, or a skimmed milk.

In the immunological method of the present invention, the secondary antibody for modification with a labeling material can be adsorbed or bound to the labeling material by a method such as physical adsorption, chemical binding, or a combination thereof. A method known in the art may be appropriately used for binding the antibody to the labeling material by physical adsorption. Examples of the method include a method which involves contacting the antibody with the labeling material by mixing in a solution such as a buffer solution, and a method which involves contacting the antibody dissolved in a buffer or the like with the labeling material. When the labeling material is, for example, gold colloid or latex, the physical adsorption method is effective. The antibody can be mixed and contacted with the gold colloid in a buffer to obtain a gold colloid-labeled antibody. Alternatively, the antibody may be modified with the labeling material by chemical binding. Examples of the method include a method which involves contacting the antibody and the labeling material by mixing with a divalent cross-linking reagent such as glutaraldehyde, carbodiimide, imide ester, or maleimide to react the reagent with amino groups, carboxyl groups, thiol groups, aldehyde groups, or hydroxy groups in both the antibody and the labeling material. When the labeling material is, for example, a fluorescent material, an enzyme, or a chemiluminescent material, the chemical binding method is effective. Such modification may require treatment for suppressing nonspecific reaction or the natural aggregation or the like of the antibody modified with the labeling material. In such a case, the aftertreatment of the labeling can be performed by a method known in the art. Examples of the method include a method which involves coating the labeling material-bound antibody by contacting with, for example, a protein (e.g., bovine serum albumin (BSA), casein, gelatin, egg albumin, or a salt thereof), a surfactant, or a skimmed milk.

For example, peroxidase (POD), alkaline phosphatase (ALP), β-galactosidase, urease, catalase, glucose oxidase, lactate dehydrogenase, or amylase can be used as the labeling material for enzyme immunoassay. For example, fluorescein isothiocyanate, tetramethylrhodamine isothiocyanate, substituted rhodamine isothiocyanate, dichlorotriazine isothiocyanate, cyanine, or merocyanine can be used for fluorescence immunoassay. For example, tritium, iodine 125, or iodine 131 can be used for radioimmunoassay. For example, a luminol system, a luciferase system, an acridinium ester system, or a dioxetane compound system can be used for luminescence immunoassay. Alternatively, fine particles made of a material such as polystyrene, a styrene-styrene sulfonate copolymer, an acrylonitrile-butadiene-styrene copolymer, a vinyl chloride-acrylic acid ester copolymer, a vinyl acetate-acrylic acid copolymer, polyacrolein, a styrenemethacrylic acid copolymer, a styrene-glycidyl (meth)acrylate copolymer, a styrene-butadiene copolymer, a methacrylic acid polymer, an acrylic acid polymer, latex, gelatin, liposome, a microcapsule, silica, alumina, carbon black, a metal compound, a metal, a metal colloid, a ceramic, or a magnetic substance can be used for immunochromatography, immunoturbidimetry, latex turbidimetry, or latex agglutination assay.

A solid-phase carrier in the form of, for example, beads, a microplate, a test tube, a stick, a membrane, or a test pieces made of a material such as polystyrene, polycarbonate, polyvinyltoluene, polypropylene, polyethylene, polyvinyl chloride, nylon, polymethacrylate, polyacrylamide, latex, liposome, gelatin, agarose, cellulose, Sepharose, glass, a metal, a ceramic, or a magnetic substance can be appropriately used as the carrier in the immunological method of the present invention.

Also disclosed is an assay kit comprising components for use in the immunological method of the present invention. The assay kit comprises at least one type of antibody capable of binding to an epitope present in GPC3. The antibody may be provided in a state immobilized on the carrier mentioned above or may be provided independently of the carrier. The kit may additionally comprise standard solutions of serially diluted free GPC3. The assay kit may further comprise at least one type of antibody capable of binding to an epitope different from that present in GPC3. Assay principles, etc., for use in the immunoassay kit of the present invention are the same as in the immunological method mentioned above. In the immunoassay kit, various aqueous solvents may be used. Examples of the aqueous solvents include purified water, saline, and various buffers such as tris buffers, phosphate buffers, and phosphate-buffered saline. The pH of this buffer can be appropriately selected from among suitable pHs. The pH value used is not limited and is generally selected within the range of pH 3 to 12.

The immunoassay kit may further appropriately contain, in addition to the components mentioned above, one or two or more components selected from proteins (e.g., bovine serum albumin (BSA), human serum albumin (HSA), casein, and salts thereof), various salts, various sugars, skimmed milk, various animal sera (e.g., normal rabbit serum), various antiseptics (e.g., sodium azide and antibiotics), activating substances, reactionpromoting substances, sensitivity-increasing substances (e.g., polyethylene glycol), nonspecific reactioninhibiting substances, and various surfactants such as nonionic surfactants, amphoteric surfactants, and anionic surfactants. The concentrations of these components contained in the assay reagent are not limited and are preferably 0.001 to 10% (W/V). Particularly preferred concentrations are appropriately selected within the range of 0.01 to 5% (W/V).

The immunoassay kit may be further combined with other reagents, in addition to the components mentioned above. Examples of these other reagents include buffers, diluting solutions for biological samples, reagent diluting solutions, reagents containing labeling materials, reagents containing substances that generate signals such as color, reagents containing substances involved in the generation of signals such as color, reagents containing substances for calibration, and reagents containing substances for accuracy control.

The immunoassay kit can have any form without limitations and may be provided as an integral-type diagnostic kit comprising all of the components constituting the immunoassay kit in order to carry out assay conveniently in a short time. Examples of the integral-type diagnostic kit include ELISA kits, fluorescence immunoassay kits, and immunochromatography kits. The ELISA kit form comprises, for example, a primary antibody-immobilized microplate, standard solutions of serially diluted free GPC3, a secondary antibody modified with an enzyme such as HRP, a washing buffer, and a substrate solution for the enzymatic reaction. The fluorescence immunoassay kit comprises, for example, a primary antibody-immobilized optical waveguide, standard solutions of serially diluted free GPC3, a secondary antibody modified with a fluorescent material, and a washing buffer. The immunochromatography kit comprises a membrane housed in a reaction cassette. In one exemplary aspect, the primary antibody is immobilized at one end (downstream) of the membrane; a developing solution is placed at the other end (upstream) of the membrane; a pad supplemented with a substrate for the labeling agent is disposed in proximity (downstream) to the developing solution; and a pad supplemented with the secondary antibody labeled as described above is disposed in the central part of the membrane.

Examples of biological samples used for detecting the expression level of GPC3 in tissues include test subject-derived preparations. The test subject-derived preparation is preferably a tissue obtained from the test subject, more preferably a liver cancer or hepatocellular cancer tissue of the test subject. The liver cancer or hepatocellular cancer tissue is collected preferably using a biopsy method known in the art. The liver biopsy refers to a method of directly inserting a thin long needle into the liver from skin surface and collecting liver tissues. The needling site is typically the intercostal space of the right lower chest. The safety of the needling site is confirmed before operation using an ultrasonic examination apparatus. Then, the needling site is disinfected. A region from the skin to the surface of the liver is subjected to anesthesia. After small incision of the skin at the needling site, a puncture needle is inserted thereto.

For microscopic observation by transmitted beams, the tissue preparation is sliced to a degree that allows beams of light for use in the microscope to sufficiently penetrate the tissue slice. At a stage prior to the slicing, the tissue preparation is fixed. Specifically, proteins in tissues or cells are coagulated by dehydration or denaturation to thereby rapidly kill the cells constituting the tissues. The resulting structure is stabilized and insolubilized. First, the tissue preparation to be fixed is cut into a fragment with a size and a shape suitable for the preparation of paraffin-embedded sections by use of a knife such as a surgical knife. Subsequently, the fragment is dipped in a fixative, which is a reagent used for carrying out fixation. Formalin, more preferably neutral buffered formalin, is preferably used as the fixative. The concentration of the neutral buffered formalin is appropriately selected according to the characteristics or physical properties of the tissue preparation. The concentration used may be appropriately changed between 1 and 50%, preferably 5 and 25%, more preferably 10 and 15%. The fixative with the tissue preparation dipped therein is appropriately degassed using a vacuum pump. For fixation, the tissue preparation is left for several hours in the fixative under conditions of ordinary pressure and room temperature. The time required for the fixation can be appropriately selected within the range of 1 hour to 7 days, preferably 2 hours to 3 days, more preferably 3 hours to 24 hours, further preferably 4 hours to 16 hours. The tissue preparation thus fixed is appropriately dipped in a phosphate buffer solution or the like for additional several hours (which can be appropriately selected within the range of 2 hours to 48 hours, preferably 3 hours to 24 hours, more preferably 4 hours to 16 hours).

Next, sections can be preferably prepared by freeze sectioning or paraffin sectioning from the tissue preparation thus fixed. Preferred examples of the freeze sectioning include a method which involves adding tissues into O.C.T. Compound (Miles Inc.), freezing the mixture, and slicing the frozen mixture using a cryostat (frozen section preparation apparatus). In the paraffin sectioning, the fixed tissue preparation is dipped in an embedding agent, which is then solidified to thereby impart thereto uniform and appropriate hardness. Paraffin can be preferably used as the embedding agent. The fixed tissue preparation is dehydrated using ethanol. Specifically, the tissue preparation is dipped in 70% ethanol, 80% ethanol, and 100% ethanol in this order and thereby dehydrated. The time required for the dipping and the number of runs can be appropriately selected within the ranges of 1 hour to several days and 1 to 3 times, respectively. The tissue preparation may be dipped therein at room temperature or 4°C. In the case of dipping at 4°C, a longer dipping time (e.g., overnight) is more preferred. After replacement of the liquid phase with xylene, the tissue preparation is embedded in paraffin. The time required for the replacement of the liquid phase with xylene can be appropriately selected within the range of 1 hour to several hours. This replacement may be performed at room temperature or 4°C. In the case of replacement at 4°C, a longer replacement time (e.g., overnight) is more preferred. The time required for the embedding in paraffin and the number of runs can be appropriately selected within the ranges of 1 hour to several hours and 1 to 4 times, respectively. This embedding may be performed at room temperature or 4°C. In the case of embedding at 4°C, a longer embedding time (e.g., overnight) is more preferred. Alternatively, the tissue preparation may be preferably embedded in paraffin using paraffin embedding apparatus (EG1160, Leica, etc.) that automatically performs paraffin embedding reaction.

The tissue preparation thus paraffin-embedded is bonded to a block base to prepare a "block". This block is sliced into the desired thickness selected from thicknesses of 1 to 20 μm by use of a microtome. The sliced tissue section is left standing on a glass slide as a permeable support and thereby fixed thereon. In this case, the glass slide coated with 0.01% poly-L-lysine (Sigma-Aldrich Corp.) and then dried may be preferably used in order to prevent the tissue section from coming off. The fixed tissue section is dried in air for an appropriate time selected from between several minutes and 1 hour.

Epitope retrieval

An epitope in an antigen whose reactivity with an antibody has been attenuated due to formalin fixation is retrieved. Protease-induced epitope retrieval (PIER) or heat-induced epitope retrieval (HIER) may be applied to the retrieval. In a non-limiting instance, PIER may be applied to one of "two identifiable tissue preparations" prepared as shown below, while HIER may be applied to the other preparation. In this case, a difference in the degree of staining between these preparations reacted with antibodies can be digitized.

In a non-limiting instance, a set of two tissue preparations is prepared, which are prepared as shown in the paragraph "Biological sample" and attached onto permeable supports. The tissue preparations are desirably two histologically identifiable tissue preparations. The term "identifiable" means that two tissue preparations to be mutually compared are composed of substantially the same cells or tissues in test subject-derived preparations serving as origins of the tissue preparations. For example, two tissue preparations prepared as adjacent sections correspond to two identifiable tissue preparations. The "two identifiable tissue preparations" refer to two tissue preparations prepared as adjacent sections, unless otherwise specified. In addition, two tissue preparations composed of cells or tissues structurally identifiable between the preparations correspond to "two identifiable tissue preparations", even if the tissue preparations are not prepared as adjacent sections. Preferred examples of such two tissue preparations composed of cells or tissues structurally identifiable therebetween include (1) tissue sections containing cells derived from the same cells at the same positions on plane coordinates in the sections, and (2) tissue sections in which at least 50% or more, preferably 60% or more, more preferably 70% or more, further preferably 80% or more, still further preferably 90% or more, particularly preferably 95% or more of the cells are present at the same positions on the plane coordinates.

The heat-induced epitope retrieval appropriately employs, for example, a heating method using microwave, a heating method using an autoclave, or a heating method using boiling treatment. In the case of boiling treatment at an output of 780 W so as to keep a liquid temperature at approximately 98°C, the time required for the retrieval including the treatment is appropriately selected from between 5 minutes and 60 minutes and is, for example, 10 minutes. The epitope retrieval treatment can be performed in a 10 mM sodium citrate buffer solution as well as commercially available Target Retrieval Solution (DakoCytomation), for example. Target Retrieval Solution is used in Examples described below. Any buffer solution or aqueous solution is preferably used as long as an epitope in the antigen that is recognized by an anti-GPC3 antibody acquires the ability to bind to the antibody as a result of the retrieval treatment so that an antigen-antibody complex mentioned later can be detected.

The protease for use in the protease-induced epitope retrieval is not limited by its type or origin. Generally available protease can be appropriately selected for use. Preferred examples of the protease used include pepsin with 0.05% concentration in 0.01 N hydrochloric acid, trypsin with 0.1% concentration further containing $CaCl_2$ with 0.01% concentration in a tris buffer solution (pH 7.6), and protease K with a concentration of 1 to 50 μg/ml in a 10 mM tris-HCl buffer solution (pH 7.8) containing 10 mM EDTA and 0.5% SDS. In the case of using protease K, the pH of the reaction solution is appropriately selected from between 6.5 and 9.5, and an SH reagent, a trypsin inhibitor, or a chymotrypsin inhibitor may be appropriately used. Specific examples of such preferred protease also include protease attached to Histofine HER2 kit (MONO) (Nichirei Biosciences Inc.). The protease-induced epitope retrieval is usually performed at 37°C. The reaction temperature may be appropriately changed within the range of 25°C to 50°C. The reaction time of the protease-induced epitope retrieval performed at 37°C is appropriately selected from between, for example, 1 minute and 5 hours and is, for example, 15 minutes, 30 minutes, 45 minutes, 1 hour, 2 hours, 3 hours, or 4 hours. After the completion of the retrieval treatment, the tissue preparation thus treated is washed with a washing buffer solution. Phosphate-buffered saline (PBS) is preferably used as the washing buffer solution. Alternatively, a tris-HCl buffer solution may be preferably used. The washing conditions adopted in this method usually involve three runs of washing at room temperature for 5 minutes. The washing time and temperature may be appropriately changed.

Reaction between tissue preparation and anti-GPC3 antibody

The tissue preparation thus treated by the heat-induced epitope retrieval and/or the tissue preparation thus treated by the protease-induced epitope retrieval are reacted with an anti-GPC3 antibody mentioned later as a primary antibody. The reaction is carried out under conditions appropriate for the recognition of an epitope in the antigen by the anti-GPC3 antibody and the subsequent formation of an antigen-antibody complex. The reaction is usually carried out overnight at 4°C or at 37°C for 1 hour. The reaction conditions may be appropriately changed within a range appropriate for the recognition of an epitope in the antigen by the antibody and the subsequent formation of an antigen-antibody complex. For example, the reaction temperature may be changed within the range of 4°C to 50°C, while the reaction time may be changed between 1 minute and 7 days. A longer reaction time is more preferred for the reaction carried out at a low temperature. After the completion of the primary antibody reaction, each tissue preparation is washed with a washing buffer solution. Phosphate-buffered saline (PBS) is preferably used as the washing buffer solution. Alternatively, a tris-HCl buffer solution may be preferably used. The washing conditions adopted in this method usually involve three runs of washing at room temperature for 5 minutes. The washing time and temperature may be appropriately changed.

Subsequently, each tissue preparation thus reacted with the primary antibody is reacted with a secondary antibody that recognizes the primary antibody. A secondary antibody labeled in advance with a labeling material for visualizing the secondary antibody is usually used. Preferred examples of the labeling material include: fluorescent dyes such as fluorescein isothiocyanate (FITC), Cy2 (Amersham Biosciences Corp.), and Alexa 488 (Molecular Probes Inc.); enzymes such as peroxidase and alkaline phosphatase; and gold colloid.

The reaction with the secondary antibody is carried out under conditions appropriate for the formation of an antigen-antibody complex between the anti-GPC3 antibody and the secondary antibody that recognizes the anti-GPC3 antibody. The reaction is usually carried out at room temperature or 37°C for 30 minutes to 1 hour. The reaction conditions may be appropriately changed within a range appropriate for the formation of an antigen-antibody complex between the anti-GPC3 antibody and the secondary antibody. For example, the reaction temperature may be changed within the range of 4°C to 50°C, while the reaction time may be changed between 1 minute and 7 days. A longer reaction time is more preferred for the reaction carried out at a low temperature. After the completion of the secondary antibody reaction, each tissue preparation is washed with a washing buffer solution. Phosphate-buffered saline (PBS) is preferably used as the washing buffer solution. Alternatively, a tris-HCl buffer solution may be preferably used. The washing conditions adopted in this method usually involve three runs of washing at room temperature for 5 minutes. The washing time and temperature may be appropriately changed.

Next, each tissue preparation thus reacted with the secondary antibody is reacted with a substance capable of visualizing the labeling material. When peroxidase is used as the labeling material in the secondary antibody, a 0.02% aqueous hydrogen peroxide solution and a diaminobenzidine (DAB) solution concentration-adjusted to 0.1% with a 0.1 M tris-HCl buffer solution (pH 7.2) are mixed in equal amounts immediately before incubation and the tissue preparation is incubated in the resulting reaction solution. A chromogenic substrate such as DAB-Ni or AEC+ (both from Dako Japan Inc.) may be appropriately selected instead of DAB. During the course of incubation, the visualization reaction can be stopped by the dipping of the tissue preparation in PBS at the stage where appropriate color development is confirmed by the occasional microscopic observation of the degree of color development.

When alkaline phosphatase is used as the labeling material in the secondary antibody, each tissue preparation is incubated in a 5-bromo-4-chloro-3-indolyl phosphoric acid (BCIP)/nitro blue tetrazolium (NBT) (Zymed Laboratories, Inc.) substrate solution (solution of NBT and BCIP dissolved at concentrations of 0.4 mM and 0.38 mM, respectively, in a 50 mM sodium carbonate buffer solution (pH 9.8) containing 10 mM $MgCl_2$ and 28 mM NaCl). Alternatively, for example, Permanent Red, Fast Red, or Fuchsin+ (all from Dako Japan Inc.) may be appropriately used instead of BCIP and NBT. Prior to the incubation, the tissue preparation may be preincubated at room temperature for 1 minute to several hours with a 0.1 M tris-HCl buffer solution (pH 9.5) containing levamisole chloride (Nacalai Tesque, Inc.), an inhibitor of endogenous alkaline phosphatase, with a concentration of 1 mM, 0.1 M sodium chloride, and 50 mM magnesium chloride. During the course of incubation, the tissue preparation is washed with water or with TBST (TBS containing 0.1% Tween 20) after stop of the reaction by the addition of TBS containing 2% polyvinyl alcohol, at the stage where the deposition of a final reaction product purple formazan is confirmed by occasional microscopic observation. When gold colloid is used as the label in the secondary antibody, metallic silver is attached to gold particles by silver intensification to thereby visualize the gold colloid. The silver intensification method is generally known to those skilled in the art.

When a fluorescent dye such as fluorescein isothiocyanate (FITC), Cy2 (Amersham Biosciences Corp.), or Alexa 488 (Molecular Probes Inc.) is used as the labeling material in the secondary antibody, the reaction step of the visualizing substance is unnecessary. Each tissue preparation is irradiated with light at an excitation wavelength for the fluorescent material. Emitted light can be appropriately detected using a fluorescence microscope.

Immunohistochemical staining score

In a non-limiting aspect, the present invention also provides a method for determining the efficacy of GPC3-targeting drug therapy or determining the continuation of GPC3-targeting drug therapy from the concentration of free GPC3. Also referred to is a method for determining the efficacy of GPC3-targeting drug therapy or determining the continuation of GPC3-targeting drug therapy, where the expression level of GPC3 detected in tissues by the method described above. In a non-limiting instance, the expression of GPC3 detected in tissues by the method described above is digitized by, for example, a non-limiting method exemplified below. Such a digitized expression level of GPC3 in tissues may be referred to as an "immunohistochemical staining score of GPC3".

The respective scores of positive cell rate (PR), staining intensity of cytoplasm (SI-cp) or staining intensity of cell membrane (SI-cm), and staining pattern of cell membrane (Sp-cm) are calculated according to the criteria shown in Table 1 by a method described in WO2009116659 and added on the basis of calculation expressions 1 and 2. The resulting score is exemplified as the non-limiting immunohistochemical staining score of GPC3 (referred to as "composite score 1" for the sake of convenience).

[Table 1-1]

| Criterion | Evaluation | Score |
|---|---|---|
| Positive cell rate (PR) | 0 | 0 |
| | 1% or more and less than 20% | 1 |
| | 20% or more and less than 50% | 2 |
| | 50% or more | 3 |
| Staining intensity (SI)<br>- Cytoplasm (SI-cp)<br>- Cell membrane (SI-cm) | Slightly positive | 0 |
| | Weakly positive | 1 |
| | Moderately positive and/or weakly positive with strong positivity | 2 |
| | Moderately positive | 3 |
| | Strongly positive | 4 |
| Staining pattern of cell membrane (SP-cm) | Negative | 0 |
| | When only a portion of the cell membranes of cells was stained | 1 |
| | When a portion of the cell membranes of most of these cells was stained and the cell membranes of some of the cells were circumferentially stained | 2 |
| | When the cell membranes of most of these cells were circumferentially stained | 3 |
| (Sp-cm scores were calculated by the evaluation of cell staining in the visual field under microscope using an objective lens with a magnification of 4 or 10.) | | |

[Expression 1]

$$\text{IHC total} = \text{PR} + \text{SI-Cp} + \text{SI-Cm} + \text{Sp-Cm}$$

[Expression 2]

$$\text{IHC cm} = \text{PR} + \text{SI-Cm} + \text{Sp-Cm}$$

[Table 1-2]

| Composite score 1 | IHC total score |
|---|---|
| High expression | 7 or higher |
| Low or moderate expression | Lower than 7 |

In addition, the H-score is known (literature: KS. McCarty Jr. et al., Use of a monoclonal anti-Estrogen receptor antibody in the immunohistochemical evaluation of human tumors. Cancer Res. Suppl. (1986) 46, 4244s-4248s), which is calculated on the basis of the proportion of cells that exhibit each staining intensity (staining intensity of cell membrane or cytoplasm) classified into 0 to 3.

Another example of the immunohistochemical staining score includes the following scoring algorithm for classification of 0 to 3+ on the basis of the staining intensity of membrane, the staining intensity of cytoplasm, and the degree of staining, and an evaluation score based on the algorithm (composite score 2).

[Table 2]

| Score | Evaluation |
|---|---|
| 0 | When cell membranes were not stained |
| | When less than 10% of tumor cells exhibited intracytoplasmic staining |
| 1+ | When less than 10% of tumor cells exhibited cell membrane staining |
| | and/or |
| | When 10% or more of tumor cells exhibited intracytoplasmic staining (note that strong intracytoplasmic staining, if any, remains at less than 50% of the tumor cells) |
| 2+ | When 10% or more of tumor cells exhibited weak or moderate cell membrane staining (note that strong cell membrane staining, if any, remains at less than 10% of the tumor cells) regardless of the presence or absence of intracytoplasmic staining in 10% or more of the tumor cells (note that intracytoplasmic staining, if any, remains at less than 50% of the tumor cells) |
| 3+ | When 10% or more of tumor cells exhibited strong cell membrane staining regardless of the presence or absence of intracytoplasmic staining |
| | or |
| | When 50% or more of tumor cells exhibited strong intracytoplasmic staining |

The composite score 1, the H-score, and the composite score 2 may be used alone or in combination as the "immunohistochemical staining score of GPC3". In a non-limiting aspect, the composite score 1 may be used as the "immunohistochemical staining score of GPC3". In another non-limiting aspect, the composite score 2 may be used as the "immunohistochemical staining score of GPC3".

GPC3-targeting drug

In the present invention, the term "GPC3-targeting drug" refers to an anti-GPC3 antibody that blocks, suppresses, inhibits, or reduces the biological activity of GPC3 including a signal pathway mediated by GPC3 or is cytotoxic to cells expressing GPC3. The term "targeting treatment" does not suggest a certain mechanism having biological effects and conceptually includes every possible effect of the pharmacological, physiological, and biochemical interactions of GPC3. Examples of the GPC3-targeting drug include: (1) antagonistic or nonantagonistic inhibitors of the binding of GPC3 to a GPC3-binding ligand, i.e., active substances that interfere with the binding of GPC3 to its ligand; (2) active substances that do not interfere with the binding of GPC3 to its ligand but instead inhibit or decrease activity brought about by the binding of GPC3 to its ligand; (3) active substances that decrease GPC3 expression; and (4) active substances capable of eliciting cytotoxic activity against cells expressing GPC3. In a non-limiting aspect, examples of the ligand can include wnt (Cancer Res. (2005) 65, 6245-6254), IGF-II (Carcinogenesis (2008) 29 (7), 1319-1326), and fibroblast growth factor 2 (Int. J. Cancer (2003) 103 (4), 455-465). In a non-limiting aspect, such active substances can include, for example, antibodies (including their antigen-binding domains), nucleic acid molecules (antisense or RNAi molecules, etc.), peptides, non-peptidic low-molecular-weight organic materials.

In a non-limiting aspect, instances of the non-peptidic low-molecular-weight organic material that may be used as a GPC3-targeting drug include non-peptidic low-molecular-weight quinoline derivatives (WO2008/046085) which act on methylation suppressor genes. Further examples thereof can include HLA-A2-restricted GPC3 peptide 144-152 (SEQ ID NO: 2) and HLA-A24-restricted GPC3 peptide 298-306 (SEQ ID NO: 3) (Clin. Cancer Res. (2006) 12 (9), 2689-2697) which elicit the cytotoxic activity of cytotoxic T cells.

Anti-GPC3 antibody

The GPC3-targeting drug employed in the present invention is an anti-GPC3 antibody. In a non-limiting aspect, examples of the anti-GPC3 antibody that may be used as the GPC3-targeting drug in the present invention can include an antibody-drug conjugate (ADC) (WO2007/137170) comprising a 1G12 antibody (WO2003/100429) (sold under catalog No. B0134R by BioMosaics Inc.) conjugated with a cytotoxic substance.

In an alternative non-limiting aspect, examples of the anti-GPC3 antibody include a humanized anti-GPC3 antibody described in WO2006/006693 or WO2009/041062. Specifically, a humanized anti-GPC3 antibody comprising heavy chain CDR1, heavy chain CDR2, and heavy chain CDR3 represented by SEQ ID NOs: 4, 5, and 6, respectively, and light chain CDR1, light chain CDR2, and light chain CDR3 represented by SEQ ID NOs: 7, 8, and 9, respectively, can be used as the GPC3-targeting drug of the present invention. The humanized anti-GPC3 antibody can be prepared using, as templates for humanization, appropriately selected human framework sequences having high sequence identity to a heavy chain framework sequence represented by SEQ ID NO: 10 or a light chain framework sequence represented by SEQ ID NO: 11.

In a further alternative non-limiting aspect, an anti-GPC3 chimeric antibody or a humanized anti-GPC3 antibody comprising heavy chain CDR1, heavy chain CDR2, and heavy chain CDR3 represented by SEQ ID NOs: 12, 13, and 14, respectively, and light chain CDR1, light chain CDR2, and light chain CDR3 represented by SEQ ID NOs: 15, 16, and 17, respectively, can be used as the GPC3-targeting drug of the present invention. The humanized anti-GPC3 antibody can be prepared using, as templates for humanization, appropriately selected human framework sequences having high sequence identity to a heavy chain framework sequence represented by SEQ ID NO: 18 or a light chain framework sequence represented by SEQ ID NO: 19.

In an alternative non-limiting aspect, an anti-GPC3 chimeric antibody or a humanized anti-GPC3 antibody comprising heavy chain CDR1, heavy chain CDR2, and heavy chain CDR3 represented by SEQ ID NOs: 20, 21, and 22, respectively, and light chain CDR1, light chain CDR2, and light chain CDR3 represented by SEQ ID NOs: 23, 24, and 25, respectively, can be used as the GPC3-targeting drug of the present invention. The humanized anti-GPC3 antibody can be prepared using, as templates for humanization, appropriately selected human framework sequences having high sequence identity to a heavy chain framework sequence represented by SEQ ID NO: 26 or a light chain framework sequence represented by SEQ ID NO: 27.

In a further alternative non-limiting aspect, an anti-GPC3 chimeric antibody or a humanized anti-GPC3 antibody comprising heavy chain CDR1, heavy chain CDR2, and heavy chain CDR3 represented by SEQ ID NOs: 28, 29, and 30, respectively, and light chain CDR1, light chain CDR2, and light chain CDR3 represented by SEQ ID NOs: 31, 32, and 33, respectively, can be used as the GPC3-targeting drug of the present invention. The humanized anti-GPC3 antibody can be prepared using, as templates for humanization, appropriately selected human framework sequences having high sequence identity to a heavy chain framework sequence represented by SEQ ID NO: 34 or a light chain framework sequence represented by SEQ ID NO: 35.

In an alternative non-limiting aspect, an anti-GPC3 chimeric antibody or a humanized anti-GPC3 antibody comprising heavy chain CDR1, heavy chain CDR2, and heavy chain CDR3 represented by SEQ ID NOs: 36, 37, and 38, respectively, and light chain CDR1, light chain CDR2, and light chain CDR3 represented by SEQ ID NOs: 39, 40, and 41, respectively, can be used as the GPC3-targeting drug of the present invention. The humanized anti-GPC3 antibody can be prepared using, as templates for humanization, appropriately selected human framework sequences having high sequence identity to a heavy chain framework sequence represented by SEQ ID NO: 42 or a light chain framework sequence represented by SEQ ID NO: 43.

In a further alternative non-limiting aspect, a humanized anti-GPC3 antibody comprising a heavy chain variable region selected from the group of heavy chain variable regions represented by SEQ ID NOs: 44, 45, 46, 47, 48, 49, and 50 and a light chain variable region represented by SEQ ID NO: 51 can be used as the GPC3-targeting drug of the present invention. In a further alternative non-limiting aspect, a humanized anti-GPC3 antibody comprising a heavy chain variable region selected from the group of heavy chain variable regions represented by SEQ ID NOs: 44, 45, 46, 47, 48, 49, and 50 and a light chain variable region selected from the group of light chain variable regions represented by SEQ ID NOs: 52, 53, 54, 55, 56, 57, 58, 59, 60, 61, 62, 63, 64, 65, and 66 can be used as the GPC3-targeting drug of the present invention.

In a further alternative non-limiting aspect, a humanized anti-GPC3 antibody comprising a heavy chain variable region represented by SEQ ID NO: 67 and a light chain variable region represented by SEQ ID NO: 68, a humanized anti-GPC3 antibody comprising a heavy chain variable region represented by SEQ ID NO: 69 and a light chain variable region represented by SEQ ID NO: 70, a humanized anti-GPC3 antibody comprising a heavy chain variable region represented by SEQ ID NO: 71 and a light chain variable region represented by SEQ ID NO: 72, or a humanized anti-GPC3 antibody comprising a heavy chain variable region represented by SEQ ID NO: 71 and a light chain variable region represented by SEQ ID NO: 73 can also be used as the GPC3-targeting drug of the present invention.

Cytotoxic activity

Alternative examples of the anti-GPC3 antibody of the present invention include an anti-GPC3 antibody having cytotoxic activity. In the present invention, non-limiting examples of the cytotoxic activity include antibody-dependent cell-mediated cytotoxicity or antibody-dependent cellular cytotoxicity (ADCC) activity, complement-dependent cytotoxicity (CDC) activity, and cytotoxic activity based on T cells. In the present invention, the CDC activity means cytotoxic activity brought about by the complement system. On the other hand, the ADCC activity means the activity of damaging target cells by, for example, immunocytes, through the binding of the immunocytes via Fcγ receptors expressed on the immunocytes to the Fc regions of antigen-binding molecules comprising antigen-binding domains capable of binding to membrane molecules expressed on the cell membranes of the target cells. Whether or not the antigen-binding molecule of interest has ADCC activity or has CDC activity can be determined by a method known in the art (e.g., Current protocols in Immunology, Chapter 7. Immunologic studies in humans, Coligan et al., ed. (1993)).

Specifically, effector cells, a complement solution, and target cells are first prepared.

(1) Preparation of effector cells

The spleens are excised from CBA/N mice or the like, and spleen cells are separated therefrom in an RPMI1640 medium (Invitrogen Corp.). The spleen cells can be washed with this medium containing 10% fetal bovine serum (FBS, HyClone Laboratories, Inc.) and then concentration-adjusted to $5 \times 10^6$ cells/mL to prepare the effector cells.

(2) Preparation of complement solution

Baby Rabbit Complement (CEDARLANE Laboratories Ltd.) can be diluted 10-fold with a medium (Invitrogen Corp.) containing 10% FBS to prepare the complement solution.

(3) Preparation of target cells

Antigen-expressing cells can be cultured at 37°C for 1 hour, together with 0.2 mCi $^{51}$Cr-sodium chromate (GE Healthcare Bio-Sciences Corp.), in a DMEM medium containing 10% FBS to thereby radiolabel the target cells. The cells thus radiolabeled can be washed three times with an RPMI1640 medium containing 10% FBS and then concentration-adjusted to $2 \times 10^5$ cells/mL to prepare the target cells.

The ADCC or CDC activity can be assayed by a method described below. For the ADCC activity assay, the target cells and the antigen-binding molecule (each 50 μl/well) are added to a U-bottom 96-well plate (Becton, Dickinson and Company) and reacted for 15 minutes on ice. Then, 100 μ1 of the effector cells is added to each well, and the plate is left standing for 4 hours in a $CO_2$ incubator. The final concentration of the antibody (antigen-binding molecule) can be set to, for example, 0 or 10 μg/ml. The radioactivity of 100 μl of the supernatant recovered from each well of the plate thus left standing is measured using a gamma counter (COBRA II AUTO-GAMMA, MODEL D5005, Packard Instrument Company). The cytotoxic activity (%) can be calculated on the basis of the calculation expression $(A - C) / (B - C) \times 100$ using the measurement value, wherein A represents radioactivity (cpm) from each sample; B represents radioactivity (cpm) from a sample supplemented with 1% NP-40 (Nacalai Tesque, Inc.); and C represents radioactivity (cpm) from a sample containing only the target cells.

For the CDC activity assay, the target cells and the antigen-binding molecule (each 50 μl/well) are added to a flat-bottomed 96-well plate (Becton, Dickinson and Company) and reacted for 15 minutes on ice. Then, 100 μl of the complement solution is added to each well, and the plate is left standing for 4 hours in a $CO_2$ incubator. The final concentration of the antibody (antigen-binding molecule) can be set to, for example, 0 or 3 μg/ml. The radioactivity of 100 μl of the supernatant recovered from each well of the plate thus left standing is measured using a gamma counter. The cytotoxic activity based on the CDC activity can be calculated in the same way as in the ADCC activity assay.

Cytotoxic substance

In a non-limiting aspect, alternative examples of the anti-GPC3 antibody of the present invention include an anti-GPC3 antibody conjugated with a cytotoxic substance. Such an anti-GPC3 antibody-drug conjugate (ADC) is specifically disclosed in, for example, WO2007/137170, though the conjugate of the present invention is not limited to those described therein. Specifically, the cytotoxic substance may be any of chemotherapeutic agents listed below or may be a compound disclosed in Alley et al. (Curr. Opin. Chem. Biol. (2010) 14, 529-537) or WO2009/140242. Antigen-binding molecules are conjugated with these compounds via appropriate linkers or the like.

Examples of chemotherapeutic agents that may be conjugated to the anti-GPC3 antibody of the present invention can include the following: azaribine, anastrozole, azacytidine, bleomycin, bortezomib, bryostatin-1, busulfan, camptothecin, 10-hydroxycamptothecin, carmustine, Celebrex, chlorambucil, cisplatin, irinotecan, carboplatin, cladribine, cyclophosphamide, cytarabine, dacarbazine, docetaxel, dactinomycin, daunomycin glucuronide, daunorubicin, dexamethasone, diethylstilbestrol, doxorubicin, doxorubicin glucuronide, epirubicin, ethinyl estradiol, estramustine, etoposide, etoposide glucuronide, floxuridine, fludarabine, flutamide, fluorouracil, fluoxymesterone, gemcitabine, hydroxyprogesterone caproate, hydroxyurea, idarubicin, ifosfamide, leucovorin, lomustine, maytansinoid, mechlorethamine, medroxyprogesterone acetate, megestrol acetate, melphalan, mercaptopurine, methotrexate, mitoxantrone, mithramycin, mitomycin, mitotane, phenylbutyrate, prednisone, procarbazine, paclitaxel, pentostatin, semustine, streptozocin, tamoxifen, taxanes, Taxol, testosterone propionate, thalidomide, thioguanine, thiotepa, teniposide, topotecan, uracil mustard, vinblastine, vinorelbine, and vincristine.

In the present invention, a preferred chemotherapeutic agent is a low-molecular-weight chemotherapeutic agent. The low-molecular-weight chemotherapeutic agent is unlikely to interfere with the functions of the anti-GPC3 antibody even after forming the anti-GPC3 antibody-drug conjugate of the present invention. In the present invention, the low-molecular-weight chemotherapeutic agent has a molecular weight of usually 100 to 2000, preferably 200 to 1000. All of the chemotherapeutic agents listed herein are low-molecular-weight chemotherapeutic agents. These chemotherapeutic agents according to the present invention include prodrugs that are converted to active chemotherapeutic agents *in vivo.* The prodrugs may be activated through enzymatic conversion or nonenzymatic conversion.

Alternative examples of the conjugated cytotoxic substance in the anti-GPC3 antibody-drug conjugate of the present invention can include toxic peptides (toxins) such as *Pseudomonas* exotoxin A, saporin-s6, diphtheria toxin, and cnidarian toxin, radioiodine, and photosensitizers. Examples of the toxic peptides preferably include the following:

diphtheria toxin A chain (Langone et al., Methods in Enzymology (1983) 93, 307-308);
*Pseudomonas* exotoxin (Nature Medicine (1996) 2, 350-353); ricin A chain (Fulton et al., J. Biol. Chem. (1986) 261, 5314-5319; Sivam et al., Cancer Res. (1987) 47, 3169-3173; Cumber et al., J. Immunol. Methods (1990) 135, 15-24; Wawrzynczak et al., Cancer Res. (1990) 50, 7519-7562; and Gheeite et al., J. Immunol. Methods (1991) 142, 223-230) ;
deglycosylated ricin A chain (Thorpe et al., Cancer Res. (1987) 47, 5924-5931);
abrin A chain (Wawrzynczak et al., Br. J. Cancer (1992) 66, 361-366; Wawrzynczak et al., Cancer Res. (1990) 50, 7519-7562; Sivam et al., Cancer Res. (1987) 47, 3169-3173; and Thorpe et al., Cancer Res. (1987) 47, 5924-5931);
gelonin (Sivam et al., Cancer Res. (1987) 47, 3169-3173; Cumber et al., J. Immunol. Methods (1990) 135, 15-24; Wawrzynczak et al., Cancer Res., (1990) 50, 7519-7562; and Bolognesi et al., Clin. exp. Immunol. (1992) 89, 341-346);
pokeweed anti-viral protein from seeds (PAP-s) (Bolognesi et al., Clin. exp. Immunol. (1992) 89, 341-346);
bryodin (Bolognesi et al., Clin. exp. Immunol. (1992) 89, 341-346);
saporin (Bolognesi et al., Clin. exp. Immunol. (1992) 89, 341-346);
momordin (Cumber et al., J. Immunol. Methods (1990) 135, 15-24; Wawrzynczak et al., Cancer Res. (1990) 50, 7519-7562; and Bolognesi et al., Clin. exp. Immunol. (1992) 89, 341-346);
momorcochin (Bolognesi et al., Clin. exp. Immunol. (1992) 89, 341-346);
dianthin 32 (Bolognesi et al., Clin. exp. Immunol. (1992) 89, 341-346);
dianthin 30 (Stirpe F., Barbieri L., FEBS letter (1986) 195, 1-8);
modeccin (Stirpe F., Barbieri L., FEBS letter (1986) 195, 1-8);
viscumin (Stirpe F., Barbieri L., FEBS letter (1986) 195, 1-8);
volkensin (Stirpe F., Barbieri L., FEBS letter (1986) 195, 1-8);
dodecandrin (Stirpe F., Barbieri L., FEBS letter (1986) 195, 1-8);
tritin (Stirpe F., Barbieri L., FEBS letter (1986) 195, 1-8) ;
luffin (Stirpe F., Barbieri L., FEBS letter (1986) 195, 1-8); and
trichokirin (Casellas et al., Eur. J. Biochem. (1988) 176, 581-588; and Bolognesi et al., Clin. exp. Immunol., (1992) 89, 341-346).

In the case of assaying the cytotoxic activity of the anti-GPC3 antibody-drug conjugate of the present invention, the target cells and the anti-GPC3 antibody-drug conjugate (each 50 μl/well) are added to a flat-bottomed 96-well plate (Becton, Dickinson and Company) and reacted for 15 minutes on ice. The plate is incubated for 1 to 4 hours in a $CO_2$ incubator. The anti-GPC3 antibody-drug conjugate can be appropriately used at a final concentration ranging from 0 to 3 μg/ml. After the culture, 100 μl of the supernatant is recovered from each well, and the radioactivity of the supernatant is measured using a gamma counter. The cytotoxic activity can be calculated in the same way as in the ADCC activity assay.

Fc region

An Fc region contained in a constant region contained in the anti-GPC3 antibody of the present invention may be obtained from human IgG, though the Fc region of the present invention is not limited by a particular subclass of IgG. The Fc region refers to an antibody heavy chain constant region comprising a hinge region and CH2 and CH3 domains from the hinge region N terminus which is a papain cleavage site (about amino acid 216 based on the EU numbering). Preferred examples of the Fc region include Fc regions having binding activity against Fcγ receptors as mentioned later. In a non-limiting aspect, examples of such Fc regions include Fc regions contained in constant regions represented by SEQ ID NO: 74 for human IgG1, SEQ ID NO: 75 for IgG2, SEQ ID NO: 76 for IgG3, and SEQ ID NO: 77 for IgG4.

Fcγ receptor (FcγR)

The Fcγ receptor (also referred to as FcγR) refers to a receptor capable of binding to the Fc region of an IgG1, IgG2, IgG3, or IgG4 monoclonal antibody and substantially means even any member of protein family encoded by Fcγ receptor genes. In humans, this family includes, but not limited to: FcγRI (CD64) including isoforms FcγRIa, FcγRIb, and FcγRIc; FcγRII (CD32) including isoforms FcγRIIa (including allotypes H131 and R131; i.e., FcγRIIa (H) and FcγRIIa (R)), FcγRIIb (including FcγRIIb-1 and FcγRIIb-2), and FcγRIIc; FcγRIII (CD16) including isoforms FcγRIIIa (including allotypes V158 and F158; i.e., FcγRIIIa (V) and FcγRIIIa (F)) and FcγRIIIb (including allotypes FcγRIIIb-NAl and FcγRIIIb-NA2); and even any unfound human FcγR or FcγR isoform or allotype. FcγR includes human, mouse, rat, rabbit, and monkey Fcγ receptors. The FcγR of the present invention is not limited to these receptors and may be derived from any organism. The mouse FcγR includes, but not limited to, FcγRI (CD64), FcγRII (CD32), FcγRIII (CD16), and FcγRIII-2 (FcγRIV, CD16-2), and even any unfound mouse FcγR or FcγR isoform or allotype. Preferred examples of such Fcγ receptors include human FcγRI (CD64), FcγRIIa (CD32), FcγRIIb (CD32), FcγRIIIa (CD16), and/or FcγRIIIb (CD16). The polypeptide sequence of human FcγRI is described in SEQ ID NO: 78 (NP_000557.1); the polypeptide sequence of human FcγRIIa (allotype H131) is described in SEQ ID NO: 79 (AAH20823.1) (allotype R131 has a sequence with substitution by Arg at amino acid 166 in SEQ ID NO: 79); the polypeptide sequence of FcγRIIb is described in SEQ ID NO: 80 (AAI46679.1); the polypeptide sequence of FcγRIIIa is described in SEQ ID NO: 81 (AAH33678.1); and the polypeptide sequence of FcγRIIIb is described in SEQ ID NO: 82 (AAI28563.1) (registration numbers of a database such as RefSeq are shown within the parentheses). Whether or not the Fcγ receptor has binding activity against the Fc region of an IgG1, IgG2, IgG3, or IgG4 monoclonal antibody can be confirmed by a method known in the art such as FACS or ELISA formats as well as BIACORE method using amplified luminescent proximity homogeneous assay (ALPHA) screening or surface plasmon resonance (SPR) phenomena (Proc. Natl. Acad. Sci. U.S.A. (2006) 103 (11), 4005-4010).

In FcγRI (CD64) including isoforms FcγRIa, FcγRIb, and FcγRIc and FcγRIII (CD16) including isoforms FcγRIIIa (including allotypes V158 and F158) and FcγRIIIb (including allotypes FcγRIIIb-NAl and FcγRIIIb-NA2), an α chain capable of binding to the IgG Fc region associates with a common γ chain having ITAM that transduces activating signals into cells. On the other hand, FcγRII (CD32) including isoforms FcγRIIa (including allotypes H131 and R131) and FcγRIIc contains ITAM in its cytoplasmic domain. These receptors are expressed in many immunocytes, such as macrophages, mast cells, and antigen-displaying cells. These receptors bind to IgG Fc regions and thereby transduce activating signals, which in turn promote the phagocytic capacity of macrophages, the production of inflammatory cytokines, the degranulation of mast cells, and the increased function of antigen-displaying cells. The Fcγ receptors that are able to transduce activating signals as described above are referred to as active Fcγ receptors herein.

On the other hand, FcγRIIb (including FcγRIIb-1 and FcγRIIb-2) contains ITIM that transduces inhibitory signals, in its intracytoplasmic domain. In B cells, activating signals from B cell receptors (BCRs) are inhibited by the cross-linking of BCR with FcγRIIb, resulting in the suppressed antibody production of BCR. The phagocytic capacity of macrophages or their ability to produce inflammatory cytokines is suppressed by the cross-linking of FcγRIII and FcγRIIb. The Fcγ receptors that are able to transduce inhibitory signals as described above are referred to as inhibitory Fcγ receptors herein.

Binding activity of Fc region against FcγR

As mentioned above, examples of the Fc region contained in the anti-GPC3 antibody of the present invention include Fc regions having binding activity against Fcγ receptors. In a non-limiting aspect, examples of such Fc regions include Fc regions contained in constant regions represented by SEQ ID NO: 74 for human IgG1, SEQ ID NO: 75 for IgG2, SEQ ID NO: 76 for IgG3, and SEQ ID NO: 77 for IgG4. Whether or not the Fcγ receptor has binding activity against the Fc region of an IgG1, IgG2, IgG3, or IgG4 monoclonal antibody can be confirmed by a method known in the art such as FACS or ELISA formats as well as BIACORE method using amplified luminescent proximity homogeneous assay (ALPHA) screening or surface plasmon resonance (SPR) phenomena (Proc. Natl. Acad. Sci. U.S.A. (2006) 103 (11), 4005-4010).

The ALPHA screening is carried out on the basis of the following principles according to ALPHA technology using two beads, a donor and an acceptor. Luminescence signals are detected only when these two beads are located in proximity through the biological interaction between a molecule bound with the donor bead and a molecule bound with the acceptor bead. A laser-excited photosensitizer in the donor bead converts ambient oxygen to singlet oxygen in an excited state. The singlet oxygen diffuses around the donor bead and reaches the acceptor bead located in proximity thereto to thereby cause chemiluminescent reaction in the bead, which finally emits light. In the absence of the interaction between the molecule bound with the donor bead and the molecule bound with the acceptor bead, singlet oxygen produced by the donor bead does not reach the acceptor bead. Thus, no chemiluminescent reaction occurs.

For example, a biotin-labeled anti-GPC3 antibody comprising the Fc region is bound to the donor bead, while a glutathione S transferase (GST)-tagged Fcγ receptor is bound to the acceptor bead. In the absence of a competing anti-GPC3 antibody comprising a modified Fc region, the anti-GPC3 antibody having the native Fc region interacts with the Fcγ receptor to generate signals of 520 to 620 nm. An anti-GPC3 antibody comprising an untagged modified Fc region competes with the anti-GPC3 antibody having the native Fc region for the interaction with the Fcγ receptor. Decrease in fluorescence caused as a result of the competition can be quantified to thereby determine relative binding affinity. The antibody biotinylation using sulfo-NHS-biotin or the like is known in the art. The Fcγ receptor can be tagged with GST by an appropriately adopted method which involves, for example: fusing a polynucleotide encoding the Fcγ receptor in flame with a polynucleotide encoding GST; operably ligating the resulting fusion gene with a vector; and allowing cells or the like carrying the vector to express the GST-tagged Fcγ receptor, which is then purified using a glutathione column. The obtained signals are preferably analyzed using, for example, software GRAPHPAD PRISM (GraphPad Software, Inc., San Diego) adapted to a one-site competition model based on nonlinear regression analysis.

One (ligand) of the substances between which the interaction is to be observed is immobilized on a thin gold film of a sensor chip. The sensor chip is irradiated with light from the back such that total reflection occurs at the interface between the thin gold film and glass. As a result, a site having a drop in reflection intensity (SPR signal) is formed in a portion of reflected light. The other (analyte) of the substances between which the interaction is to be observed is flowed on the surface of the sensor chip and bound to the ligand so that the mass of the immobilized ligand molecule is increased to change the refractive index of the solvent on the sensor chip surface. This change in the refractive index shifts the position of the SPR signal (on the contrary, the dissociation of the bound molecules gets the signal back to the original position). The Biacore system plots on the ordinate the amount of the shift, i.e., change in mass on the sensor chip surface, and displays time-dependent change in mass as assay data (sensorgram). Kinetics: an association rate constant (ka) and a dissociation rate constant (kd) can be determined from the curve of the sensorgram, and affinity (KD) can be determined from the ratio between these constants. Inhibition assay is also preferably used in the BIACORE method. Examples of the inhibition assay are described in Lazor et al. (Proc. Natl. Acad. Sci. U.S.A. (2006) 103 (11), 4005-4010).

Fcγ receptor (FcyR)-binding modified Fc region

In addition to the Fc regions contained in constant regions represented by SEQ ID NO: 74 for human IgG1, SEQ ID NO: 75 for IgG2, SEQ ID NO: 76 for IgG3, and SEQ ID NO: 77 for IgG4, an FcyR-binding modified Fc region having higher binding activity against Fcγ receptors than that of the Fc region of native human IgG against Fcγ receptors may be appropriately used as the Fc region contained in the anti-GPC3 antibody of the present invention. The "Fc region of native human IgG" described herein means an Fc region having a fucose-containing sugar chain as a sugar chain bound to position 297 (EU numbering) of the Fc region contained in the human IgG1, IgG2, IgG3, or IgG4 constant region represented by SEQ ID NO: 74, 75, 76, or 77. Such an FcyR-binding modified Fc region can be prepared by the amino acid modification of the native human IgG Fc region. Whether or not the FcyR-binding modified Fc region has higher binding activity against FcyR than that of the native human IgG Fc region against FcyR can be appropriately confirmed by a method known in the art such as FACS or ELISA formats as well as BIACORE method using amplified luminescent proximity homogeneous assay (ALPHA) screening or surface plasmon resonance (SPR) phenomena as described above.

In the present invention, the "modification of amino acid(s)" or "amino acid modification" of the Fc region includes modification to an amino acid sequence different from the amino acid sequence of the starting Fc region. Any Fc region can be used as the starting Fc region as long as the modified form of the starting Fc region can bind to the human Fcγ receptor in a neutral region of pH. Alternatively, an Fc region further modified from an already modified Fc region as the starting Fc region may be preferably used as the Fc region of the present invention. The starting Fc region may mean the polypeptide itself, a composition containing the starting Fc region, or an amino acid sequence encoding the starting Fc region. The starting Fc region can include Fc regions known in the art produced by recombination reviewed in the paragraph about the antibody. The starting Fc region is not limited by its origin and can be obtained from an arbitrary nonhuman animal organism or a human. Preferred examples of the arbitrary organism include an organism selected from mice, rats, guinea pigs, hamsters, gerbils, cats, rabbits, dog, goats, sheep, cattle, horses, camels, and nonhuman primates. In another aspect, the starting Fc region may be obtained from a cynomolgus monkey, a marmoset, a rhesus monkey, a chimpanzee, or a human. Preferably, the starting Fc region can be obtained from human IgG1, though the starting Fc region of the present invention is not limited by a particular class of IgG. This means that the Fc region of human IgG1, IgG2, IgG3, or IgG4 can be appropriately used as the starting Fc region. Likewise, this means herein that the Fc region of arbitrary IgG class or subclass from the arbitrary organism can be preferably used as the starting Fc region. Examples of variants of naturally occurring IgG or manipulated forms thereof are described in literatures known in the art (Curr. Opin. Biotechnol. (2009) 20 (6), 685-91; Curr. Opin. Immunol. (2008) 20 (4), 460-470; Protein Eng. Des. Sel. (2010) 23 (4), 195-202; and International Publication Nos. WO2009/086320, WO2008/092117, WO2007/041635, and WO2006/105338), though the variants or the manipulated forms of the present invention are not limited to those described therein.

Examples of the modification include one or more variations, for example, a variation that substitutes amino acid(s) in the starting Fc region by amino acid residue(s) different therefrom, the insertion of one or more amino acid residues into the amino acid sequence of the starting Fc region, and/or the deletion of one or more amino acids from the amino acid sequence of the starting Fc region. Preferably, the amino acid sequence of the Fc region thus modified comprises an amino acid sequence containing at least a nonnatural portion of the Fc region. Such a variant inevitably has less than 100% sequence identity or similarity to the starting Fc region. In a preferred embodiment, the variant has an amino acid sequence with approximately 75% to less than 100% sequence identity or similarity, more preferably approximately 80% to less than 100%, further preferably approximately 85% to less than 100%, still further preferably approximately 90% to less than 100%, most preferably approximately 95% to less than 100% sequence identity or similarity to the amino acid sequence of the starting Fc region. In a non-limiting aspect of the present invention, the starting Fc region and the FcyR-binding modified Fc region of the present invention differ by at least one amino acid. The difference in amino acid between the starting Fc region and the FcyR-binding modified Fc region of the present invention may be preferably determined by a difference in amino acid with the identified position of its amino acid residue defined particularly by the EU numbering.

The amino acid(s) in the Fc region can be modified by an appropriately adopted method known in the art such as site-directed mutagenesis (Kunkel et al., Proc. Natl. Acad. Sci. USA (1985) 82, 488-492) or overlap extension PCR. Also, a plurality of methods known in the art can be adopted as methods for modifying an amino acid to substitute the amino acid by an amino acid other than natural one (Annu. Rev. Biophys. Biomol. Struct. (2006) 35, 225-249; and Proc. Natl. Acad. Sci. U.S.A. (2003) 100 (11), 6353-6357). For example, a tRNA-containing cell-free translation system (Clover Direct (Protein Express, an R & D oriented company)) comprising a non-natural amino acid bound with an amber suppressor tRNA complementary to UAG codon (amber codon), which is a stop codon, is also preferably used.

The FcyR-binding modified Fc region (contained in the antigen-binding molecule of the present invention) having higher binding activity against Fcγ receptors than that of the native human IgG Fc region against Fcγ receptors can be obtained by any method. Specifically, the FcyR-binding modified Fc region can be obtained by the amino acid modification of a human IgG immunoglobulin Fc region used as the starting Fc region. Examples of the IgG immunoglobulin Fc region preferred for the modification include Fc regions contained in human IgG (IgG1, IgG2, IgG3, and IgG4, and modified forms thereof) constant regions represented by SEQ ID NOs: 74, 75, 76, and 77.

The modification to other amino acids can include amino acid modification at any position as long as the resulting Fc region has higher binding activity against Fcγ receptors than that of the native human IgG Fc region against Fcγ receptors. When the antigen-binding molecule contains a human IgG1 Fc region as a human Fc region, the modification preferably allows the Fc region to contain a fucose-containing sugar chain as a sugar chain bound to position 297 (EU numbering) and is effective for producing higher binding activity against Fcγ receptors than that of the native human IgG Fc region against Fcγ receptors. Such amino acid modification has been reported in, for example, International Publication Nos. WO2007/024249, WO2007/021841, WO2006/031370, WO2000/042072, WO2004/029207, WO2004/099249, WO2006/105338, WO2007/041635, WO2008/092117, WO2005/070963, WO2006/020114, WO2006/116260, and WO2006/023403.

Examples of amino acids that may undergo such modification include at least one or more amino acids selected from the group consisting of position 221, position 222, position 223, position 224, position 225, position 227, position 228, position 230, position 231, position 232, position 233, position 234, position 235, position 236, position 237, position 238, position 239, position 240, position 241, position 243, position 244, position 245, position 246, position 247, position 249, position 250, position 251, position 254, position 255, position 256, position 258, position 260, position 262, position 263, position 264, position 265, position 266, position 267, position 268, position 269, position 270, position 271, position 272, position 273, position 274, position 275, position 276, position 278, position 279, position 280, position 281, position 282, position 283, position 284, position 285, position 286, position 288, position 290, position 291, position 292, position 293, position 294, position 295, position 296, position 297, position 298, position 299, position 300, position 301, position 302, position 303, position 304, position 305, position 311, position 313, position 315, position 317, position 318, position 320, position 322, position 323, position 324, position 325, position 326, position 327, position 328, position 329, position 330, position 331, position 332, position 333, position 334, position 335, position 336, position 337, position 339, position 376, position 377, position 378, position 379, position 380, position 382, position 385, position 392, position 396, position 421, position 427, position 428, position 429, position 434, position 436 and position 440 based on the EU numbering. The modification of these amino acids can yield the Fc region (FcyR-binding modified Fc region) having higher binding activity against Fcγ receptors than that of the native human IgG Fc region against Fcγ receptors.

Examples of particularly preferred modification for use in the present invention include at least one or more amino acid modifications selected from the group consisting of modifications of

amino acid 221 to Lys or Tyr,
amino acid 222 to Phe, Trp, Glu, or Tyr,
amino acid 223 to Phe, Trp, Glu, or Lys,
amino acid 224 to Phe, Trp, Glu, or Tyr,

amino acid 225 to Glu, Lys, or Trp,
amino acid 227 to Glu, Gly, Lys, or Tyr,
amino acid 228 to Glu, Gly, Lys, or Tyr,
amino acid 230 to Ala, Glu, Gly, or Tyr,
amino acid 231 to Glu, Gly, Lys, Pro, or Tyr,
amino acid 232 to Glu, Gly, Lys, or Tyr,
amino acid 233 to Ala, Asp, Phe, Gly, His, Ile, Lys, Leu, Met, Asn, Gln, Arg, Ser, Thr, Val, Trp, or Tyr,
amino acid 234 to Ala, Asp, Glu, Phe, Gly, His, Ile, Lys, Met, Asn, Pro, Gln, Arg, Ser, Thr, Val, Trp, or Tyr,
amino acid 235 to Ala, Asp, Glu, Phe, Gly, His, Ile, Lys, Met, Asn, Pro, Gln, Arg, Ser, Thr, Val, Trp, or Tyr,
amino acid 236 to Ala, Asp, Glu, Phe, His, Ile, Lys, Leu, Met, Asn, Pro, Gln, Arg, Ser, Thr, Val, Trp, or Tyr,
amino acid 237 to Asp, Glu, Phe, His, Ile, Lys, Leu, Met, Asn, Pro, Gln, Arg, Ser, Thr, Val, Trp, or Tyr,
amino acid 238 to Asp, Glu, Phe, Gly, His, Ile, Lys, Leu, Met, Asn, Gln, Arg, Ser, Thr, Val, Trp, or Tyr,
amino acid 239 to Asp, Glu, Phe, Gly, His, Ile, Lys, Leu, Met, Asn, Pro, Gln, Arg, Thr, Val, Trp, or Tyr,
amino acid 240 to Ala, Ile, Met, or Thr,
amino acid 241 to Asp, Glu, Leu, Arg, Trp, or Tyr,
amino acid 243 to Leu, Glu, Leu, Gln, Arg, Trp, or Tyr,
amino acid 244 to His,
amino acid 245 to Ala,
amino acid 246 to Asp, Glu, His, or Tyr,
amino acid 247 to Ala, Phe, Gly, His, Ile, Leu, Met, Thr, Val, or Tyr,
amino acid 249 to Glu, His, Gln, or Tyr,
amino acid 250 to Glu, or Gln,
amino acid 251 to Phe,
amino acid 254 to Phe, Met, or Tyr,
amino acid 255 to Glu, Leu, or Tyr,
amino acid 256 to Ala, Met, or Pro,
amino acid 258 to Asp, Glu, His, Ser, or Tyr,
amino acid 260 to Asp, Glu, His, or Tyr,
amino acid 262 to Ala, Glu, Phe, Ile, or Thr,
amino acid 263 to Ala, Ile, Met, or Thr,
amino acid 264 to Asp, Glu, Phe, Gly, His, Ile, Lys, Leu, Met, Asn, Pro, Gln, Arg, Ser, Thr, Trp, or Tyr,
amino acid 265 to Ala, Leu, Phe, Gly, His, Ile, Lys, Leu, Met, Asn, Pro, Gln, Arg, Ser, Thr, Val, Trp, or Tyr, amino acid 266 to Ala, Ile, Met, or Thr,
amino acid 267 to Asp, Glu, Phe, His, Ile, Lys, Leu, Met, Asn, Pro, Gln, Arg, Thr, Val, Trp, or Tyr,
amino acid 268 to Asp, Glu, Phe, Gly, Ile, Lys, Leu, Met, Pro, Gln, Arg, Thr, Val, or Trp,
amino acid 269 to Phe, Gly, His, Ile, Lys, Leu, Met, Asn, Pro, Arg, Ser, Thr, Val, Trp, or Tyr,
amino acid 270 to Glu, Phe, Gly, His, Ile, Leu, Met, Pro, Gln, Arg, Ser, Thr, Trp, or Tyr,
amino acid 271 to Ala, Asp, Glu, Phe, Gly, His, Ile, Lys, Leu, Met, Asn, Gln, Arg, Ser, Thr, Val, Trp, or Tyr,
amino acid 272 to Asp, Phe, Gly, His, Ile, Lys, Leu, Met, Pro, Arg, Ser, Thr, Val, Trp, or Tyr,
amino acid 273 to Phe, or Ile,
amino acid 274 to Asp, Glu, Phe, Gly, His, Ile, Leu, Met, Asn, Pro, Arg, Ser, Thr, Val, Trp, or Tyr,
amino acid 275 to Leu, or Trp,
amino acid 276 to Asp, Glu, Phe, Gly, His, Ile, Leu, Met, Pro, Arg, Ser, Thr, Val, Trp, or Tyr,
amino acid 278 to Asp, Glu, Gly, His, Ile, Lys, Leu, Met, Asn, Pro, Gln, Arg, Ser, Thr, Val, or Trp,
amino acid 279 to Ala,
amino acid 280 to Ala, Gly, His, Lys, Leu, Pro, Gln, Trp, or Tyr,
amino acid 281 to Asp, Lys, Pro, or Tyr,
amino acid 282 to Glu, Gly, Lys, Pro, or Tyr,
amino acid 283 to Ala, Gly, His, Ile, Lys, Leu, Met, Pro, Arg, or Tyr,
amino acid 284 to Asp, Glu, Leu, Asn, Thr, or Tyr,
amino acid 285 to Asp, Glu, Lys, Gln, Trp, or Tyr,
amino acid 286 to Glu, Gly, Pro, or Tyr,
amino acid 288 to Asn, Asp, Glu, or Tyr,
amino acid 290 to Asp, Gly, His, Leu, Asn, Ser, Thr, Trp, or Tyr,
amino acid 291 to Asp, Glu, Gly, His, Ile, Gln, or Thr, amino acid 292 to Ala, Asp, Glu, Pro, Thr, or Tyr,
amino acid 293 to Phe, Gly, His, Ile, Leu, Met, Asn, Pro, Arg, Ser, Thr, Val, Trp, or Tyr,
amino acid 294 to Phe, Gly, His, Ile, Lys, Leu, Met, Asn, Pro, Arg, Ser, Thr, Val, Trp, or Tyr,
amino acid 295 to Asp, Glu, Phe, Gly, His, Ile, Lys, Met, Asn, Pro, Arg, Ser, Thr, Val, Trp, or Tyr,

amino acid 296 to Ala, Asp, Glu, Gly, His, Ile, Lys, Leu, Met, Asn, Gln, Arg, Ser, Thr, or Val,
amino acid 297 to Asp, Glu, Phe, Gly, His, Ile, Lys, Leu, Met, Pro, Gln, Arg, Ser, Thr, Val, Trp, or Tyr,
amino acid 298 to Ala, Asp, Glu, Phe, His, Ile, Lys, Met, Asn, Gln, Arg, Thr, Val, Trp, or Tyr,
amino acid 299 to Ala, Asp, Glu, Phe, Gly, His, Ile, Lys, Leu, Met, Asn, Pro, Gln, Arg, Ser, Val, Trp, or Tyr,
amino acid 300 to Ala, Asp, Glu, Gly, His, Ile, Lys, Leu, Met, Asn, Pro, Gln, Arg, Ser, Thr, Val, or Trp,
amino acid 301 to Asp, Glu, His, or Tyr,
amino acid 302 to Ile,
amino acid 303 to Asp, Gly, or Tyr,
amino acid 304 to Asp, His, Leu, Asn, or Thr,
amino acid 305 to Glu, Ile, Thr, or Tyr,
amino acid 311 to Ala, Asp, Asn, Thr, Val, or Tyr,
amino acid 313 to Phe,
amino acid 315 to Leu,
amino acid 317 to Glu or Gln,
amino acid 318 to His, Leu, Asn, Pro, Gln, Arg, Thr, Val, or Tyr,
amino acid 320 to Asp, Phe, Gly, His, Ile, Leu, Asn, Pro, Ser, Thr, Val, Trp, or Tyr,
amino acid 322 to Ala, Asp, Phe, Gly, His, Ile, Pro, Ser, Thr, Val, Trp, or Tyr,
amino acid 323 to Ile,
amino acid 324 to Asp, Phe, Gly, His, Ile, Leu, Met, Pro, Arg, Thr, Val, Trp, or Tyr,
amino acid 325 to Ala, Asp, Glu, Phe, Gly, His, Ile, Lys, Leu, Met, Pro, Gln, Arg, Ser, Thr, Val, Trp, or Tyr,
amino acid 326 to Ala, Asp, Glu, Gly, Ile, Leu, Met, Asn, Pro, Gln, Ser, Thr, Val, Trp, or Tyr,
amino acid 327 to Ala, Asp, Glu, Phe, Gly, His, Ile, Lys, Leu, Met, Asn, Pro, Arg, Thr, Val, Trp, or Tyr,
amino acid 328 to Ala, Asp, Glu, Phe, Gly, His, Ile, Lys, Met, Asn, Pro, Gln, Arg, Ser, Thr, Val, Trp, or Tyr,
amino acid 329 to Asp, Glu, Phe, Gly, His, Ile, Lys, Leu, Met, Asn, Gln, Arg, Ser, Thr, Val, Trp, or Tyr,
amino acid 330 to Cys, Glu, Phe, Gly, His, Ile, Lys, Leu, Met, Asn, Pro, Arg, Ser, Thr, Val, Trp, or Tyr,
amino acid 331 to Asp, Phe, His, Ile, Leu, Met, Gln, Arg, Thr, Val, Trp, or Tyr,
amino acid 332 to Ala, Asp, Glu, Phe, Gly, His, Lys, Leu, Met, Asn, Pro, Gln, Arg, Ser, Thr, Val, Trp, or Tyr,
amino acid 333 to Ala, Asp, Glu, Phe, Gly, His, Ile, Leu, Met, Pro, Ser, Thr, Val, or Tyr,
amino acid 334 to Ala, Glu, Phe, Ile, Leu, Pro, or Thr,
amino acid 335 to Asp, Phe, Gly, His, Ile, Leu, Met, Asn, Pro, Arg, Ser, Val, Trp, or Tyr,
amino acid 336 to Glu, Lys, or Tyr,
amino acid 337 to Glu, His, or Asn,
amino acid 339 to Asp, Phe, Gly, Ile, Lys, Met, Asn, Gln, Arg, Ser, or Thr,
amino acid 376 to Ala, or Val,
amino acid 377 to Gly, or Lys,
amino acid 378 to Asp,
amino acid 379 to Asn,
amino acid 380 to Ala, Asn, or Ser,
amino acid 382 to Ala, or Ile,
amino acid 385 to Glu,
amino acid 392 to Thr,
amino acid 396 to Leu,
amino acid 421 to Lys,
amino acid 427 to Asn,
amino acid 428 to Phe, or Leu,
amino acid 429 to Met,
amino acid 434 to Trp,
amino acid 436 to Ile, or
amino acid 440 to Gly, His, Ile, Leu, or Tyr

based on the EU numbering in the Fc region. The number of amino acids to be modified is not limited. Only one amino acid may be modified, or two or more amino acids may be modified. Examples of combinations of amino acid modifications at two or more positions include combinations as described in Table 3 (Tables 3-1 to 3-3). Also, WO2007/047291 discloses specific examples of the anti-GPC3 antibody comprising the FcyR-binding modified Fc region having higher binding activity against Fcγ receptors than that of the native human IgG Fc region against Fcγ receptors.

[Table 3-1]

| Combination of amino acids | Combination of amino acids |
|---|---|
| K370E/P396L/D270E | S239Q/1332Q |
| Q419H/P396L/D270E | S267D/I332R |
| V240A/P396L/D270E | S267B/I332E |
| R255L/P390L/D270E | R267L/A327S |
| R255L/P396L/D270E | S267Q/A327S |
| R255L/P396L/D270E/R292G | S298A/I332E |
| R255L/P396L/D270E | 3304T/I332E |
| R255L/P396L/D270E/Y300L | S324G/I332D |
| F243L/D270E/K392N/P396L | S324G/I332E |
| F243L/R255L/D270E/P396L | S3241/I332D |
| F243L/R292P/Y300L/V305I/P396L | S324I/I332E |
| F243L/R292P/Y300L/P396L | T260H/I332E |
| F243L/R292P/Y300L | T335D/1332E |
| F243L/R292P/P396L | V240I/V266I |
| F243L/R292P/V305I | V264I/I332E |
| F243L/R292P | D265F/N297B/J332E |
| S29SA/E333A/K334A | D265Y/M297D/I332E |
| E380A/T307A | F243L/V262I/V264W |
| K326M/E333S | N2970/A330Y/I332E |
| K326A/E333A | K297D/T299S/I332E |
| S317A/K353A | N2970/T299F/I332E |
| A327D/I332E | N297D/T299H/I332E |
| A330L/I332E | N291T)/I299I/I332E |
| A330Y/I332E | N297D/T299L/I332E |
| E258H/I332E | N297D/T299V/I332E |
| E272H/I332E | P230A/E233D/I332E |
| K272I/K276D | P244H/P245A/P247V |
| E272R/I332E | S239D/A330L/I332E |
| K283H/I332E | S239D/A330Y/I332E |
| E293R/I332E | S239D/H268E/A330Y |
| F241L/V262I | S239N/I332E/A327A |
| F241W/P243W | S239D/I332E/A330I |

[Table 3-2]

| | |
|---|---|
| F243L/V26I | S239D/N297D/I332E |
| H268D/A330Y | S239D/S298A/I332E |
| H268E/A330Y | S239D/V264I/I332E |
| K246H/I332E | S239E/N297D/I332E |

(continued)

| | |
|---|---|
| L234D/I332E | S239E/V264I/I332E |
| L234E/I332E | S339K/A330L/I332E |
| L234G/I332E | S239N/A330V/I332E |
| L234I/I332E | S239N/S298A/J332B |
| L234I/J/235D | S239Q/V264I/I332E |
| L234Y/I332E | V264E/N297/I332E |
| L235D/I332E | V264I/A330L/I332S |
| L235E/I332E | V264I/A330Y/I332E |
| L235I/I332E | V264I/S298A/I332E |
| L235S/I332E | Y290D/N297D/I332E |
| L328A/I332D | Y296E/N297D/S332E |
| L328D/I332D | Y296H/N297D/I332E |
| L328D/I332E | Y296N/N297D/I332E |
| L32SE/I332D | Y296Q/N297I/I332E |
| L328E/I332E | Y296T/N297D/I332E |
| L328F/I332D | D265Y/N297D/T299L/I332E |
| L32SP/I332E | F241E/F243Q/V262T/V264E |
| L328H/I332E | F241E/F243R/V264E/V264R |
| L328I/J332D | F241E/K243Y/V262T/V264R |
| L328I/I332E | F241L/F243L/V262I/V264I |
| L328M/I332D | F241R/P243Q/V262T/V264R |
| L328M/I332K | F241S/F234H/V262T/V364T |
| L378N/1332D | F241W/F243W/V262A/V264A |
| L32SN/I332K | F241Y/F243Y/V2621T/V264T |
| L328Q/I332D | I332E/A330Y/H268E/A327A |
| L326Q/1332E | N297D/I332E/S239D/A330L |
| L328T/I332D | N297D/S298A/A320Y/I332E |
| L32ST/I332K | V239D/A330Y/I332E/K326E |
| L328V/I332D | S239D/A330Y/I332E/K326T |
| L328V/I332E | S239D/A332Y/I332E/L234I |
| L328Y/I332D | S239D/A330Y/I332E/L235D |

[Table 3-3]

| | |
|---|---|
| L328Y/I332E | S239D/A330Y/I332E/V240I |
| N297D/I332E | S239D/A330Y/I332E/V264T |
| N297E/I332E | S239D/A330Y/I332E/V266I |
| N297S/I332K | S239D/D265F/N297D/I332E |
| P227G/I332E | S239D/D265H/N297D/I332E |

(continued)

| | |
|---|---|
| P230A/E233D | S239D/D265I/N297D/I332E |
| Q295E/I332E | S239D/D265L/N297D/I332E |
| R255Y/I332E | S239D/D265T/N297D/I332E |
| S239B/B32D | S239D/D265V/N297D/I332E |
| S239D/I332E | S239D/S265Y/N297D/I332E |
| S239D/I332N | S239D/I332E/A330Y/A327A |
| S239D/I332Q | S239D/I332K/H268E/A327A |
| S239E/D265G | S239D/I333E/H268E/A330V |
| S239E/D265N | S299D/N297D/I332E/A330Y |
| S239E/D265Q | S339D/N297D/I332E/K326E |
| S239E/I332D | S239D/N297D/5332E/I235D |
| S239E/I332E | S239D/V264I/A330L/I332E |
| S239E/I332N | S239D/V264I/S298A/I332E |
| S239E/I332Q | S239E/V264I/A330Y/I332K |
| S239N/I332D | F241E/F243Q/V262T/V264E/I332E |
| S239N/I332E | F241E/F243R/V26SE/V264R/I332E |
| S239N/I332N | FS41E/F243Y/V262T/V264R/I332E |
| S239N/I332Q | F241R/F2432Q/V262T/V264R/I332E |
| S239Q/I332D | S239D/I332E/H268E/A330Y/A327A |
| S239Q/I332E | S239E/V264I/S298A/A330Y/I332E |
| S239Q/I332N | F241Y/F243Y/V262T/V264T/N297D/I332E |
| S267E/L328F | G236D/S267B |
| S239D/S267E | |

The Fcγ receptor-binding domain contained in the anti-GPC3 antibody of the present invention can be assayed for its binding activity against the Fcγ receptor appropriately using pH conditions selected from acidic to neutral regions of pH. The acidic to neutral regions of pH as the conditions under which the Fcγ receptor-binding domain contained in the antigen-binding molecule of the present invention is assayed for its binding activity against the Fcγ receptor usually mean pH 5.8 to pH 8.0. The pH range is preferably indicated by arbitrary pH values from pH 6.0 to pH 7.4 and is preferably selected from pH 6.0, 6.1, 6.2, 6.3, 6.4, 6.5, 6.6, 6.7, 6.8, 6.9, 7.0, 7.1, 7.2, 7.3, and 7.4. Particularly, a pH range of 6.15 to 7.4, which is close to the pH of cancer tissues, is preferred (Vaupel et al., Cancer Res. (1989) 49, 6449-6665). The binding affinity of the Fc region for the human Fcγ receptor can be evaluated under assay conditions involving an arbitrary temperature of 10°C to 50°C. Preferably, a temperature of 15°C to 40°C is used for determining the binding affinity of the Fc region for the human Fcγ receptor. More preferably, an arbitrary temperature of 20°C to 35°C, for example, any one temperature of 20, 21, 22, 23, 24, 25, 26, 27, 28, 29, 30, 31, 32, 33, 34, and 35°C, is also used for determining the binding affinity of the Fc region for the Fcγ receptor. The temperature 25°C is one non-limiting example in an aspect of the present invention.

The phrase "FcyR-binding modified Fc region having higher binding activity against Fcγ receptors than that of the native Fc region against Fcγ receptors" described herein means that the FcyR-binding modified Fc region has higher binding activity against any of the human Fcγ receptors FcyRI, FcyRIIa, FcyRIIb, FcyRIIIa, and /or FcyRIIIb than that of the native Fc region against the human Fcγ receptor. The phrase means that, for example, on the basis of the analysis method described above, the anti-GPC3 antibody comprising the FcyR-binding modified Fc region exhibits 105% or more, preferably 110% or more, 115% or more, 120% or more, or 125% or more, particularly preferably 130% or more, 135% or more, 140% or more, 145% or more, 150% or more, 155% or more, 160% or more, 165% or more, 170% or more, 175% or more, 180% or more, 185% or more, 190% or more, 195% or more, 2 times or more, 2.5 times or more, 3 times or more, 3.5 times or more, 4 times or more, 4.5 times or more, 5 times or more, 7.5 times or more, 10 times or more, 20 times or more, 30 times or more, 40 times or more, 50 times or more, 60 times or more, 70 times or more, 80 times or more, 90 times or more, 100 times or more binding activity compared with the binding activity of an anti-GPC3 antibody comprising the native Fc region of human IgG serving as a control. The native Fc region used may be the starting Fc region or may be the native Fc region of an antibody of the same subclass as the anti-GPC antibody concerned.

In the present invention, a native human IgG Fc region having a fucose-containing sugar chain as a sugar chain bound to amino acid 297 (EU numbering) is preferably used as the native Fc region of human IgG serving as a control. Whether or not the sugar chain bound to amino acid 297 (EU numbering) is a fucose-containing sugar chain can be confirmed using an approach known in the art. Whether or not the sugar chain bound to the native human IgG Fc region is a fucose-containing sugar chain can be determined by, for example, a method as shown below. The native human IgG to be tested liberates a sugar chain through reaction with N-Glycosidase F (Roche Diagnostics K.K.) (Weitzhandler et al., J. Pharma. Sciences (1994) 83, 12, 1670-1675). Next, proteins are removed through reaction with ethanol, and the resulting reaction solution (Schenk et al., J. Clin. Investigation (2001) 108 (11) 1687-1695) is evaporated to dryness and then fluorescently labeled with 2-aminobenzamide (Bigge et al., Anal. Biochem. (1995) 230 (2) 229-238). After removal of the reagent by solidphase extraction using a cellulose cartridge, the 2-AB-fluorescently labeled sugar chain is analyzed by normal-phase chromatography. The detected peak in the chromatogram can be observed to thereby determine whether or not the sugar chain bound to the native Fc region of human IgG is a fucose-containing sugar chain.

An anti-GPC3 antibody having an IgG monoclonal antibody Fc region can be appropriately used as the anti-GPC3 antibody comprising the native Fc region of an antibody of the same subclass serving as a control. Structural examples of the Fc region include Fc regions contained in constant regions represented by SEQ ID NOs: 74 (having A added to the N terminus of the sequence of database registration No. AAC82527.1), 75 (having A added to the N terminus of the sequence of database registration No. AAB59393.1), 76 (database registration No. CAA27268.1), and 77 (having A added to the N terminus of the sequence of database registration No. AAB59394.1). In the case of using a certain isotype of anti-GPC3 antibody as a test substance, the anti-GPC3 antibody comprising the Fc region to be tested is studied for its effect of binding activity against Fcγ receptors by use of an anti-GPC3 antibody of the certain isotype as a control. The anti-GPC3 antibody comprising the Fc region thus confirmed to have higher binding activity against Fcγ receptors is appropriately selected.

Fc region having higher binding activity against active Fcγ receptor than its binding activity against inhibitory Fcγ receptor

As described above, preferred examples of the active Fcγ receptors include FcyRI (CD64) including FcyRIa, FcyRIb, and FcyRIc, FcyRIIa, and FcγRIII (CD16) including isoforms FcyRIIIa (including allotypes V158 and F158) and FcyRIIIb (including allotypes FcyRIIIb-NAl and FcyRIIb-NA2). Preferred examples of the inhibitory Fcγ receptors include FcyRIIb (including FcyRIIb-1 and FcyRIIb-2) .

In a non-limiting aspect, alternative examples of the anti-GPC3 antibody of the present invention include an anti-GPC3 antibody comprising an Fc region having higher binding activity against active Fcγ receptors than its binding activity against inhibitory Fcγ receptors. In this case, the phrase "having higher binding activity against active Fcγ receptors than its binding activity against inhibitory Fcγ receptors" means that the Fc region has higher binding activity against any of the human Fcγ receptors FcyRIa, FcyRIIa, FcyRIIIa, and/or FcyRIIIb than its binding activity against FcyRIIb. The phrase means that, for example, on the basis of the analysis method described above, the antigen-binding molecule comprising the Fc region exhibits 105% or more, preferably 110% or more, 120% or more, 130% or more, or 140% or more, particularly preferably 150% or more, 160% or more, 170% or more, 180% or more, 190% or more, 200% or more, 250% or more, 300% or more, 350% or more, 400% or more, 450% or more, 500% or more, 750% or more, 10 times or more, 20 times or more, 30 times or more, 40 times or more, 50 times, 60 times, 70 times, 80 times, 90 times, or 100 times or more binding activity against any of the human Fcγ receptors FcyRIa, FcyRIIa, FcyRIIIa, and/or FcyRIIIb compared with its binding activity against FcyRIIb. The IgG antibody comprising such an Fc region is known to have enhancement in the ADCC activity. Thus, the anti-GPC3 antibody comprising the Fc region is useful as the GPC3-targeting drug of the present invention.

In a non-limiting aspect of the present invention, examples of the Fc region having higher binding activity against active Fcγ receptors than its binding activity against inhibitory Fcγ receptors (having selective binding activity against active Fcγ receptors) preferably include Fc regions in which at least one or more amino acids selected from the group consisting of position 221, position 222, position 223, position 224, position 225, position 227, position 228, position 230, position 231, position 232, position 233, position 234, position 235, position 236, position 237, position 238, position 239, position 240, position 241, position 243, position 244, position 245, position 246, position 247, position 249, position 250, position 251, position 254, position 255, position 256, position 258, position 260, position 262, position 263, position 264, position 265, position 266, position 267, position 268, position 269, position 270, position 271, position 272, position 273, position 274, position 275, position 276, position 278, position 279, position 280, position 281, position 282, position 283, position 284, position 285, position 286, position 288, position 290, position 291, position 292, position 293, position 294, position 295, position 296, position 297, position 298, position 299, position 300, position 301, position 302, position 303, position 304, position 305, position 311, position 313, position 315, position 317, position 318, position 320, position 322, position 323, position 324, position 325, position 326, position 327, position 328, position 329, position 330, position 331, position 332, position 333, position 334, position 335, position 336, position 337, position 339, position 376, position 377, position 378, position 379, position 380, position 382, position 385, position 392, position 396, position 421, position 427, position 428, position 429, position 434, position 436 and position 440 (EU numbering)
are modified to amino acids different from those in the native Fc region.
In a non-limiting aspect of the present invention, further examples of the Fc region having higher binding activity against active Fcγ receptors than its binding activity against inhibitory Fcγ receptors (having selective binding activity against active Fcγ receptors) preferably include Fc regions in which a plurality of amino acids described in Tables 3-1 to 3-3 are modified to amino acids different from those in the native Fc region.

Fc region having modified sugar chain

The Fc region contained in the anti-GPC3 antibody provided by the present invention can also include an Fc region modified such that a higher proportion of fucosedeficient sugar chains is bound to the Fc region or a higher proportion of bisecting N-acetylglucosamine is added to the Fc region in the composition of sugar chains bound to the Fc region. The removal of a fucose residue from N-acetylglucosamine at the reducing end of a N-glycoside-linked complex sugar chain bound to an antibody Fc region is known to enhance its affinity for FcyRIIIa (Sato et al., Expert Opin. Biol. Ther. (2006) 6 (11), 1161-1173). An IgG1 antibody comprising such an Fc region is known to have enhancement in the ADCC activity. Thus, the antigen-binding molecule comprising the Fc region is also useful as the antigen-binding molecule contained in the pharmaceutical composition of the present invention. Examples of an antibody that lacks a fucose residue in N-acetylglucosamine at the reducing end of a N-glycoside-linked complex sugar chain bound to the antibody Fc region include the following antibodies: glycosylated antibodies (e.g., International Publication No. WO1999/054342); and
antibodies deficient in fucose added to the sugar chain (e.g., International Publication Nos. WO2000/061739, WO2002/031140, and WO2006/067913).
Also, WO2006/046751 and WO2009/ 041062 disclose specific examples of the anti-GPC3 antibody comprising the Fc region modified such that a higher proportion of fucosedeficient sugar chains is bound to the Fc region or a higher proportion of bisecting N-acetylglucosamine is added to the Fc region in the composition of sugar chains bound to the Fc region.
More specifically, in an alternative non-limiting aspect of the antibody that lacks a fucose residue in N-acetylglucosamine at the reducing end of a N-glycoside-linked complex sugar chain bound to the antibody Fc region, the antibody deficient in fucose added to the sugar chain (e.g., International Publication Nos. WO2000/061739, WO2002/031140, and WO2006/067913) may be prepared. For this purpose, host cells less able to add fucose to sugar chains are prepared as a result of altering the activity of forming the sugar chain structures of polypeptides that undergo sugar chain modification. The host cells are allowed to express the desired antibody gene, and the antibody deficient in fucose in its sugar chain can be recovered from the culture solution of the host cells. Non-limiting preferred examples of the activity of forming the sugar chain structures of polypeptides can include the activity of an enzyme or a transporter selected from the group consisting of fucosyltransferase (EC 2.4.1.152), fucose transporter (SLC35C1), GDP-mannose 4,6-dehydratase (GMD) (EC 4.2.1.47), GDP-keto-6-deoxymannose 3,5-epimerase/4-reductase (Fx) (EC 1.1.1.271), and GDP-β-L-fucose pyrophosphorylase (GFPP) (EC 2.7.7.30). These enzymes or transporters are not necessarily limited by their structures as long as the enzymes or the transporters can exert their activity. These proteins capable of exerting such activity are referred to as functional proteins herein. In a non-limiting aspect, examples of methods for altering the activity include the deletion of the activity. Host cells that lack the activity can be prepared by an appropriately adopted method known in the art such as a method which involves disrupting the genes of these functional proteins to render the genes unfunctional (e.g., International Publication Nos. WO2000/061739, WO2002/031140, and WO2006/067913). Such host cells that lack the activity may be prepared by, for example, a method which involves disrupting the endogenous genes of these functional proteins in cells such as CHO cells, BHK cells, NS0 cells, SP2/0 cells, YO myeloma cells, P3X63 mouse myeloma cells, PER cells, PER.C6 cells, HEK293 cells, or hybridoma cells to render the genes unfunctional.
Antibodies containing sugar chains having bisecting GlcNAc (e.g., International Publication No. WO2002/079255) are known in the art. In a non-limiting aspect, host cells expressing genes encoding functional proteins having β-1,4-mannosyl-glycoprotein 4-β-N-acetylglucosaminyltransferase (GnTIII) (EC 2.4.1.144) activity or β-1,4-galactosyltransferase (GalT) (EC 2.4.1.38) activity are prepared in order to prepare such an antibody containing sugar chains having bisecting GlcNAc. In another non-limiting preferred aspect, host cells coexpressing a gene encoding a functional protein having human mannosidase II (ManII) (3.2.1.114) activity, a gene encoding a functional protein having β-1,2-acetylglucosaminyltransferase I (GnTI) (EC 2.4.1.94) activity, a gene encoding a functional protein having β-1,2-acetylglucosaminyltransferase II (GnTII) (EC 2.4.1.143) activity, a gene encoding a functional protein having mannosidase I (ManI) (EC 3.2.1.113) activity, and an α-1,6-fucosyltransferase (EC 2.4.1.68) gene, in addition to the functional proteins described above, are prepared (International Publication Nos. WO2004/065540).

The host cells less able to add fucose to sugar chains and the host cells having the activity of forming sugar chains having bisecting GlcNAc structures as described above can be transformed with antibody genecontaining expression vectors to respectively prepare the antibody that lacks a fucose residue in N-acetylglucosamine at the reducing end of a N-glycoside-linked complex sugar chain bound to the antibody Fc region and the antibody containing sugar chains having bisecting GlcNAc. The methods for producing these antibodies are also applicable to a method for producing the antigen-binding molecule comprising the Fc region modified such that a higher proportion of fucosedeficient sugar chains is bound to the Fc region or a higher proportion of bisecting N-acetylglucosamine is added to the Fc region in the composition of sugar chains bound to the Fc region of the present invention. The composition of sugar chains bound to the Fc region contained in the antigen-binding molecule of the present invention prepared by such a production method can be confirmed by the method described in the paragraph "Fcγ receptor (FcyR)-binding modified Fc region".

Anti-GPC3 antibody having altered isoelectric point

In a non-limiting aspect, further examples of the anti-GPC3 antibody that may be used in the present invention include an anti-GPC3 antibody having an amino acid residue modified to alter its isoelectric point (pI). Preferred examples of the "alteration of the electric charge of an amino acid residue" in the anti-GPC3 antibody provided by the present invention are as follows: alteration to increase the pI value can be performed by, for example, at least one substitution selected from the substitution of Q by K at position 43, the substitution of D by N at position 52, and the substitution of Q by R at position 105 based on the Kabat numbering in the anti-GPC3 antibody heavy chain variable region represented by SEQ ID NO: 50, which is consequently modified to, for example, the amino acid sequence represented by SEQ ID NO: 67. Also, this alteration can be performed by, for example, at least one substitution selected from the substitution of E by Q at position 17, the substitution of Q by R at position 27, and the substitution of Q by R at position 105 based on the Kabat numbering in the anti-GPC3 antibody light chain variable region represented by SEQ ID NO: 51 or 66, which is consequently modified to, for example, the amino acid sequence represented by SEQ ID NO: 68. On the other hand, alteration to decrease the pI value can be performed by at least one substitution selected from the substitution of K by T at position 19, the substitution of Q by E at position 43, the substitution of G by E at position 61, the substitution of K by S at position 62, the substitution of K by Q at position 64, and the substitution of G by D at position 65 based on the Kabat numbering in the anti-GPC3 antibody heavy chain variable region represented by SEQ ID NO: 50, which is consequently modified to, for example, the amino acid sequence represented by SEQ ID NO: 69 or 71. Also, this alteration can be performed by, for example, at least one substitution selected from the substitution of R by Q at position 24, the substitution of Q by E at position 27, the substitution of K by T at position 74, the substitution of R by S at position 77, and the substitution of K by E at position 107 based on the Kabat numbering in the anti-GPC3 antibody light chain variable region represented by SEQ ID NO: 51 or 66, which is consequently modified to, for example, the amino acid sequence represented by SEQ ID NO: 70, 72, or 73. Further examples of the alteration to decrease the pI value include the substitution of at least one amino acid selected from amino acids 268, 274, 355, 356, 358, and 419 based on the EU numbering in the heavy chain constant region represented by SEQ ID NO: 74. Preferred examples of these substitutions can include at least one substitution selected from the substitution of H by Q at position 268, the substitution of K by Q at position 274, the substitution of R by Q at position 355, the substitution of D by E at position 356, the substitution of L by M at position 358, and the substitution of Q by E at position 419 based on the EU numbering in the heavy chain constant region represented by SEQ ID NO: 31. As a result of these substitutions, a chimera having human antibody IgG1 and IgG4 constant regions is constructed. Specifically, these substitutions can yield an antibody having the desired pI without influencing the immunogenicity of the modified antibody.

Modification to reduce heterogeneity

An IgG constant region deficient in Gly at position 446 and Lys at position 447 based on the EU numbering in the IgG constant region represented by SEQ ID NO: 74, 75, 76, or 77 may also be used as the constant region contained in the anti-GPC3 antibody of the present invention. Deficiency in both of these amino acids can reduce heterogeneity derived from the end of the heavy chain constant region of the antibody.

Antibody modification

The posttranslational modification of a polypeptide refers to chemical modification given to the polypeptide translated during polypeptide biosynthesis. Since the primary structure of an antibody is composed of a polypeptide, the anti-GPC3 antibody of the present invention also includes a modified form that has received the posttranslational modification of the polypeptide constituting the primary structure of the anti-GPC3 antibody. The posttranslational modification of a polypeptide can be broadly classified into the addition of a functional group, the addition of a polypeptide or a peptide (ISGylation, SUMOylation, or ubiquitination), the conversion of the chemical properties of an amino acid (silylation, deamination, or deamidation), and structural conversion (disulfidation or protease degradation). In a non-limiting aspect, examples of the posttranslational modification according to the present invention include the addition of a peptide or a functional group to an amino acid residue as a unit constituting the polypeptide. Examples of such modification can specifically include phosphorylation (serine, threonine, tyrosine, aspartic acid, etc.), glucosylation (serine, threonine, aspartic acid, etc.), acylation (lysine), acetylation (lysine), hydroxylation (lysine and proline), prenylation (cysteine), palmitoylation (cysteine), alkylation (lysine and arginine), polyglutamylation (glutamic acid), carboxylation (glutamic acid), polyglycylation (glutamic acid), citrullination (arginine), and succinimide formation (aspartic acid). For example, an anti-GPC3 antibody that has received the modification of N-terminal glutamine to pyroglutamic acid by pyroglutamylation is also included in the anti-GPC3 antibody of the present invention, as a matter of course. Also, for example, a posttranslationally modified anti-GPC3 antibody comprising heavy and light chains or heavy chains linked via a "disulfide bond", which means a covalent bond formed between two sulfur atoms is included in the anti-GPC3 antibody of the present invention. A thiol group contained in an amino acid cysteine can form a disulfide bond or crosslink with a second thiol group. In general IgG molecules, CH1 and CL regions are linked via a disulfide bond, and two polypeptides constituting heavy chains are linked via a disulfide bond between cysteine residues at positions 226 and 229 based on the EU numbering. A posttranslationally modified anti-GPC3 antibody having such a linkage via a disulfide bond is also included in the anti-GPC3 antibody of the present invention.

GPC3-targeting drug therapy

The term "GPC3-targeting drug therapy" refers to the administration of a GPC3-targeting drug to a patient. In the present invention the GPC3-targeting drug is an anti-GPC3 antibody.

The phrase "efficacy of GPC3-targeting drug therapy for cancer" or "GPC3-targeting drug therapy has efficacy for cancer" means that the GPC3-targeting drug therapy produces desired or beneficial effects on a patient diagnosed with cancer. The desired or beneficial effects can include: (1) the inhibition of the further growth or diffusion of cancer cells; (2) the killing of cancer cells; (3) the inhibition of cancer recurrence; (4) the alleviation, reduction, mitigation, or inhibition of cancer-related symptoms (pain, etc.) or reduction in the frequency of the symptoms; and (5) improvement in the survival rate of the patient. The inhibition of cancer recurrence includes the inhibition of the growth of cancer already treated by radiation, chemotherapy, surgical operation, or other techniques, at the primary site of the cancer and its neighboring tissues, and the absence of the growth of cancer at a new distal site. The desired or beneficial effects may be subjectively perceived by the patient or may be objectively found. In the case of, for example, a human patient, the human is able to recognize improvement in energy or vitality or reduction in pain as improvement or a therapy-responsive sign perceived by the patient. Alternatively, a clinician is able to notice decrease in tumor size or the amount of tumor tissues on the basis of findings gained by physical examination, experimental parameters, tumor markers, or X-ray photography. Some experimental signs that can be observed by the clinician in response to treatment include normalized test results of, for example, leukocyte counts, erythrocyte counts, platelet counts, erythrocyte sedimentation rates, and levels of various enzymes. The clinician is further able to observe decrease in detectable tumor marker level. Alternatively, other tests, such as sonography, nuclear magnetic resonance test, and positron emission test, may be used for evaluating objective improvement.

Any cancer having high expression of targeted GPC3 corresponds to the cancer to be treated by the GPC3-targeting drug therapy of the present invention. One example of such cancer include cancer selected from breast cancer, uterine cervix cancer, colon cancer, uterine body cancer, head and neck cancer, liver cancer, lung cancer, malignant carcinoid, malignant glioma, malignant lymphoma, malignant melanoma, ovary cancer, pancreatic cancer, prostatic cancer, renal cancer, skin cancer, gastric cancer, testicle cancer, thyroid cancer, urothelial cancer, and the like.

Method for determining efficacy of GPC3-targeting drug therapy or method for determining continuation of GPC3-targeting drug therapy

The present invention provides method for determining the efficacy of GPC3-targeting drug therapy for GPC3-expressing cancer in a patient or determining the continuation of GPC3-targeting drug therapy for a patient, comprising monitoring a concentration of free GPC3 in a biological sample isolated from the patient before the start of GPC3-targeting drug therapy and/or the patient treated with the GPC3-targeting drug therapy, wherein the GPC3-targeing drug comprises an anti-GPC3 antibody, wherein the biological sample is blood, plasma, or serum, and wherein when the concentration of free GPC3 is above a predetermined value, the GPC3-targeting drug therapy is determined to be effective or the GPC3-targeting drug therapy is determined to be. The "patient before the start of GPC3-targeting drug therapy" refers to a patient diagnosed with cancer, having no history of administration of the GPC3-targeting drug. The patient may be a patient for which the efficacy of the GPC3-targeting drug therapy has been determined from the expression level of GPC3 in the tissues. Further, the "patient treated with GPC3-targeting drug therapy" refers to a patient having a history of administration of the GPC3-targeting drug. The administration route of the GPC3-targeting drug can be appropriately selected from administration routes suitable for the properties, etc., of the GPC3-targeting drug to be administered. Examples of the administration route include parenteral administration. Further examples of the parenteral administration include injection, transnasal administration, transpulmonary administration, and percutaneous administration. Further examples of the injection include systemic or local administration based on intravenous injection, intramuscular injection, intraperitoneal injection, and subcutaneous injection.

Known results gained by conventional techniques before the completion of the present invention show that free GPC3 secreted into plasma by processing at a particular site in the polypeptide sequence of GPC3 by an enzyme such as convertase, phospholipase D, or Notum is detected in plasma isolated from liver cancer patients, whereas free GPC3 is not detected in plasma isolated from healthy individuals (Patent Literature 7, etc.). It has been expected from such results that the concentration of free GPC3 detected in serum or plasma is decreased over time with the continuation of the treatment, if the GPC3-targeting drug therapy has efficacy. As a result of conducting diligent studies under such circumstances, surprisingly, the present inventors have found that the concentration of free GPC3 is stabilized or increased, rather than decreased, in serum or plasma isolated from a patient with stable disease that may respond to the GPC3-targeting drug therapy. The present inventors have also found that when the concentration of free GPC3 detected in serum or plasma before administration of GPC3-targeting drug therapy is equal to or higher than the predetermined concentration, the efficacy of the GPC3-targeting drug therapy is determined.

The present invention provides a method method for determining the efficacy of GPC3-targeting drug therapy for GPC3-expressing cancer in a patient or determining the continuation of GPC3-targeting drug therapy for a patient, comprising monitoring a concentration of free GPC3 in a biological sample isolated from the patient before the start of GPC3-targeting drug therapy and/or the patient treated with the GPC3-targeting drug therapy, wherein the GPC3-targeing drug comprises an anti-GPC3 antibody, wherein the biological sample is blood, plasma, or serum, and wherein when the concentration of free GPC3 is above a predetermined value, the GPC3-targeting drug therapy is determined to be effective or the GPC3-targeting drug therapy is determined to be continuedThe predetermined value may be determined from particular values such as 0.1 ng/mL, 0.2 ng/mL, 0.3 ng/mL, 0.4 ng/mL, 0.5 ng/mL, 0.6 ng/mL, 0.7 ng/mL, 0.8 ng/mL, 0.9 ng/mL, 1.0 ng/mL, 2.0 ng/mL, 3.0 ng/mL, 4.0 ng/mL, 5.0 ng/mL, 6.0 ng/mL, 7.0 ng/mL, 8.0 ng/mL, 9.0 ng/mL, 10.0 ng/mL, 15.0 ng/mL, 20.0 ng/mL, 25.0 ng/mL, 30.0 ng/mL, 35.0 ng/mL, 40.0 ng/mL, 45.0 ng/mL, 50.0 ng/mL, 55.0 ng/mL, 60.0 ng/mL, 65.0 ng/mL, 70.0 ng/mL, 75.0ng/mL, 80.0 ng/mL, 85.0 ng/mL, 90.0 ng/mL, 100.0 ng/mL or may be determined as a numerical range containing particular values arbitrarily selected as the upper and lower limits from the above group of particular values. As an example, such a numerical range can be appropriately selected from numerical ranges of 0.1 ng/mL to 100 ng/mL. Examples of the numeric range include 0.1 to 100 ng/mL, 0.5 to 80 ng/mL, 1.0 to 60 ng/mL, 2.0 to 55 ng/mL, 3.0 to 50 ng/mL, 4.0 to 45 ng/mL, 5.0 to 40 ng/mL, 6.0 to 35 ng/mL, 7.0 to 30 ng/mL, 8.0 to 25 ng/mL, 9.0 to 20 ng/mL, and 10 to 20 ng/mL. The numerical range is, for example, preferably 0.1 to 0.35 ng/mL, more preferably 0.15 to 0.3 ng/mL, though the numerical range of the present invention is not limited to these ranges. The predetermined value of the concentration of free GPC3 can slightly vary depending on many factors, for example, the assay method used, the type of a sample for free GPC3 assay, storage conditions (e.g., temperature and duration) of the sample, and the ethnic identity of the patient. In the method for predicting, expecting, or determining the efficacy or determining the continuation of the therapy, a concentration in a blood, plasma, or serum sample isolated from the patient is measured as the concentration of free GPC3.

The concentration of free GPC3 can be measured in a sample isolated before and/or after the start of the GPC3-targeting drug therapy and may be measured in a plurality of samples collected at predetermined time intervals.. The predetermined time intervals are appropriately set. In a non-limiting aspect of the intervals, the samples can be collected at intervals of 1 day, 2 days, 3 days, 4 days, 5 days, 6 days, 7 days (i.e., 1 week), 8 days, 9 days, 10 days, 11 days, 12 days, 13 days, 14 days (i.e., 2 weeks), 15 days, 16 days, 17 days, 18 days, 19 days, 20 days, 21 days (i.e., 3 weeks), 22 days, 23 days, 24 days, 25 days, 26 days, 27 days, 28 days (i.e., 4 weeks), 29 days, 30 days, 1 month, 5 weeks, 6 weeks, 7 weeks, 8 weeks, 9 weeks, 10 weeks, 2 months, 3 months, 4 months, 5 months, or 6 months after the initial administration of the GPC3-targeting drug, or at arbitrary points in time between the start and completion of the therapy, for example, after 1, 2, 3, 4 or more treatment cycles. The dosing intervals, i.e., the treatment cycles, can be appropriately set. One non-limiting example thereof includes 1 day, 2 days, 3 days, 4 days, 5 days, 6 days, 7 days (i.e., 1 week), 8 days, 9 days, 10 days, 11 days, 12 days, 13 days, 14 days (i.e., 2 weeks), 15 days, 16 days, 17 days, 18 days, 19 days, 20 days, 21 days (i.e., 3 weeks), 22 days, 23 days, 24 days, 25 days, 26 days, 27 days, 28 days (i.e., 4 weeks), 29 days, 30 days, 1 month, 5 weeks, 6 weeks, 7 weeks, 8 weeks, 9 weeks, 10 weeks, 2 months, 3 months, 4 months, 5 months, or 6 months.

Also disclosed is a method that comprises monitoring a concentration of free GPC3 in blood, plasma, or serum isolated 30 days or 1 month after the start of GPC3-targeting drug therapy from the patient treated with the therapy, wherein when the concentration of free GPC3 ranges from 0.1 ng/mL to 100 ng/mL, the efficacy of the GPC3-targeting drug therapy is determined. Also disclosed is a method that comprises monitoring a concentration of free GPC3 in blood, plasma, or serum isolated 2 months, 3 months, 4 months, 5 months, or 6 months after the start of GPC3-targeting drug therapy from the patient treated with the therapy, wherein when the concentration of free GPC3 ranges from 0.1 ng/mL to 100 ng/mL, the efficacy of the GPC3-targeting drug therapy is determined.

Also disclosed is a method that comprises monitoring a concentration of free GPC3 in blood, plasma, or serum isolated 30 days or 1 month after the start of GPC3-targeting drug therapy from the patient treated with the therapy, wherein when the concentration of free GPC3 ranges from 0.1 ng/mL to 100 ng/mL, the continuation of the GPC3-targeting drug therapy is determined. Also disclosed is a method that comprises monitoring a concentration of free GPC3 in blood, plasma, or serum isolated 2 months, 3 months, 4 months, 5 months, or 6 months after the start of GPC3-targeting drug therapy from the patient treated with the therapy, wherein when the concentration of free GPC3 ranges from 0.1 ng/mL to 100 ng/mL, the continuation of the GPC3-targeting drug therapy is determined.

In another non-limiting aspect of the present invention, the concentration of free GPC3 can be compared with a concentration of free GPC3 ("baseline concentration") measured in a blood, plasma, or serum sample isolated before the start of the GPC3-targeting drug therapy from the patient. In this aspect, the "predetermined value" of the concentration of free GPC3 means that the concentration of free GPC3 in the biological sample isolated from the patient treated with the GPC3-targeting drug therapy is higher than the baseline concentration. Specifically, when the concentration of free GPC3 after the start of the GPC3-targeting drug therapy is larger than that before the start of the therapy in one patient, the efficacy of the GPC3-targeting drug therapy for cancer in the patient is predicted, expected, or determined or the continuation of the therapy is determined. The rate at which the concentration of free GPC3 after the start of the GPC3-targeting drug therapy is larger than that before the start of the therapy can be appropriately selected by those skilled in the art and is not limited to a particular value. Such a rate can be appropriately selected from a numerical range of 1 time to $10^6$ times. When the rate is, for example, 1 time or more, 1.05 times or more, 1.1 times or more, 1.2 times or more, 1.3 times or more, 1.4 times or more, 1.5 times or more, 1.6 times or more, 1.7 times or more, 1.8 times or more, 1.9 times or more, 2 times or more, 2.1 times or more, 2.2 times or more, 2.3 times or more, 2.4 times or more, 2.5 times or more, 2.6 times or more, 2.7 times or more, 2.8 times or more, 2.9 times or more, 3 times or more, 3.1 times or more, 3.2 times or more, 3.3 times or more, 3.4 times or more, 3.5 times or more, 3.6 times or more, 3.7 times or more, 3.8 times or more, 3.9 times or more, 4 times or more, 4.1 times or more, 4.2 times or more, 4.3 times or more, 4.4 times or more, 4.5 times or more, 4.6 times or more, 4.7 times or more, 4.8 times or more, 4.9 times or more, 5 times or more, 5.1 times or more, 5.2 times or more, 5.3 times or more, 5.4 times or more, 5.5 times or more, 5.6 times or more, 5.7 times or more, 5.8 times or more, 5.9 times or more, 6 times or more, 6.1 times or more, 6.2 times or more, 6.3 times or more, 6.4 times or more, 6.5 times or more, 6.6 times or more, 6.7 times or more, 6.8 times or more, 6.9 times or more, 7 times or more, 7.1 times or more, 7.2 times or more, 7.3 times or more, 7.4 times or more, 7.5 times or more, 7.6 times or more, 7.7 times or more, 7.8 times or more, 7.9 times or more, 8 times or more, 8.1 times or more, 8.2 times or more, 8.3 times or more, 8.4 times or more, 8.5 times or more, 8.6 times or more, 8.7 times or more, 8.8 times or more, 8.9 times or more, 9 times or more, 9.1 times or more, 9.2 times or more, 9.3 times or more, 9.4 times or more, 9.5 times or more, 9.6 times or more, 9.7 times or more, 9.8 times or more, 9.9 times or more, 10 times or more, 11 times or more, 12 times or more, 13 times or more, 14 times or more, 15 times or more, 16 times or more, 17 times or more, 18 times or more, 19 times or more, 20 times or more, 21 times or more, 22 times or more, 23 times or more, 24 times or more, 25 times or more, 26 times or more, 27 times or more, 28 times or more, 29 times or more, 30 times or more, 31 times or more, 32 times or more, 33 times or more, 34 times or more, 35 times or more, 36 times or more, 37 times or more, 38 times or more, 39 times or more, 40 times or more, 41 times or more, 42 times or more, 43 times or more, 44 times or more, 45 times or more, 46 times or more, 47 times or more, 48 times or more, 49 times or more, 50 times or more, 55 times or more, 60 times or more, 65 times or more, 70 times or more, 75 times or more, 80 times or more, 85 times or more, 90 times or more, 95 times or more, 100 times or more, 105 times or more, 110 times or more, 120 times or more, 130 times or more, 140 times or more, 150 times or more, 160 times or more, 170 times or more, 180 times or more, 190 times or more, 200 times or more, 220 times or more, 240 times or more, 260 times or more, 280 times or more, 300 times or more, 320 times or more, 340 times or more, 360 times or more, 380 times or more, 400 times or more, 420 times or more, 440 times or more, 460 times or more, 480 times or more, 500 times or more, 550 times or more, 600 times or more, 650 times or more, 700 times or more, 750 times or more, 800 times or more, 850 times or more, 900 times or more, 950 times or more, 1000 times or more, 2000 times or more, 3000 times or more, 4000 times or more, 5000 times or more, 6000 times or more, 7000 times or more, 8000 times or more, 9000 times or more, $10^4$ times or more, $2 \times 10^4$ times or more, $4 \times 10^4$ times or more, $6 \times 10^4$ times or more, $8 \times 10^4$ times or more, $10^5$ times or more, $2 \times 10^5$ times or more, $4 \times 10^5$ times or more, $6 \times 10^5$ times or more, $8 \times 10^5$ times or more, or $10^6$ times or more, the efficacy of the GPC3-targeting drug therapy for cancer in the patient is predicted, expected, or determined or the continuation of the therapy is determined.

In a non-limiting aspect, the method of the present invention comprises monitoring a concentration of free GPC3 in blood, plasma, or serum isolated 30 days or 1 month after the start of GPC3-targeting drug therapy from the patient treated with the therapy, wherein when the concentration of free GPC3 is larger than the baseline concentration, the efficacy of the GPC3-targeting drug therapy is determined. In another non-limiting aspect, the method of the present invention comprises monitoring a concentration of free GPC3 in blood, plasma, or serum isolated 2 months, 3 months, 4 months, 5 months, or 6 months after the start of GPC3-targeting drug therapy from the patient treated with the therapy, wherein when the concentration of free GPC3 is 1 time or more to $10^6$ times or more the baseline concentration, the efficacy of the GPC3-targeting drug therapy is determined.

As described above, when the concentration of free GPC3 is equal to or larger than the baseline concentration, the efficacy of the GPC3-targeting drug therapy is determined. In this procedure, the expression level of GPC3 in a tissue, particularly, a cancer tissue (including a liver cancer tissue), isolated from the patient may be taken into consideration. Specifically, when the concentration of free GPC3 in the patient is equal to or larger than the baseline concentration and the expression level of GPC3 in a tissue, particularly, a cancer tissue (including a liver cancer tissue), isolated from the patient is larger than a particular evaluation score, the efficacy of the GPC3-targeting drug therapy is determined. In another non-limiting aspect, the method of the present invention comprises monitoring a concentration of free GPC3 in blood, plasma, or serum isolated 2 months, 3 months, 4 months, 5 months, or 6 months after the start of GPC3-targeting drug therapy from the patient treated with the therapy, wherein when the concentration of free GPC3 is 1 time or more to $10^6$ times or more the baseline concentration and the expression level of GPC3 in a tissue, particularly, a cancer tissue (including a liver cancer tissue), isolated from the patient is equal to or larger than a predetermined immunohistochemical staining score, the efficacy of the GPC3-targeting drug therapy is determined.

In a non-limiting aspect, examples of the case where the expression level of GPC3 in a tissue, particularly, a cancer tissue (including a liver cancer tissue), isolated from the patient is larger than a predetermined immunohistochemical staining score can include high expression and low or moderate expression (IHC total score: 7 or higher and lower than 7, respectively) in a composite score calculated as a result of staining according to the staining method 1. In a non-limiting aspect, alternative examples of the case where the expression level of GPC3 is larger than a predetermined immunohistochemical staining score can include GPC3-IHC scores of 1+, 2+, and 3+ calculated as a result of staining according to the staining method 2.

In a non-limiting aspect, the method of the present invention comprises monitoring a concentration of free GPC3 in blood, plasma, or serum isolated 30 days or 1 month after the start of GPC3-targeting drug therapy from the patient treated with the therapy, wherein when the concentration of free GPC3 is larger than the baseline concentration, the continuation of the GPC3-targeting drug therapy is determined. In another non-limiting aspect, the method of the present invention comprises monitoring a concentration of free GPC3 in blood, plasma, or serum isolated 2 months, 3 months, 4 months, 5 months, or 6 months after the start of GPC3-targeting drug therapy from the patient treated with the therapy, wherein when the concentration of free GPC3 is 1 time or more to $10^6$ times or more the baseline concentration, the continuation of the GPC3-targeting drug therapy is determined.

As described above, when the concentration of free GPC3 is equal to or larger than the baseline concentration, the continuation of the GPC3-targeting drug therapy is determined. In this procedure, the expression level of GPC3 in a tissue, particularly, a cancer tissue (including a liver cancer tissue), isolated from the patient may be also taken into consideration. Specifically, when the concentration of free GPC3 in the patient is larger than the baseline concentration and the expression level of GPC3 in a tissue, particularly, a cancer tissue (including a liver cancer tissue), isolated from the patient is larger than a particular evaluation score, the continuation of the GPC3-targeting drug therapy is determined. In another non-limiting aspect, the method of the present invention comprises monitoring a concentration of free GPC3 in blood, plasma, or serum isolated 2 months, 3 months, 4 months, 5 months, or 6 months after the start of GPC3-targeting drug therapy from the patient treated with the therapy, wherein when the concentration of free GPC3 is 1 time or more to $10^6$ times or more the baseline concentration and the expression level of GPC3 in a tissue, particularly, a cancer tissue (including a liver cancer tissue), isolated from the patient is larger than a predetermined immunohistochemical staining score, the continuation of the GPC3-targeting drug therapy is determined.

Examples of the case where the expression level of GPC3 in a tissue, particularly, a cancer tissue (including a liver cancer tissue), isolated from the patient is larger than a predetermined immunohistochemical staining score can include high expression and low or moderate expression (IHC total score: 7 or higher and lower than 7, respectively) in a composite score calculated as a result of staining according to the staining method 1. Alternative examples of the case where the expression level of GPC3 is equal to or larger than a predetermined immunohistochemical staining score can include GPC3-IHC scores of 1+, 2+, and 3+ calculated as a result of staining according to the staining method 2.

Drug and preparation

In the present invention, the drug usually refers to an agent for the treatment or prevention of a disease or for examination or diagnosis. In particular, the present invention provides a GPC3-targeting drug for use in a method of treating GPC3-expressing cancer, where in the method the method comprises:(a) performing the method of the present invention; and (b) administering the GPC3-targetingto the patient for which the GPC3-targeting drug has been determined to be effective or that the GPC3-targeting drug has been determined to be continued,
wherein the GPC3-targeting drug comprises an anti-GPC3 antibody. The phrase "having a predetermined value of a concentration of free GPC3 in a biological sample isolated from the cancer patient after the start of GPC3-targeting drug therapy" may be translated into the phrase "the concentration of free GPC3 in the biological sample isolated from the cancer patient after the start of GPC3-targeting drug therapy has been increased as a result of receiving the GPC3-targeting drug therapy".

The drug of the present invention can be formulated using a method generally known to those skilled in the art. For example, the drug of the present invention can be parenterally used in the form of an injection in a sterile solution or suspension with water or any other pharmaceutically acceptable solution. For example, the active ingredient can be appropriately combined with pharmacologically acceptable carriers or media, specifically, sterile water or saline, a plant oil, an emulsifier, a suspending agent, a surfactant, a stabilizer, a flavor, an excipient, a vehicle, an antiseptic, a binder, and the like and mixed therewith in a unit dosage form required for generally accepted pharmaceutical practice to produce preparations. The amount of the active ingredient in these preparations is set to give an appropriate volume within a prescribed range.

Sterile compositions for injection can be formulated according to usual pharmaceutical practice using a vehicle such as injectable distilled water. Examples of injectable aqueous solutions include saline and isotonic solutions containing glucose or other adjuvants (e.g., D-sorbitol, D-mannose, D-mannitol, and sodium chloride). An appropriate solubilizer, for example, an alcohol (ethanol, etc.), a polyalcohol (propylene glycol, polyethylene glycol, etc.), or a nonionic surfactant (Polysorbate 80(TM), HCO-50, etc.) may be used in combination therewith.

Examples of oil solutions include sesame oil and soybean oil. Benzyl benzoate and/or benzyl alcohol may be used as a solubilizer in combination therewith. These injectable solutions may be mixed with a buffer (e.g., a phosphate buffer solution and a sodium acetate buffer solution), a soothing agent (e.g., procaine hydrochloride), a stabilizer (e.g., benzyl alcohol and phenol), and an antioxidant. The prepared injections are usually charged into appropriate ampules.

The drug of the present invention is preferably administered by parenteral administration. For example, the drug is administered in a dosage form of an injection, a transnasal agent, a transpulmonary agent, or a percutaneous agent. The drug can be administered systemically or locally by, for example, intravenous injection, intramuscular injection, intraperitoneal injection, or subcutaneous injection.

The administration method can be appropriately selected according to the age and symptoms of the patient. The single dose of a pharmaceutical preparation containing the drug can be set within the range of, for example, 0.0001 mg to 1000 mg per kg body weight. Alternatively, the dose can be set to, for example, 0.001 to 100000 mg per patient, though the dose of the present invention is not necessarily limited to these numeric values. The dose and the administration method vary depending on the body weight, age, symptoms, etc. of the patient. Those skilled in the art can set an appropriate dose and administration method in consideration of these conditions. As a preferred example of the dose and the administration method of the present invention, the drug of the present invention can be administered to achieve a blood trough level equal to or higher than a predetermined level in the patient. Preferred examples of the blood trough level can include 150 μg/mL or higher, 160 μg/mL or higher, 170 μg/mL or higher, 180 μg/mL or higher, 190 μg/mL or higher, 200 μg/mL or higher, 210 μg/mL or higher, 220 μg/mL or higher, 230 μg/mL or higher, 240 μg/mL or higher, 250 μg/mL or higher, 260 μg/mL or higher, 270 μg/mL or higher, 280 μg/mL or higher, 290 μg/mL or higher, 300 μg/mL or higher, and 400 μg/mL or higher. More preferred examples thereof can include 200 μg/mL or higher.

The preparation of the present invention comprises an instruction stating that the preparation is to be further administered to a cancer patient having a predetermined value of a concentration of free GPC3 in a biological sample isolated from the cancer patient after the start of GPC3-targeting drug therapy. In another non-limiting aspect, the preparation of the present invention comprises an instruction stating that the preparation is to be further administered to a cancer patient in which the concentration of free GPC3 in the biological sample isolated from the cancer patient after the start of GPC3-targeting drug therapy has been increased as a result of receiving the GPC3-targeting drug therapy.

In a non-limiting aspect, the present invention provides the preparation comprising an instruction stating that the patient is selected on the basis of a method comprising monitoring a concentration of free GPC3 in a biological sample isolated from the patient treated with the GPC3-targeting drug therapy, wherein when the concentration of free GPC3 is a predetermined value, the efficacy of the GPC3-targeting drug therapy is determined or the continuation of the therapy is determined.

In a non-limiting aspect, the present invention provides the preparation comprising an instruction stating that the patient is selected on the basis of a method comprising monitoring a concentration of free GPC3 in a biological sample isolated from the patient, wherein when the concentration is a predetermined value, the efficacy of the GPC3-targeting drug therapy for cancer in the patient is predicted, expected, or determined or the continuation of the therapy is determined. The predetermined value may be determined from particular values such as 0.1 ng/mL, 0.2 ng/mL, 0.3 ng/mL, 0.4 ng/mL, 0.5 ng/mL, 0.6 ng/mL, 0.7 ng/mL, 0.8 ng/mL, 0.9 ng/mL, 1.0 ng/mL, 2.0 ng/mL, 3.0 ng/mL, 4.0 ng/mL, 5.0 ng/mL, 6.0 ng/mL, 7.0 ng/mL, 8.0 ng/mL, 9.0 ng/mL, 10.0 ng/mL, 15.0 ng/mL, 20.0 ng/mL, 25.0 ng/mL, 30.0 ng/mL, 35.0 ng/mL, 40.0 ng/mL, 45.0 ng/mL, 50.0 ng/mL, 55.0 ng/mL, 60.0 ng/mL, 65.0 ng/mL, 70.0 ng/mL, 75.0 ng/mL, 80.0 ng/mL, 85.0 ng/mL, 90.0ng/mL, 100.0 ng/mL or may be determined as a numerical range containing particular values arbitrarily selected as the upper and lower limits from the above group of particular values. As an example, such a numerical range can be appropriately selected from numerical ranges of 0.1 ng/mL to 100 ng/mL. Examples of the numeric range include 0.1 to 100 ng/mL, 0.5 to 80 ng/mL, 1.0 to 60 ng/mL, 2.0 to 55 ng/mL, 3.0 to 50 ng/mL, 4.0 to 45 ng/mL, 5.0 to 40 ng/mL, 6.0 to 35 ng/mL, 7.0 to 30 ng/mL, 8.0 to 25 ng/mL, 9.0 to 20 ng/mL, and 10 to 20 ng/mL. The numerical range is, for example, preferably 0.1 to 0.35 ng/mL, more preferably 0.15 to 0.3 ng/mL, though the numerical range of the present invention is not limited to these ranges. The predetermined value of the concentration of free GPC3 can slightly vary depending on many factors, for example, the assay method used, the type of a sample for free GPC3 assay, storage conditions (e.g., temperature and duration) of the sample, and the ethnic identity of the patient. In the method for predicting, expecting, or determining the efficacy or determining the continuation of the therapy, a concentration in a blood, plasma, or serum sample isolated from the patient is measured as the concentration of free GPC3.

The concentration of free GPC3 can be measured in a sample isolated before and/or after the start of the GPC3-targeting drug therapy and may be measured in a plurality of samples collected at predetermined time intervals. When the concentration of free GPC3 in any one of the plurality of samples collected at predetermined time intervals is the predetermined concentration, the efficacy of the GPC3-targeting drug therapy for cancer in the patient is predicted, expected, or determined or the continuation of the therapy is determined. The predetermined time intervals at which the sample is collected after the start of the GPC3-targeting drug therapy are appropriately set. In a non-limiting aspect of the intervals, the samples can be collected at intervals of 1 day, 2 days, 3 days, 4 days, 5 days, 6 days, 7 days (i.e., 1 week), 8 days, 9 days, 10 days, 11 days, 12 days, 13 days, 14 days (i.e., 2 weeks), 15 days, 16 days, 17 days, 18 days, 19 days, 20 days, 21 days (i.e., 3 weeks), 22 days, 23 days, 24 days, 25 days, 26 days, 27 days, 28 days (i.e., 4 weeks), 29 days, 30 days, 1 month, 5 weeks, 6 weeks, 7 weeks, 8 weeks, 9 weeks, 10 weeks, 2 months, 3 months, 4 months, 5 months, or 6 months after the initial administration of the GPC3-targeting drug, or at arbitrary points in time between the start and completion of the therapy, for example, after 1, 2, 3, 4 or more treatment cycles. The dosing intervals, i.e., the treatment cycles, can be appropriately set. One non-limiting example thereof includes 1 day, 2 days, 3 days, 4 days, 5 days, 6 days, 7 days (i.e., 1 week), 8 days, 9 days, 10 days, 11 days, 12 days, 13 days, 14 days (i.e., 2 weeks), 15 days, 16 days, 17 days, 18 days, 19 days, 20 days, 21 days (i.e., 3 weeks), 22 days, 23 days, 24 days, 25 days, 26 days, 27 days, 28 days (i.e., 4 weeks), 29 days, 30 days, 1 month, 5 weeks, 6 weeks, 7 weeks, 8 weeks, 9 weeks, 10 weeks, 2 months, 3 months, 4 months, 5 months, or 6 months.

In one instance, the instruction states that the patient is selected on the basis of a method comprising monitoring a concentration of free GPC3 in blood, plasma, or serum isolated 30 days or 1 month after the start of GPC3-targeting drug therapy from the patient treated with the therapy, wherein when the concentration of free GPC3 ranges from 0.1 ng/mL to 100 ng/mL, the efficacy of the GPC3-targeting drug therapy is determined. In another non-limiting aspect, the instruction states that the patient is selected on the basis of a method comprising monitoring a concentration of free GPC3 in blood, plasma, or serum isolated 2 months, 3 months, 4 months, 5 months, or 6 months after the start of GPC3-targeting drug therapy from the patient treated with the therapy, wherein when the concentration of free GPC3 ranges from 0.1 ng/mL to 100 ng/mL, the efficacy of the GPC3-targeting drug therapy is determined.

In one instance, the instruction states that the patient is selected on the basis of a method comprising monitoring a concentration of free GPC3 in blood, plasma, or serum isolated 30 days or 1 month after the start of GPC3-targeting drug therapy from the patient treated with the therapy, wherein when the concentration of free GPC3 ranges from 0.1 ng/mL to 100 ng/mL, the continuation of the GPC3-targeting drug therapy is determined. In another instance, the instruction states that the patient is selected on the basis of a method comprising monitoring a concentration of free GPC3 in blood, plasma, or serum isolated 2 months, 3 months, 4 months, 5 months, or 6 months after the start of GPC3-targeting drug therapy from the patient treated with the therapy, wherein when the concentration of free GPC3 ranges from 0.1 ng/mL to 100 ng/mL, the continuation of the GPC3-targeting drug therapy is determined.

In another non-limiting aspect of the present invention, the concentration of free GPC3 can be compared with a concentration of free GPC3 ("baseline concentration") measured in a blood, plasma, or serum sample isolated before the start of the GPC3-targeting drug therapy from the patient. In this aspect, the "predetermined value" of the concentration of free GPC3 means that the concentration of free GPC3 in the biological sample isolated from the patient treated with the GPC3-targeting drug therapy is higher than the baseline concentration. Specifically, when the concentration of free GPC3 after the start of the GPC3-targeting drug therapy is larger than that before the start of the therapy in one patient, the efficacy of the GPC3-targeting drug therapy for cancer in the patient is predicted, expected, or determined or the continuation of the therapy is determined. The rate at which the concentration of free GPC3 after the start of the GPC3-targeting drug therapy is equal to or larger than that before the start of the therapy can be appropriately selected by those skilled in the art and is not limited to a particular value. Such a rate can be appropriately selected from a numerical range of 1 time to $10^6$ times. When the rate is, for example, 1 time or more, 1.05 times or more, 1.1 times or more, 1.2 times or more, 1.3 times or more, 1.4 times or more, 1.5 times or more, 1.6 times or more, 1.7 times or more, 1.8 times or more, 1.9 times or more, 2 times or more, 2.1 times or more, 2.2 times or more, 2.3 times or more, 2.4 times or more, 2.5 times or more, 2.6 times or more, 2.7 times or more, 2.8 times or more, 2.9 times or more, 3 times or more, 3.1 times or more, 3.2 times or more, 3.3 times or more, 3.4 times or more, 3.5 times or more, 3.6 times or more, 3.7 times or more, 3.8 times or more, 3.9 times or more, 4 times or more, 4.1 times or more, 4.2 times or more, 4.3 times or more, 4.4 times or more, 4.5 times or more, 4.6 times or more, 4.7 times or more, 4.8 times or more, 4.9 times or more, 5 times or more, 5.1 times or more, 5.2 times or more, 5.3 times or more, 5.4 times or more, 5.5 times or more, 5.6 times or more, 5.7 times or more, 5.8 times or more, 5.9 times or more, 6 times or more, 6.1 times or more, 6.2 times or more, 6.3 times or more, 6.4 times or more, 6.5 times or more, 6.6 times or more, 6.7 times or more, 6.8 times or more, 6.9 times or more, 7 times or more, 7.1 times or more, 7.2 times or more, 7.3 times or more, 7.4 times or more, 7.5 times or more, 7.6 times or more, 7.7 times or more, 7.8 times or more, 7.9 times or more, 8 times or more, 8.1 times or more, 8.2 times or more, 8.3 times or more, 8.4 times or more, 8.5 times or more, 8.6 times or more, 8.7 times or more, 8.8 times or more, 8.9 times or more, 9 times or more, 9.1 times or more, 9.2 times or more, 9.3 times or more, 9.4 times or more, 9.5 times or more, 9.6 times or more, 9.7 times or more, 9.8 times or more, 9.9 times or more, 10 times or more, 11 times or more, 12 times or more, 13 times or more, 14 times or more, 15 times or more, 16 times or more, 17 times or more, 18 times or more, 19 times or more, 20 times or more, 21 times or more, 22 times or more, 23 times or more, 24 times or more, 25 times or more, 26 times or more, 27 times or more, 28 times or more, 29 times or more, 30 times or more, 31 times or more, 32 times or more, 33 times or more, 34 times or more, 35 times or more, 36 times or more, 37 times or more, 38 times or more, 39 times or more, 40 times or more, 41 times or more, 42 times or more, 43 times or more, 44 times or more, 45 times or more, 46 times or more, 47 times or more, 48 times or more, 49 times or more, 50 times or more, 55 times or more, 60 times or more, 65 times or more, 70 times or more, 75 times or more, 80 times or more, 85 times or more, 90 times or more, 95 times or more, 100 times or more, 105 times or more, 110 times or more, 120 times or more, 130 times or more, 140 times or more, 150 times or more, 160 times or more, 170 times or more, 180 times or more, 190 times or more, 200 times or more, 220 times or more, 240 times or more, 260 times or more, 280 times or more, 300 times or more, 320 times or more, 340 times or more, 360 times or more, 380 times or more, 400 times or more, 420 times or more, 440 times or more, 460 times or more, 480 times or more, 500 times or more, 550 times or more, 600 times or more, 650 times or more, 700 times or more, 750 times or more, 800 times or more, 850 times or more, 900 times or more, 950 times or more, 1000 times or more, 2000 times or more, 3000 times or more, 4000 times or more, 5000 times or more, 6000 times or more, 7000 times or more, 8000 times or more, 9000 times or more, $10^4$ times or more, $2 \times 10^4$ times or more, $4 \times 10^4$ times or more, $6 \times 10^4$ times or more, $8 \times 10^4$ times or more, $10^5$ times or more, $2 \times 10^5$ times or more, $4 \times 10^5$ times or more, $6 \times 10^5$ times or more, $8 \times 10^5$ times or more, or $10^6$ times or more, the efficacy of the GPC3-targeting drug therapy for cancer in the patient is predicted, expected, or determined or the continuation of the therapy is determined.

In a non-limiting aspect, the instruction states that the patient is selected on the basis of a method comprising monitoring a concentration of free GPC3 in blood, plasma, or serum isolated 30 days or 1 month after the start of GPC3-targeting drug therapy from the patient treated with the therapy, wherein when the concentration of free GPC3 is larger than the baseline concentration, the efficacy of the GPC3-targeting drug therapy is determined. In another non-limiting aspect, the instruction states that the patient is selected on the basis of a method comprising monitoring a concentration of free GPC3 in blood, plasma, or serum isolated 2 months, 3 months, 4 months, 5 months, or 6 months after the start of GPC3-targeting drug therapy from the patient treated with the therapy, wherein when the concentration of free GPC3 is 1 time or more to $10^6$ times or more the baseline concentration, the efficacy of the GPC3-targeting drug therapy is determined.

As described above, the instruction states that when the concentration of free GPC3 is larger than the baseline concentration, the efficacy of the GPC3-targeting drug therapy is determined. In this case, the instruction may state that the expression level of GPC3 in a tissue, particularly, a cancer tissue (including a liver cancer tissue), isolated from the patient is also taken into consideration. Specifically, the instruction may state that when the concentration of free GPC3 in the patient is larger than the baseline concentration and the expression level of GPC3 in a tissue, particularly, a cancer tissue (including a liver cancer tissue), isolated from the patient is equal to or larger than a particular evaluation score, the efficacy of the GPC3-targeting drug therapy is determined. In another non-limiting aspect, the instruction can state that the patient is selected on the basis of a method comprising monitoring a concentration of free GPC3 in blood, plasma, or serum isolated 2 months, 3 months, 4 months, 5 months, or 6 months after the start of GPC3-targeting drug therapy from the patient treated with the therapy, wherein when the concentration of free GPC3 is 1 time or more to $10^6$ times or more the baseline concentration and the expression level of GPC3 in a tissue, particularly, a cancer tissue (including a liver cancer tissue), isolated from the patient is larger than a predetermined immunohistochemical staining score, the efficacy of the GPC3-targeting drug therapy is determined.

In a non-limiting aspect, examples of the case where the expression level of GPC3 in a tissue, particularly, a cancer tissue (including a liver cancer tissue), isolated from the patient is larger than a predetermined immunohistochemical staining score can include high expression and low or moderate expression (IHC total score: 7 or higher and lower than 7, respectively) in a composite score calculated as a result of staining according to the staining method 1. In a non-limiting aspect, alternative examples of the case where the expression level of GPC3 is larger than a predetermined immunohistochemical staining score can include GPC3-IHC scores of 1+, 2+, and 3+ calculated as a result of staining according to the staining method 2.

In a non-limiting aspect, the instruction states that the patient is selected on the basis of a method comprising monitoring a concentration of free GPC3 in blood, plasma, or serum isolated 30 days or 1 month after the start of GPC3-targeting drug therapy from the patient treated with the therapy, wherein when the concentration of free GPC3 is larger than the baseline concentration, the continuation of the GPC3-targeting drug therapy is determined. In another non-limiting aspect, the instruction states that the patient is selected on the basis of a method comprising monitoring a concentration of free GPC3 in blood, plasma, or serum isolated 2 months, 3 months, 4 months, 5 months, or 6 months after the start of GPC3-targeting drug therapy from the patient treated with the therapy, wherein when the concentration of free GPC3 is 1 time or more to $10^6$ times or more the baseline concentration, the continuation of the GPC3-targeting drug therapy is determined.

As described above, the instruction states that when the concentration of free GPC3 is larger than the baseline concentration, the continuation of the GPC3-targeting drug therapy is determined. In this case, the instruction may state that the expression level of GPC3 in a tissue, particularly, a cancer tissue (including a liver cancer tissue), isolated from the patient is also taken into consideration. Specifically, the instruction may state that when the concentration of free GPC3 in the patient is larger than the baseline concentration and the expression level of GPC3 in a tissue, particularly, a cancer tissue (including a liver cancer tissue), isolated from the patient is equal to or larger than a particular evaluation score, the continuation of the GPC3-targeting drug therapy is determined. In another non-limiting aspect, the instruction can state that the patient is selected on the basis of a method comprising monitoring a concentration of free GPC3 in blood, plasma, or serum isolated 2 months, 3 months, 4 months, 5 months, or 6 months after the start of GPC3-targeting drug therapy from the patient treated with the therapy, wherein when the concentration of free GPC3 is 1 time or more to $10^6$ times or more the baseline concentration and the expression level of GPC3 in a tissue, particularly, a cancer tissue (including a liver cancer tissue), isolated from the patient is equal to or larger than a predetermined immunohistochemical staining score, the continuation of the GPC3-targeting drug therapy is determined.

Examples of the case where the expression level of GPC3 in a tissue, particularly, a cancer tissue (including a liver cancer tissue), isolated from the patient is larger than a predetermined immunohistochemical staining score can include high expression and low or moderate expression (IHC total score: 7 or higher and lower than 7, respectively) in a composite score calculated as a result of staining according to the staining method 1. Alternative examples of the case where the expression level of GPC3 is equal to or larger than a predetermined immunohistochemical staining score can include GPC3-IHC scores of 1+, 2+, and 3+ calculated as a result of staining according to the staining method 2.

Hereinafter, the present invention will be described specifically with reference to Examples. However, the present invention is not limited by these Examples.

[Example 1]

GC33 is a recombinant humanized IgG1 monoclonal antibody capable of binding to human GPC3 with high affinity (WO2006/006693). In order to confirm the dose limiting toxicity (DLT) of GC33 in patients with advanced and/or recurrent hepatocellular cancer (HCC), a phase-I multicenter clinical trial was carried out (GC-001US test). In this test aimed at confirming safety and/or tolerability in the patients with advanced and/or recurrent HCC, the pharmacokinetic profiles of GC33, and its antitumor effects, and searching for biomarkers, GC33 (2.5 mg/kg to 20 mg/kg) was administered by injection through an intravenous drip to each HCC patient once a week.

The HCC patients subjected to the administration had histologically or cytologically confirmed advanced or metastatic HCC unsuitable for surgical operation and/or curative treatment. Eligible patients were at least 18 years old and exhibited Eastern Cooperative Oncology Group Performance Status of 0 or 1 and Child-Pugh class A or B. The patients also had at least one lesion that was evaluable according to the response evaluation criteria in solid tumors (RECIST). The provision of HCC tumor tissues (needle biopsy preparations) for use in GPC3 immunohistochemical staining (GPC3-IHC), appropriate hematopoietic functions (absolute neutrophil count $\geq$ 1500/$\mu$l, platelet $\geq$ 50000/$\mu$l), hepatic functions (total bilirubin $\leq$ 3 times the normal level, aspartate aminotransferase and alanine aminotransferase $\leq$ 5 times the normal level, PT-INR $\leq$ 2.0), and renal functions (serum creatinine $\leq$ twice the normal level) were evaluated as other criteria. The registered subjects excluded pregnant, nursing, or pregnancy test-positive (women who underwent menstruation within 12 months from the registration date were subjected to the pregnancy test) patients, patients who did not plan to use appropriate fertility control, HIV antibody-positive patients, patients having active infection requiring treatment except for HBV or HCV, patients having other active malignant tumors with a disease-free interval shorter than 5 years, patients having a past history of transplantation, patients confirmed to have brain metastasis with symptoms, patients having central nervous system disorder or other mental disorders that interfered with consent or understanding of the protocol, patients who presented central nervous system symptoms attributed to hepatic encephalopathy, and patients who exhibited known hypersensitivity to other antibody drugs or pharmaceutical agents produced using CHO cells. Alternatively, patients who received treatment including major surgical operation, radiation therapy, and other chemotherapies within 4 weeks before the administration of the GPC3-targeting drug, patients who received treatment with sorafenib within 2 weeks before the administration, or patients who received needle biopsy within 1 week before the administration were excluded from the subjects registered in the GPC3-targeting drug therapy, but were subjected to the GPC3-targeting treatment after a predetermined wash-out period. The protocol was carried out according to the guideline of the Good Clinical Practice (GCP) and approved by each participating ethical committee on clinical trials. All patients signed their names on written informed consent before registration. The patients received the continuous administration of GC33 (each cycle involved four doses of GC33) unless the disease progressed or unacceptable toxicity appeared. Tumor was evaluated on the basis of a baseline and repetitively evaluated every two cycles until the disease progressed. The state of the disease was evaluated by principal investigators.

The expression of GPC3 proteins in HCC tumor tissues was evaluated by GPC3 immunohistochemical staining (GPC3-IHC). The median measurement of GPC3-IHC was carried out by Charles River Laboratory (USA). Unstained slides of HCC tumor tissues prepared from tumor blocks formalinfixed and paraffin-embedded after excision by needle biopsy in each hospital were subjected to immunohistochemical staining. The histochemical staining approach such as epitope retrieval for the measurement by Charles River Laboratory (USA) was performed according to a method described in WO2009/116659. The antibodies used were a mouse GC33 antibody and a mouse IgG2a antibody as a negative control antibody (WO2006/006693).

As for GPC3-IHC (staining method 1) carried out by Charles River Laboratory, the respective scores of positive cell rate (PR), staining intensity of cytoplasm (SI-cp) or staining intensity of cell membrane (SI-cm), and staining pattern of cell membrane (Sp-cm) were calculated according to the criteria shown in Table 4 and added on the basis of calculation expressions 1 and 2 to evaluate each stained preparation. The evaluation of each stained preparation was finalized at the Peer review meeting involving three pathologists.

[Table 4]

| Criterion | Evaluation | Score |
|---|---|---|
| Positive cell rate (PR) | 0 | 0 |
| | 1% or more and less than 20% | 1 |
| | 20% or more and less than 50% | 2 |
| | 50% or more | 3 |
| Staining intensity (SI) | Slightly positive | 0 |
| - Cytoplasm (SI-cp) | Weakly positive | 1 |
| - Cell membrane (SI-cm) | Moderately positive and/or weakly positive with strong positivity | 2 |
| | Moderately positive | 3 |
| | Strongly positive | 4 |
| Staining pattern of cell membrane (SP-cm) | Negative | 0 |
| | When only a portion of the cell membranes of cells was stained | 1 |
| | When a portion of the cell membranes of most of these cells was stained and the cell membranes of some of the cells were circumferentially stained | 2 |
| | When the cell membranes of most of these cells were circumferentially stained | 3 |
| (Sp-cm scores were calculated by the evaluation of cell staining in the visual field under microscope using an objective lens with a magnification of 4 or 10) | | |

[Expression 3]

$$\text{IHC total} = \text{PR} + \text{SI-Cp} + \text{SI-Cm} + \text{Sp-Cm}$$

One out of 20 cases that were registered in this test and received the administration failed to produce a preparation, and 3 of the cases did not contain tumor cells sufficient for evaluation. Finally, 16 cases were able to be evaluated. These cases were divided into two groups on the basis of an IHC total score around 7, which was half the maximum value (14) in staining based on epitope retrieval using autoclaving. The evaluation results of each case are shown in Table 5 and FIG. 1.

[Table 5]

| Evaluation by GPC-IHC (IHC total score) | The number of patients (percentage to the total number 20) |
|---|---|
| High expression (7 or higher) | 9 (45%) |
| Low or moderate expression (Lower than 7) | 7 (35%) |
| Unevaluable | 4 (20%) |

[Example 2]

Antitumor effects were evaluated by the administration of GC33 in GPC3-targeting treatment. The durations of GC33 administration to 20 cases as described above are shown in FIG. 2. As a result of evaluating the state of the disease, 5-month or longer stable disease (SD) was confirmed in 4 cases.

The expression of GPC3 in tumor tissues was examined for its relation to the antitumor effects of GC33. As a result of showing the relation of the IHC total scores of 16 cases evaluable by GPC3-IHC to the duration of administration, all cases confirmed to have SD in a 5-month or longer period were included in a high-value group when the 16 cases were divided into two groups (with an IHC total score of 7 or higher and with an IHC total score lower than 7). In addition, the obtained results showed that the percentage of the long-period SD cases in the high-value group was also high (Table 6).

[Table 6]

| IHC total score | High-value group | | Low-value group | |
|---|---|---|---|---|
| 6 or higher | 36% | (4/11) | 0% | (0/5) |
| 7 or higher | 44% | (4/9) | 0% | (0/7) |
| 8 or higher | 50% | (3/6) | 10% | (1/10) |
| 9 or higher | 50% | (2/4) | 17% | (2/12) |
| 10 or higher | 50% | (1/2) | 21% | (3/14) |

Subsequently, progression-free survival duration or progression-free survival (PFS) was compared between the group with an IHC total score of 7 or higher and the group with an IHC total score lower than 7. The results showed that the group with an IHC total score of 7 or higher had significantly long PFS (FIG. 3).

Some of the tumor samples thus evaluated were used in the additional evaluation of GPC3-IHC. The median measurement of staining of preparations obtained from 14 cases was carried out by Ventana Medical Systems, Inc. (USA) according to an instruction attached to antiglypican 3 Mouse GC33 Monoclonal Primary Antibody (Ventana Medical Systems, Inc.) using an automatic staining apparatus BenchMark (manufactured by Ventana Medical Systems, Inc.). In GPC3-IHC (staining method 2) carried out by Ventana Medical Systems, Inc., the preparations were stained according to the attached instruction and then scored on 4 scales of 0 to 3+ in terms of the degree, intensity, etc. of staining in tumor cells. As a result, distribution shown in Table 7 was obtained.

[Table 7]

| GPC3-IHC score | The number of patients (percentage to 14 evaluable cases) |
|---|---|
| 3+ | 3 (21.4%) |
| 2+ | 1 (7.1%) |
| 1+ | 7 (50%) |
| 0+ | 3 (21.4%) |

In the staining method 2, cases exhibiting long-period SD were included at a high percentage and with no omission in a 2+/3+ group when the cases were divided into two groups (with a score of 0 and 1+ and with a score of 2+ and 3+) (Table 8).

[Table 8]

| GPC3-IHC score | High-value group | | Low-value group | |
|---|---|---|---|---|
| 1+/2+/3+ | 18% | (2/11) | 0% | (0/3) |
| 2+/3+ | 50% | (2/4) | 0% | (0/10) |
| 3+ | 33% | (1/3) | 9% | (1/11) |

[Example 3]

The concentration of free GPC3 was measured in the serum of patients who received the administration of GC33 in GPC3-targeting treatment. Mouse anti-GPC3 monoclonal antibodies M3C11 and L9G11 (WO2004/022739) were each diluted into 7.5 μg/mL with an immobilizing buffer (0.05 mol/L sodium bicarbonate, pH 9.6) and then dispensed to a plate at a concentration of 100 μL/well. Then, the plate was left standing at room temperature for 1 hour. Each well was washed three times with a washing buffer (0.05 mol/L tris-buffered saline, pH 8.0, 0.05% Tween-20). Then, a blocking buffer (25 mmol/L tris-HCl buffer, pH 8.1, 0.5 mmol/L magnesium chloride, 72 mmol/L sodium chloride, 0.05% ProClin 150, 5 mg/mL bovine serum albumin, 0.025% Tween-20, 1% Block Ace) was dispensed thereto at a concentration of 200 μL/well. The plate was left standing at room temperature for 2 hours to prepare an antibody-immobilized plate. If the plate was not immediately used, the plate was stored at 4°C and then used in the measurement.

The serum of each patient collected in the clinical trial was diluted 4-fold with a diluting buffer (25 mmol/L tris-HCl buffer, pH 8.1, 0.5 mmol/L magnesium chloride, 72 mmol/L sodium chloride, 0.05% ProClin 150, 5 mg/mL bovine serum albumin, 0.025% Tween-20, 0.4% Block Ace) and added to the plate at a concentration of 100 μL/well. The plate was left standing overnight at 4°C. The GPC3 standard used was recombinant GPC3 with serine residues at positions 495 and 509 substituted by alanine residues so as not to permit the binding of heparan sulfate sugar chains (Hippo et al., Cancer Res. (2004) 64, 2418-2423).

Subsequently, each well of the plate was washed three times with a washing buffer, and a biotin-labeled anti-glypican-3 polyclonal antibody (manufactured by R&D systems, Inc.) diluted into 0.3 μg/mL with a diluting buffer was added thereto at a concentration of 100 μL/well. The plate was further left standing at 25°C for 1 hour, and each well was washed three times with a washing buffer. Then, HRP-labeled streptavidin (Streptavidin-Poly HRP80; manufactured by Stereospecific Detection Technologies (SDT)) diluted with a diluting buffer according to the instruction was added thereto at a concentration of 100 μL/well. The plate was left standing at 25°C for 1 hour. Then, each well of the plate was washed three times with a washing buffer. Then, color was developed using TMB Microwell Peroxidase Substrate System (manufactured by Kirkegaard & Perry Laboratories Inc.) according to the instruction attached to the kit. The absorbance of the reaction solution in each well was measured at 450 nm and 650 nm. A calibration curve prepared on the basis of the standard sample containing the recombinant GPC3 was used to calculate the GPC3 antigen level in the serum of each patient from the obtained absorbance of each well.

[Example 4]

Change in the serum concentration of detected free GPC3 calculated in Example 3 is shown in FIG. 4 for two groups, i.e., the high-value group and the low-value group, of tumor tissue GPC3-IHC scores determined in Example 2. A large number of cases with a measurable level of free GPC3 was included in the group evaluated as having high expression of GPC3 on the basis of the GPC3-IHC score (FIG. 4A). A rise in the concentration of free GPC3 or stabilization thereof was observed in cases exhibiting long SD. By contrast, a small number of cases with a measurable level of free GPC3 was included in the group evaluated as having low expression of GPC3 or being negative on the basis of the GPC3-IHC score (FIG. 4B).

Progression-free survival duration or progression-free survival (PFS) was compared between a group having a measurable level of GPC3 in serum collected during screening or before initial administration (GPC3-positive group) and a group with a GPC3 level below the detection limit. The PFS of the serum GPC3-positive group before the practice of GPC3-targeting treatment was confirmed to be longer than that of the negative group (FIG. 5A). A logrank test was further conducted if the serum GPC3-positive group involved serum in which serum GPC3 was measured after the start of administration of the GPC3-targeting drug. The test results showed that the PFS of this group was significantly longer than that of the negative group (cases with a GPC3 level below the measurement limit, regardless of before or after administration of the GPC3-targeting drug) and the positive group (FIG. 5B).

[Example 5]

As shown in Tables 9 and 10, serum GPC3-positive high-value groups of GPC3-IHC scores evaluated using the staining method 1 and the staining method 2 were shown to have a higher percentage of long SD than that of the high-value group of GPC3-IHC scores (Tables 6 and 8 to 10) .

[Table 9]

| GPC3-IHC (staining method 1) | Serum GPC3-positive with high IHC value (7 or higher) | | Others | |
|---|---|---|---|---|
| Serum GPC3 before administration GPC3 | 60% | (3/5) | 9% | (1/11) |
| Serum GPC3 after administration GPC3 | 80% | (4/5) | 0% | (0/11) |

[Table 10]

| GPC3-IHC (staining method 2) | Serum GPC3-positive with IHC (2-3+) | | Others | |
|---|---|---|---|---|
| Serum GPC3 before administration GPC3 | 100% | (2/2) | 0% | (0/12) |
| Serum GPC3 after administration GPC3 | 67% | (2/3) | 0% | (0/11) |

[Example 6]

In the clinical trial, additional 7 cases (one of which was assessed as having an IHC total score of 6 as a result of final evaluation) were further registered as cases having an IHC total score of 7 or higher in GPC3-IHC on the basis of results of the staining method 1 and Child-Pugh score A. Their serum GPC3 levels were measured according to the method of Example 3. A total of 27 cases that received the administration of the GPC3-targeting drug were evaluated for their PFS in the same way as in Example 4. The relation of the serum GPC3 levels to PFS in these cases was studied using the logrank test. The test results showed that the PFS of a group having a measurable level of serum GPC3 before the administration (FIG. 6A) and the PFS of a group having a measurable level of serum GPC3 either before or after the administration (FIG. 6B) were both significantly longer than that of a group with a serum GPC3 level below the measurement limit.

[Example 7]

In order to confirm the efficacy and safety of GC33 in patients with advanced and/or recurrent hepatocellular cancer (HCC), a phase-II multicenter randomized doubleblind placebo-controlled clinical trial which involved administering 1600 mg of GC33 every other week was carried out (NP27884 study), targeting adult patients with unresectable advanced or metastatic hepatocellular cancer having a past history of treatment. These patients were randomized to a GC33 group (the fixed dose of 1600 mg was administered every other week after administration of two doses at a 1-week interval; n = 121 cases) or a placebo group (n = 60 cases) at a ratio of 2:1 and stratified to 3 cohorts on the basis of GPC3 expression levels (0, 1+, and 2+/3+) by IHC staining using GPC3-IHC kit (manufactured by Ventana Medical Systems, Inc.). Primary analysis was carried out at the time of occurrence of progression-free survival (PFS) events in 128 cases planned in the protocol.

The HCC patients subjected to the administration had histologically confirmed advanced or metastatic HCC (except for fibrolamellar type) unsuitable for curative therapy (surgical resection, liver transplantation, etc.) and/or local therapy or exacerbated after treatment and had a past history of treatment based on systemic therapy with at least one agent. Eligible patients were at least 18 years old with the capability of providing a tumor sample for GPC3 assay and exhibited Eastern Cooperative Oncology Group Performance Status of 0 or 1 and Child-Pugh class A. The patients also had at least one lesion that was evaluable according to the response evaluation criteria in solid tumors (RECIST). Appropriate hematopoietic functions (absolute neutrophil count $\geq$ 1500/$\mu$l, platelet $\geq$ 50000/$\mu$l, hemoglobin $\geq$ 8.0 g/dl), hepatic functions (total bilirubin $\leq$ 2 mg/dl, aspartate aminotransferase and alanine aminotransferase $\leq$ 5 times the upper limit of the normal level), and renal functions (serum creatinine $\leq$ twice the upper limit of the normal level) were evaluated as other criteria. Registrable female subjects were premenopausal female patients confirmed to be negative for a serum pregnancy test conducted within 10 days before the start of administration of the study drug, women without the possibility of pregnancy as a result of surgical contraception or after a lapse of 1 year or longer after menopause, and female patients other than the postmenopausal women (12-month or longer absence of menstruation) or the surgically contracepted women (resection of the ovary and/or the uterus), who consented to use two types of appropriate fertility control methods during clinical trial treatment and for at least 3 months or longer after the completion of administration of the study drug. Registrable male subjects were patients who consented to use fertility control based on the barrier method during clinical trial treatment and for at least 40 days after the completion of administration of the study drug. On the other hand, the registered subjects excluded patients who received major surgical operation within 2 weeks before the administration of the GPC3-targeting drug or did not get over severe disorder, patients confirmed to have brain or leptomeningeal metastasis, patients having a past history of malignant tumor within the last 5 years, patients having active infection requiring treatment except for hepatitis B or hepatitis C, patients having a past history of NCI-CTCAE v4.0 Grade 3 or higher hemorrhage within 4 weeks before the start of administration of the study drug, patients having a past history of organ transplantation including liver transplantation, patients who were scheduled to receive or were receiving the administration of an anticancer agent other than the agent to be administered in this test, patients who received the administration of an anticancer agent within 2 weeks before trial registration, patients who did not completely get over adverse reactions associated with the preceding locoregional or systemic therapy of hepatocellular cancer, patients under interferon therapy, patients who had baseline QTc exceeding 470 ms or exhibited baseline resting bradycardia (less than 45 beads/min.), patients who received the administration of an anticoagulant or a thrombolytic agent for therapeutic purposes within 2 weeks before the start of administration of the study drug (except for the administration of the agent at a low dose for the purpose of removing clogs in a catheter or for preventive purposes), pregnant or nursing patients, HIV-positive patients or patients having an AIDS-related disease, patients having a past history of hypersensitivity for similar agents (monoclonal antibodies, protein-containing preparations, and Chinese hamster ovary-derived preparations), and patients having a serious comorbidity judged by a principal investigator or a sub-investigator as being possibly worsened due to the study drug.

The protocol was carried out according to the guideline of the Good Clinical Practice (GCP) and approved by each participating ethical committee on clinical trials. All patients signed their names on written informed consent before registration. The patients received the continuous administration of GC33 unless the disease progressed or unacceptable toxicity appeared. Tumor was evaluated on the basis of a baseline and evaluated after 4 cycles, 7 cycles, and 10 cycles from the start of administration and then repetitively every four cycles until the disease progressed. Each cycle involved two weeks. The state of the disease was evaluated by principal investigators.

The expression of GPC3 proteins in HCC tumor tissues was evaluated by GPC3 immunohistochemical staining (GPC3-IHC). The central measurement of GPC3-IHC was carried out by Ventana Medical Systems, Inc. (USA). Unstained slides of HCC tumor tissues prepared from tumor blocks formalin-fixed and paraffin-embedded after excision by needle biopsy in each hospital were subjected to immunohistochemical staining. The antibody used was a mouse GC33 antibody.

[Example 8]

In the cases who received GC33 or a placebo in GPC3-targeting treatment, the serum concentration of free GPC3 was measured before the initial administration using a combination of two types of different antibodies capable of binding to free GPC3 (a combination of a GT30 antibody and a GT607 antibody or a combination of GT114 and GT165). GT30, GT607, GT114, and GT165 were prepared according to a method described in WO2004/022739 and selected as antibodies capable of binding to free GPC3. The H and L chains of GT30 are shown in SEQ ID NOs: 83 and 84, respectively. The H and L chains of GT607 are shown in SEQ ID NOs: 85 and 86, respectively. The H and L chains of GT114 are shown in SEQ ID NOs: 87 and 88, respectively. The H and L chains of GT165 are shown in SEQ ID NOs: 89 and 90, respectively.

An antibody-bound particle solution containing GT30 or GT114 bound to magnetic particle beads (manufactured by JSR Corp.) was added at a concentration of 25 μL/well to a 96-well microplate. Subsequently, a standard sample solution for a calibration curve (the GPC3 standard described in Example 3 was used) or an appropriately diluted serum sample was added thereto at a concentration of 25 μL/well, and further, alkaline phosphatase-labeled GT607 or GT165 was added thereto at a concentration of 25 μL/well. After shaking at 25°C for 20 minutes, each well was washed 5 times with a washing solution, with the magnetic beads collected using Dyna-Mag-96 Side Skirted (manufactured by VERITAS Corp.). A luminescent substrate solution preheated to 37°C was added thereto at a concentration of 50 μL/well. The plate was shaken at room temperature for 1 minute and then left standing for 4 minutes to emit light. Chemiluminescence intensity was measured using a luminometer (manufactured by VERITAS Corp.).

A calibration curve (standard curve) prepared on the basis of the standard sample containing the recombinant GPC3 was used to calculate the GPC3 antigen level in the serum of each patient from the obtained chemiluminescence intensity of each well.

[Example 9]

Once the PFS events of 128 cases were obtained from among 125 GC33-administered cases and 60 placeboadministered cases as described above, the effects of administration of GC33 in GPC3-targeting treatment were evaluated on the basis of PFS. In addition, overall survival (OS) was evaluated as a secondary endpoint when reaching 78 events.

The GC33-administered group was further divided into two groups (a group exposed to GC33 at a lower level than a cutoff value: low-GC33-exposed group, and a group exposed to GC33 at a higher level than a cutoff value: high-GC33-exposed group) using, as the cutoff value, the median value 230 μg/ml of projected blood trough levels of GC33 before administration of day 1 in the 3rd cycle (on the 4th week from the start of initial administration) based on population PK models obtained using the serum GC33 concentration values of this phase-II clinical trial. The progression-free survival duration or progression-free survival (PFS) or the overall survival duration or overall survival (OS) was compared as an index for clinical effects between these groups or between these groups and the placebo group by the Kaplan-Meier method.

[Example 10]

The serum concentrations of detected free GPC3 calculated in Example 8 were divided into two groups, i.e., a low-value group and a high-value group, on the basis of the median value of the concentrations measured in a system having GT30 and GT607 in combination. The PFS or OS curves of low-GC33-exposed, high-GC33-exposed, and placebo groups, as shown in Example 9, are shown in FIGs. 7A to 7D. Likewise, the serum concentrations of free GPC3 were divided into two groups on the basis of the median value of the concentrations measured in a system having GT114 and GT165 in combination. The PFS or OS curves of these groups are shown in FIGs. 8A to 8D.

In all cases, the group with a low concentration of free GPC3 exhibited the low effect of prolonging the PFS and OS durations, whereas the high-GC33-exposed group with a high concentration of free GPC3 in serum exhibited significantly low hazard ratios of the PFS and OS durations to the low-GC33-exposed group or the placebo group.

As a result of evaluating the cutoff value of free GPC3 that achieved the smallest significant difference, the cutoff value was 175 pg/mL for the GT30-GT607 system and 259.7 pg/mL for the GT114-GT165 system. The PFS and OS curves of a patient group that exhibited a free GPC3 level higher than the cutoff value in each system are shown in FIGs. 7E and 7F and FIGs. 8E and 8F, respectively. In this case as well, a significantly low hazard ratio, i.e., the prolongation of each survival duration, was exhibited in the high-GC33-exposed group.

[Deleted]

[Industrial Applicability]

The present invention contributes to improvement in the efficacy of GPC3-targeting drug therapy and improvement in QOL of a patient to be treated, and is useful in the treatment of cancer including liver cancer.

[Free Text for Sequence Listing]

SEQ ID NO: 44: Modified antibody fragment
SEQ ID NO: 45: Modified antibody fragment
SEQ ID NO: 46: Modified antibody fragment
SEQ ID NO: 47: Modified antibody fragment
SEQ ID NO: 48: Modified antibody fragment
SEQ ID NO: 49: Modified antibody fragment
SEQ ID NO: 50: Modified antibody fragment
SEQ ID NO: 51: Modified antibody fragment
SEQ ID NO: 52: Modified antibody fragment
SEQ ID NO: 53: Modified antibody fragment
SEQ ID NO: 54: Modified antibody fragment
SEQ ID NO: 55: Modified antibody fragment
SEQ ID NO: 56: Modified antibody fragment
SEQ ID NO: 57: Modified antibody fragment
SEQ ID NO: 58: Modified antibody fragment
SEQ ID NO: 59: Modified antibody fragment

SEQ ID NO: 60: Modified antibody fragment
SEQ ID NO: 61: Modified antibody fragment
SEQ ID NO: 62: Modified antibody fragment
SEQ ID NO: 63: Modified antibody fragment
SEQ ID NO: 64: Modified antibody fragment
SEQ ID NO: 65: Modified antibody fragment
SEQ ID NO: 66: Modified antibody fragment
SEQ ID NO: 67: Modified antibody fragment
SEQ ID NO: 68: Modified antibody fragment
SEQ ID NO: 69: Modified antibody fragment
SEQ ID NO: 70: Modified antibody fragment
SEQ ID NO: 71: Modified antibody fragment
SEQ ID NO: 72: Modified antibody fragment
SEQ ID NO: 73: Modified antibody fragment

## Claims

1. A method for determining the efficacy of GPC3-targeting drug therapy for GPC3-expressing cancer in a patient or determining the continuation of GPC3-targeting drug therapy for a patient, comprising monitoring a concentration of free GPC3 in a biological sample isolated from the patient before the start of GPC3-targeting drug therapy and/or the patient treated with the GPC3-targeting drug therapy, wherein the GPC3-targeting drug comprises an anti-GPC3 antibody, wherein the biological sample is blood, plasma, or serum, and wherein when the concentration of free GPC3 is above a predetermined value, the GPC3-targeting drug therapy is determined to be effective or the GPC3-targeting drug therapy is determined to be continued.

2. The method according to claim 1, wherein:

(a) the predetermined value of free GPC3 is within the range from 0.1 ng/mL to 100 ng/mL;
(b) the concentration of free GPC3 is measured using an immunological method;
(c) the concentration of free GPC3 is larger than that in a biological sample isolated before the start of the GPC3-targeting drug therapy from the patient;
(d) the patient shows high expression of GPC3 in an immunohistochemical staining score;
(e) the cancer is cancer having high expression of GPC3 liver cancer; and/or
(f) the GPC3-targeting drug is administered to achieve a blood trough level of 200 μg/ml or higher in the cancer patient.

3. The method according to claim 1 or 2, wherein:

(A) the anti-GPC3 antibody has antibody-dependent cellular cytotoxicity (ADCC) activity and/or complement-dependent cytotoxicity (CDC) activity;
(B) the anti-GPC3 antibody is an anti-GPC3 chimeric antibody or a humanized anti-GPC3 antibody comprising any of the following (1) to (5) :

(1) heavy chain CDR1, heavy chain CDR2, and heavy chain CDR3 represented by SEQ ID NOs: 4, 5, and 6, respectively, and light chain CDR1, light chain CDR2, and light chain CDR3 represented by SEQ ID NOs: 7, 8, and 9, respectively;
(2) heavy chain CDR1, heavy chain CDR2, and heavy chain CDR3 represented by SEQ ID NOs: 12, 13, and 14, respectively, and light chain CDR1, light chain CDR2, and light chain CDR3 represented by SEQ ID NOs: 15, 16, and 17, respectively;
(3) heavy chain CDR1, heavy chain CDR2, and heavy chainCDR3 represented by SEQ ID NOs: 20, 21, and 22, respectively, and light chain CDR1, light chain CDR2, and light chain CDR3 represented by SEQ ID NOs: 23, 24, and 25, respectively;
(4) heavy chain CDR1, heavy chain CDR2, and heavy chain CDR3 represented by SEQ ID NOs: 28, 29, and 30, respectively, and light chain CDR1, light chain CDR2, and light chain CDR3 represented by SEQ ID NOs: 31, 32, and 33, respectively; and
(5) heavy chain CDR1, heavy chain CDR2, and heavy chain CDR3 represented by SEQ ID NOs: 36, 37, and 38, respectively, and light chain CDR1, light chain CDR2, and light chain CDR3 represented by SEQ ID NOs: 39, 40, and 41, respectively; and/or

(C) the anti-GPC3 antibody comprises any of the following (1) to (6) :

(1) a heavy chain variable region selected from the group of heavy chain variable regions represented by SEQ ID NOs: 44, 45, 46, 47, 48, 49, and 50 and a light chain variable region represented by SEQ ID NO: 51;
(2) a heavy chain variable region selected from the group of heavy chain variable regions represented by SEQ ID NOs: 44, 45, 46, 47, 48, 49, and 50 and a light chain variable region selected from the group of light chain variable regions represented by SEQ ID NOs: 52, 53, 54, 55, 56, 57, 58, 59, 60, 61, 62, 63, 64, 65, and 66;
(3) a heavy chain variable region represented by SEQ ID NO:67 and a light chain variable region represented by SEQ ID NO: 68;
(4) a heavy chain variable region represented by SEQ ID NO: 69 and a light chain variable region represented by SEQ ID NO: 70;
(5) a heavy chain variable region represented by SEQ ID NO: 71 and a light chain variable region represented by SEQ ID NO: 72; and

(6) a heavy chain variable region represented by SEQ ID NO: 71 and a light chain variable region represented by SEQ ID NO: 73.

**4.** The method according to any one of claims 1 to 3, wherein the anti-GPC3 antibody has been conjugated with a cytotoxic substance.

**5.** The method according to claim 1 or 2, the cancer is cancer selected from breast cancer, uterine cervix cancer, colon cancer, uterine body cancer, head and neck cancer, lung cancer, malignant glioma, malignant lymphoma, ovary cancer, pancreatic cancer, prostatic cancer, renal cancer, skin cancer, gastric cancer, testicle cancer, thyroid cancer, urothelial cancer.

**6.** A GPC3-targeting drug for use in a method of treating GPC3-expressing cancer, where in the method the method comprises:

(a) performing the method of any one of claims 1 to 6; and
(b) administering the GPC3-targeting drug to the patient for which the GPC3-targeting drug has been determined to be effective or that the GPC3-targeting drug has been determined to be continued,

wherein the GPC3-targeting drug comprises an anti-GPC3 antibody.

**Patentansprüche**

**1.** Verfahren zum Bestimmen der Wirksamkeit einer auf GPC3-zielgerichteten Arzneimitteltherapie für GPC3-exprimierenden Krebs in einem Patienten oder zum Bestimmen der Fortsetzung einer GPC3-zielgerichteten Arzneimitteltherapie für einen Patienten, umfassend das Überwachen einer Konzentration von freiem GPC3 in einer biologischen Probe, die von dem Patienten vor dem Beginn der GPC3-zielgerichteten Arzneimitteltherapie und/oder des mit der GPC3-zielgerichteten Arzneimitteltherapie behandelten Patienten isoliert wurde, wobei das GPC3-zielgerichtete Arzneimittel einen Anti-GPC3-Antikörper umfasst, wobei es sich bei der biologischen Probe um Blut, Plasma oder Serum handelt und wobei, wenn die Konzentration von freiem GPC3 über einem vorbestimmten Wert liegt, bestimmt wird, dass die GPC3-zielgerichtete Arzneimitteltherapie wirksam ist, oder bestimmt wird, dass die GPC3-zielgerichtete Arzneimitteltherapie fortgesetzt werden soll.

**2.** Verfahren nach Anspruch 1, wobei:

(a) der vorbestimmte Wert von freiem GPC3 innerhalb des Bereichs von 0,1 ng/ml bis 100 ng/ml liegt;
(b) die Konzentration von freiem GPC3 unter Verwendung eines immunologischen Verfahrens gemessen wird;
(c) die Konzentration von freiem GPC3 größer ist als die in einer biologischen Probe, die vor Beginn der GPC3-zielgerichteten Arzneimitteltherapie aus dem Patienten isoliert wurde;
(d) der Patient eine hohe Expression von GPC3 in einem immunhistochemischen Färbungsscore zeigt;
(e) der Krebs ein Krebs mit hoher Expression von GPC3-Leberkrebs ist; und/oder
(f) das GPC3-zielgerichtete Arzneimittel verabreicht wird, um bei dem Krebspatienten einen Bluttalspiegel von 200 $\mu$g/ml oder mehr zu erreichen.

**3.** Verfahren nach Anspruch 1 oder 2, wobei:

(A) der Anti-GPC3-Antikörper eine antikörperabhängige zelluläre Zytotoxizität- (ADCC-) Aktivität und/oder eine komplementabhängige Zytotoxizität- (CDC-) Aktivität aufweist;
(B) der Anti-GPC3-Antikörper ein chimärer Anti-GPC3-Antikörper oder ein humanisierter Anti-GPC3-Antikörper ist, der eine der Folgenden von (1) bis (5) umfasst:

(1) CDR1 der schweren Kette, CDR2 der schweren Kette und CDR3 der schweren Kette, dargestellt durch SEQ ID NO: 4, 5 beziehungsweise 6, und CDR1 der leichten Kette, CDR2 der leichten Kette und CDR3 der leichten Kette, dargestellt durch SEQ ID NO: 7, 8 beziehungsweise 9;
(2) CDR1 der schweren Kette, CDR2 der schweren Kette und CDR3 der schweren Kette, dargestellt durch die SEQ ID NO: 12, 13 beziehungsweise 14, und CDR1 der leichten Kette, CDR2 der leichten Kette und CDR3 der leichten Kette, dargestellt durch die SEQ ID NO: 15, 16 beziehungsweise 17;
(3) CDR1 der schweren Kette, CDR2 der schweren Kette und CDR3 der schweren Kette, dargestellt durch die SEQ ID NO: 20, 21 beziehungsweise 22, und CDR1 der leichten Kette, CDR2 der leichten Kette und CDR3 der leichten Kette, dargestellt durch die SEQ ID NO: 23, 24 beziehungsweise 25;
(4) CDR1 der schweren Kette, CDR2 der schweren Kette und CDR3 der schweren Kette, dargestellt durch die SEQ ID NO: 28, 29 beziehungsweise 30, und CDR1 der leichten Kette, CDR2 der leichten Kette und CDR3 der leichten Kette, dargestellt durch die SEQ ID NO: 31, 32 beziehungsweise 33; und
(5) CDR1 der schweren Kette, CDR2 der schweren Kette und CDR3 der schweren Kette, jeweils dargestellt durch die SEQ ID NO: 36, 37 und 38, und CDR1 der leichten Kette, CDR2 der leichten Kette und CDR3 der leichten Kette, jeweils dargestellt durch die SEQ ID NO: 39, 40 und 41; und/oder

(C) der Anti-GPC3-Antikörper eine der Folgenden von (1) bis ( 6) umfasst:

(1) eine variable Region der schweren Kette, ausgewählt aus der Gruppe der variablen Regionen der schweren Kette, dargestellt durch SEQ ID NO: 44, 45, 46, 47, 48, 49 und 50, und eine variable Region der leichten Kette, dargestellt durch SEQ ID NO: 51;
(2) eine variable Region der schweren Kette, ausgewählt aus der Gruppe der variablen Regionen der schweren Kette, dargestellt durch SEQ ID NO: 44, 45, 46, 47, 48, 49 und 50, und eine variable Region der leichten Kette, ausgewählt aus der Gruppe der variablen Regionen der leichten Kette, dargestellt durch SEQ ID NO: 52, 53, 54, 55, 56, 57, 58, 59, 60, 61, 62, 63, 64, 65 und 66;
(3) eine variable Region der schweren Kette, dargestellt durch SEQ ID NO:67 und eine variable Region der leichten Kette, dargestellt durch SEQ ID NO: 68;
(4) eine variable Region der schweren Kette, dargestellt durch SEQ ID NO: 69, und eine variable Region der leichten Kette, dargestellt durch SEQ ID NO: 70;
(5) eine variable Region der schweren Kette, die durch SEQ ID NO: 71 dargestellt wird, und eine variable Region der leichten Kette, die durch SEQ ID NO: 72 dargestellt wird; und
(6) eine variable Region der schweren Kette, dargestellt durch SEQ ID NO: 71, und eine variable Region der leichten Kette, dargestellt durch SEQ ID NO: 73.

**4.** Verfahren nach einem der Ansprüche 1 bis 3, wobei der Anti-GPC3-Antikörper mit einer zytotoxischen Substanz konjugiert worden ist.

**5.** Verfahren nach Anspruch 1 oder 2, wobei es sich bei dem Krebs um Krebs handelt, der aus Brustkrebs, Gebärmutterhalskrebs, Dickdarmkrebs, Gebärmutterkörperkrebs, Kopf- und Halskrebs, Lungenkrebs, malignem Gliom, malignem Lymphom, Eierstockkrebs, Bauchspeicheldrüsenkrebs, Prostatakrebs, Nierenkrebs, Hautkrebs, Magenkrebs, Hodenkrebs, Schilddrüsenkrebs, Urothelkrebs ausgewählt ist.

**6.** GPC3-zielgerichtetes Arzneimittel für die Verwendung in einem Verfahren zum Behandeln von GPC3-exprimierendem Krebs, wobei in dem Verfahren das Verfahren umfasst:

(a) Durchführen des Verfahrens nach einem der Ansprüche 1 bis 6; und
(b) Verabreichen des GPC3-zielgerichteten Arzneimittels an den Patienten, für den bestimmt worden ist, dass das GPC3-zielgerichtete Arzneimittel wirksam ist, oder für den bestimmt worden ist, dass das GPC3-zielgerichtete Arzneimittel fortgesetzt wird,

wobei das GPC3-zielgerichtete Arzneimittel einen Anti-GPC3-Antikörper umfasst.

## Revendications

**1.** Procédé pour déterminer l'efficacité d'un traitement médicamenteux ciblant GPC3 pour un cancer exprimant GPC3 chez un patient ou pour déterminer la poursuite d'un traitement médicamenteux ciblant GPC3 chez un patient, comprenant la surveillance d'une concentration de GPC3 libre dans un échantillon biologique isolé provenant du patient avant le début du traitement médicamenteux ciblant GPC3 et/ou du patient traité avec le traitement médicamenteux ciblant GPC3, dans lequel le médicament ciblant GPC3 comprend un anticorps anti-GPC3, dans lequel l'échantillon biologique est du sang, du plasma ou du sérum, et dans lequel lorsque la concentration de GPC3 libre est supérieure à une valeur prédéterminée, le traitement médicamenteux ciblant GPC3 est déterminé comme étant efficace ou le traitement médicamenteux ciblant GPC3 est déterminé comme étant poursuivi.

**2.** Procédé selon la revendication 1, dans lequel :

(a) la valeur prédéterminée de GPC3 libre est dans la plage de 0,1 ng/mL à 100 ng/mL ;
(b) la concentration de GPC3 libre est mesurée en utilisant un procédé immunologique ;
(c) la concentration de GPC3 libre est supérieure à celle d'un échantillon biologique isolé avant le début du traitement médicamenteux ciblant GPC3 du patient ;
(d) le patient présente une expression élevée de GPC3 dans un score de coloration immunohistochimique ;
(e) le cancer est un cancer ayant une expression élevée de cancer du foie exprimant GPC3 ; et/ou
(f) le médicament ciblant GPC3 est administré pour atteindre un niveau sanguin de 200 μg/ml ou plus chez le patient cancéreux.

**3.** Procédé selon la revendication 1 ou 2, dans lequel :

(A) l'anticorps anti-GPC3 a une activité de cytotoxicité cellulaire dépendante des anticorps (ADCC) et/ou une activité de cytotoxicité dépendante du complément (CDC) ;
(B) l'anticorps anti-GPC3 est un anticorps chimérique anti-GPC3 ou un anticorps anti-GPC3 humanisé comprenant l'un des éléments suivants (1) à (5) :

(1) une CDR1 à chaîne lourde, une CDR2 à chaîne lourde et une CDR3 à chaîne lourde représentées par les SEQ ID NO : 4, 5 et 6, respectivement, et une CDR1 à chaîne légère, une CDR2 à chaîne légère et une CDR3 à chaîne légère représentées par les SEQ ID NO : 7, 8 et 9, respectivement ;
(2) une CDR1 à chaîne lourde, une CDR2 à chaîne lourde et une CDR3 à chaîne lourde représentées par SEQ ID NO: 12, 13 et 14, respectivement, et une CDR1 à chaîne légère, une CDR2 à chaîne légère et une CDR3 à chaîne légère représentées par SEQ ID NO : 15, 16 et 17, respectivement ;
(3) une CDR1 à chaîne lourde, une CDR2 à chaîne lourde et une CDR3 à chaîne lourde représentées par les SEQ ID NO : 20, 21 et 22, respectivement, et une CDR1 à chaîne légère, une CDR2 à chaîne légère et une CDR3 à chaîne légère représentées par les SEQ ID NO : 23, 24 et 25, respectivement ;
(4) une CDR1 à chaîne lourde, une CDR2 à chaîne lourde et une CDR3 à chaîne lourde représentées par SEQ ID NO : 28, 29 et 30, respectivement, et une CDR1 à chaîne légère, une CDR2 à chaîne légère et une CDR3 à chaîne légère représentées par SEQ ID NO : 31, 32 et 33, respectivement ; et
(5) une CDR1 à chaîne lourde, une CDR2 à chaîne lourde et une CDR3 à chaîne lourde représentées par les SEQ ID NO : 36, 37 et 38, respectivement, et une CDR1 à chaîne légère, une CDR2 à chaîne légère et une CDR3 à chaîne légère représentées par les SEQ ID NO : 39, 40 et 41, respectivement ; et/ou

(C) l'anticorps anti-GPC3 comprend l'un quelconque des éléments (1) à (6) suivants :

(1) une région variable de chaîne lourde choisie dans le groupe des régions variables de chaîne lourde représentées par les SEQ ID NO : 44, 45, 46, 47, 48, 49 et 50 et une région variable de chaîne légère représentée par SEQ ID NO : 51 ;
(2) une région variable de chaîne lourde choisie dans le groupe des régions variables de chaîne lourde représentées par les SEQ ID NO : 44, 45, 46, 47, 48, 49 et 50 et une région variable de chaîne légère choisie dans le groupe des régions variables de chaîne légère représentées par les SEQ ID NO : 52, 53, 54, 55, 56, 57, 58, 59, 60, 61, 62, 63, 64, 65 et 66 ;
(3) une région variable de chaîne lourde représentée par SEQ ID NO : 67 et une région variable de chaîne légère représentée par SEQ ID NO : 68 ;
(4) une région variable de chaîne lourde représentée par SEQ ID NO : 69 et une région variable de chaîne légère représentée par SEQ ID NO: 70 ;
(5) une région variable de chaîne lourde représentée par SEQ ID NO : 71 et une région variable de chaîne légère représentée par SEQ ID NO : 72 ; et
(6) une région variable de chaîne lourde représentée par SEQ ID NO : 71 et une région variable de chaîne légère représentée par SEQ ID NO : 73.

**4.** Procédé selon l'une quelconque des revendications 1 à 3, dans lequel l'anticorps anti-GPC3 a été conjugué avec une substance cytotoxique.

**5.** Procédé selon la revendication 1 ou 2, le cancer est un cancer choisi parmi le cancer du sein, le cancer du col de l'utérus, le cancer du côlon, le cancer du corps utérin, le cancer de la tête et du cou, le cancer du poumon, le gliome malin, le lymphome malin, le cancer de l'ovaire, le cancer du pancréas, le cancer de la prostate, le cancer du rein, le cancer de la peau, le cancer gastrique, le cancer des testicules, le cancer de la thyroïde, le cancer urothélial.

**6.** Médicament ciblant GPC3 pour une utilisation dans un procédé de traitement d'un cancer exprimant GPC3, dans lequel, dans le procédé, le procédé comprend :

(a) l'exécution du procédé selon l'une quelconque des revendications 1 à 6 ; et
(b) l'administration du médicament ciblant GPC3 au patient pour lequel le médicament ciblant GPC3 a été déterminé comme étant efficace ou pour lequel le médicament ciblant GPC3 a été déterminé comme étant poursuivi,

dans lequel le médicament ciblant GPC3 comprenant un anticorps anti-GPC3.

FIG.1A

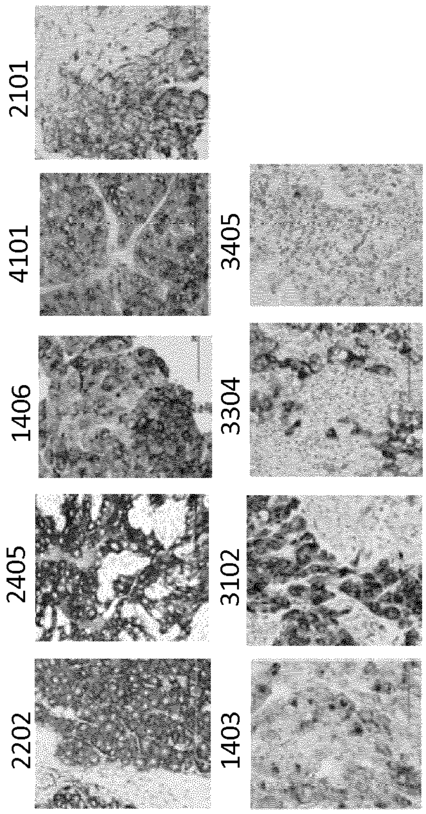
2202
2405
1406
4101
2101
1403
3102
3304
3405

FIG.1B

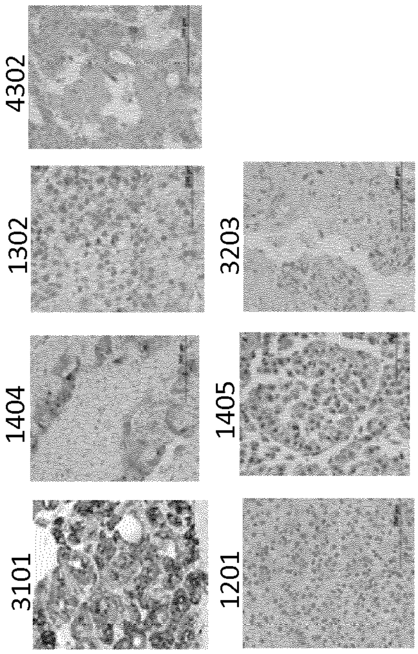
3101
1404
1302
4302
1201
1405
3203

FIG.2

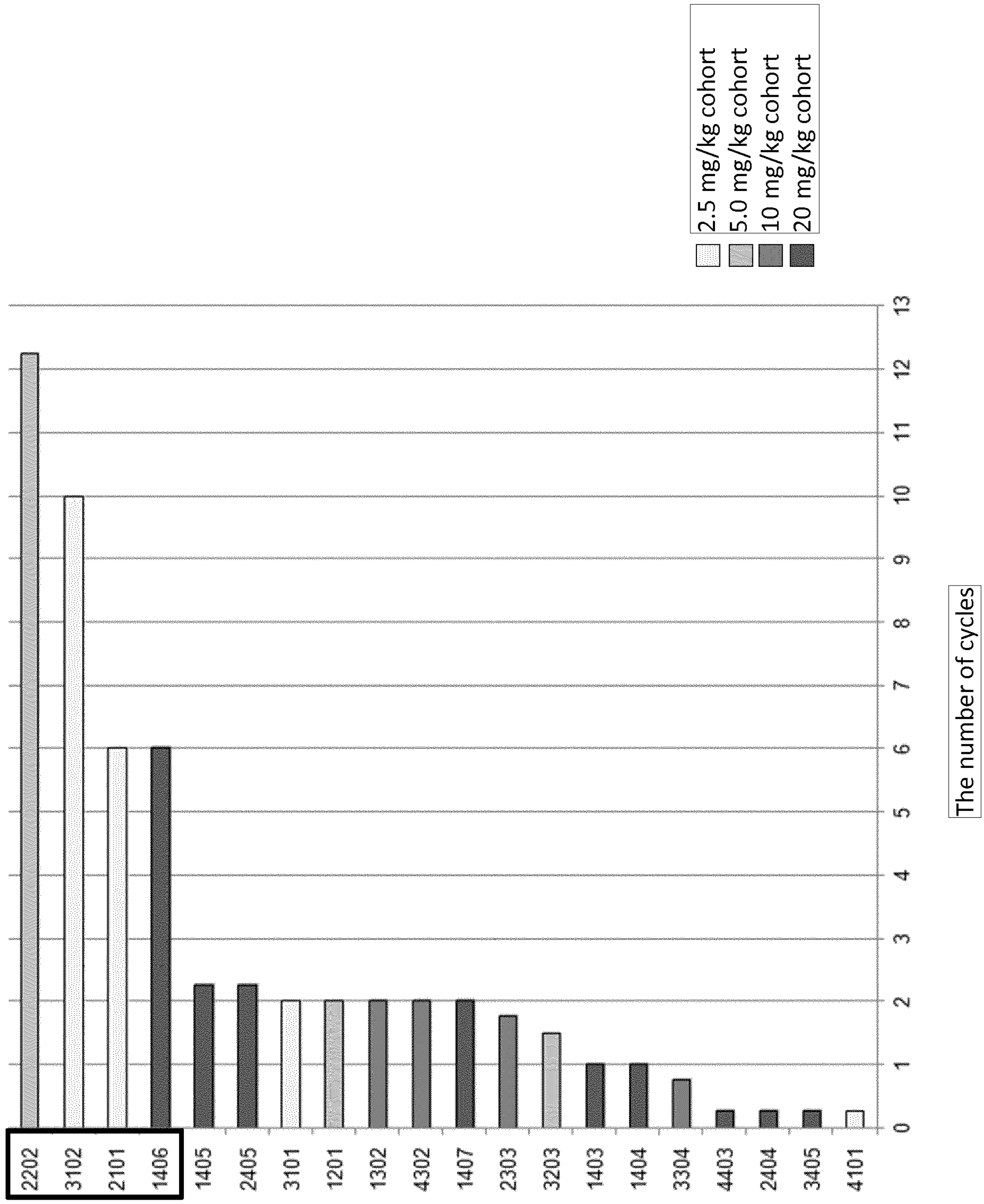

2.5 mg/kg cohort
5.0 mg/kg cohort
10 mg/kg cohort
20 mg/kg cohort
The number of cycles
0
1
2
3
4
5
6
7
8
9
10
11
12
13
2202
3102
2101
1406
1405
2405
3101
1201
1302
4302
1407
2303
3203
1403
1404
3304
4403
2404
3405
4101

FIG.3

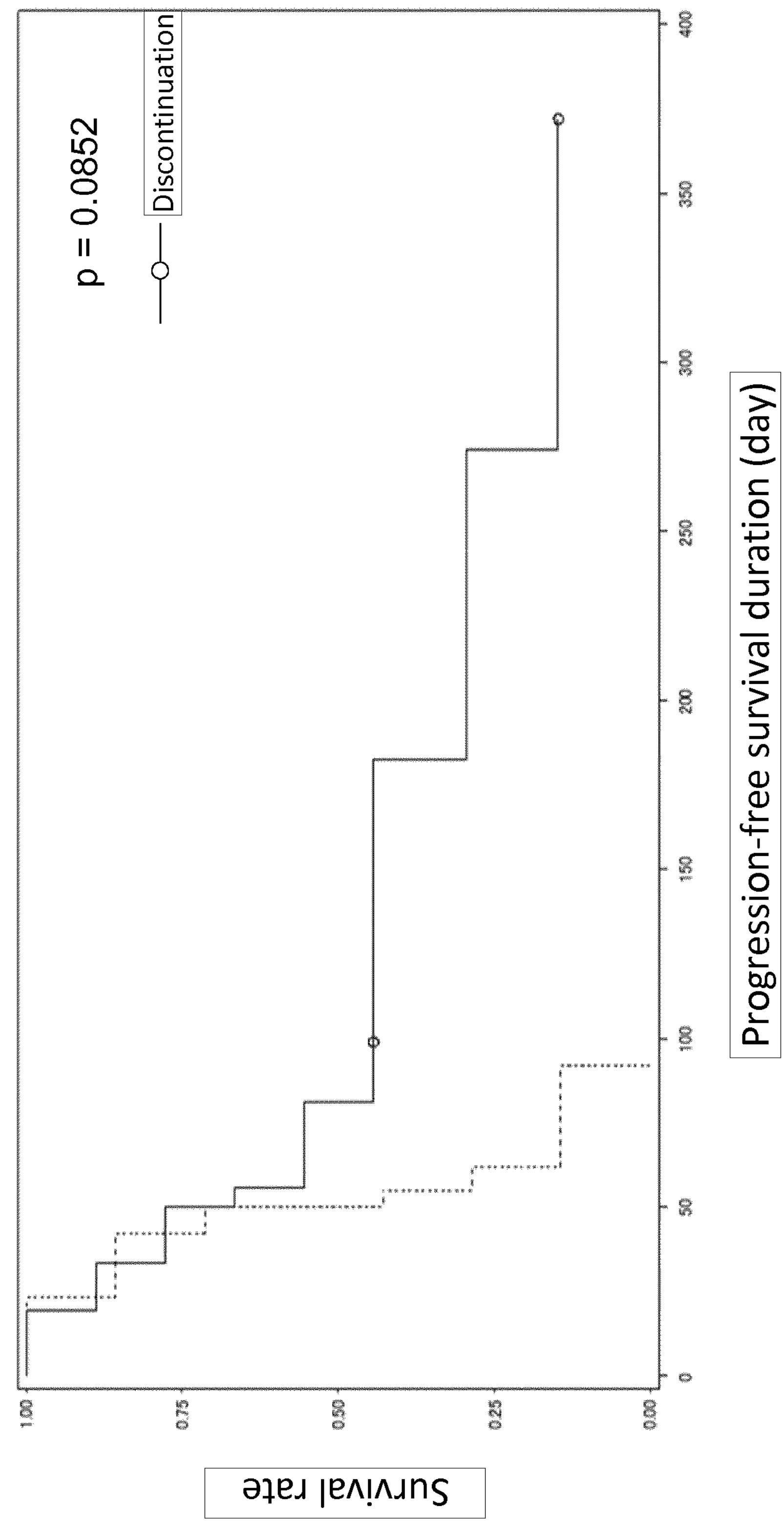
p = 0.0852
Discontinuation
Survival rate
1.00
0.75
0.50
0.25
0.00
0
50
100
150
200
250
300
350
400
Progression-free survival duration (day)

FIG.4 A

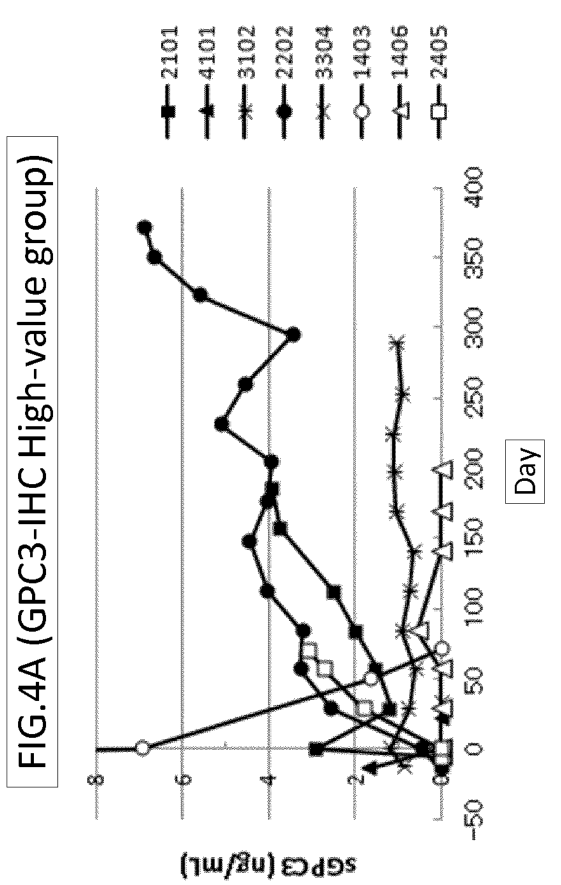
FIG.4A (GPC3-IHC High-value group)
sGPC3 (ng/mL)
Day
8
6
4
2
0
-50
0
50
100
150
200
250
300
350
400
2101
4101
3102
2202
3304
1403
1406
2405

FIG.4 B

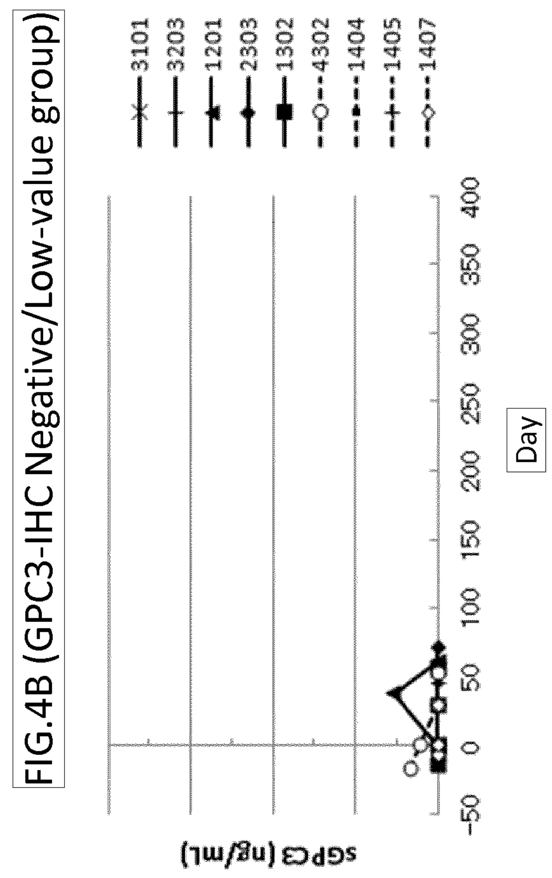
FIG.4B (GPC3-IHC Negative/Low-value group)
sGPC3 (ng/mL)
Day
-50
0
50
100
150
200
250
300
350
400
3101
3203
1201
2303
1302
4302
1404
1405
1407

FIG.5A

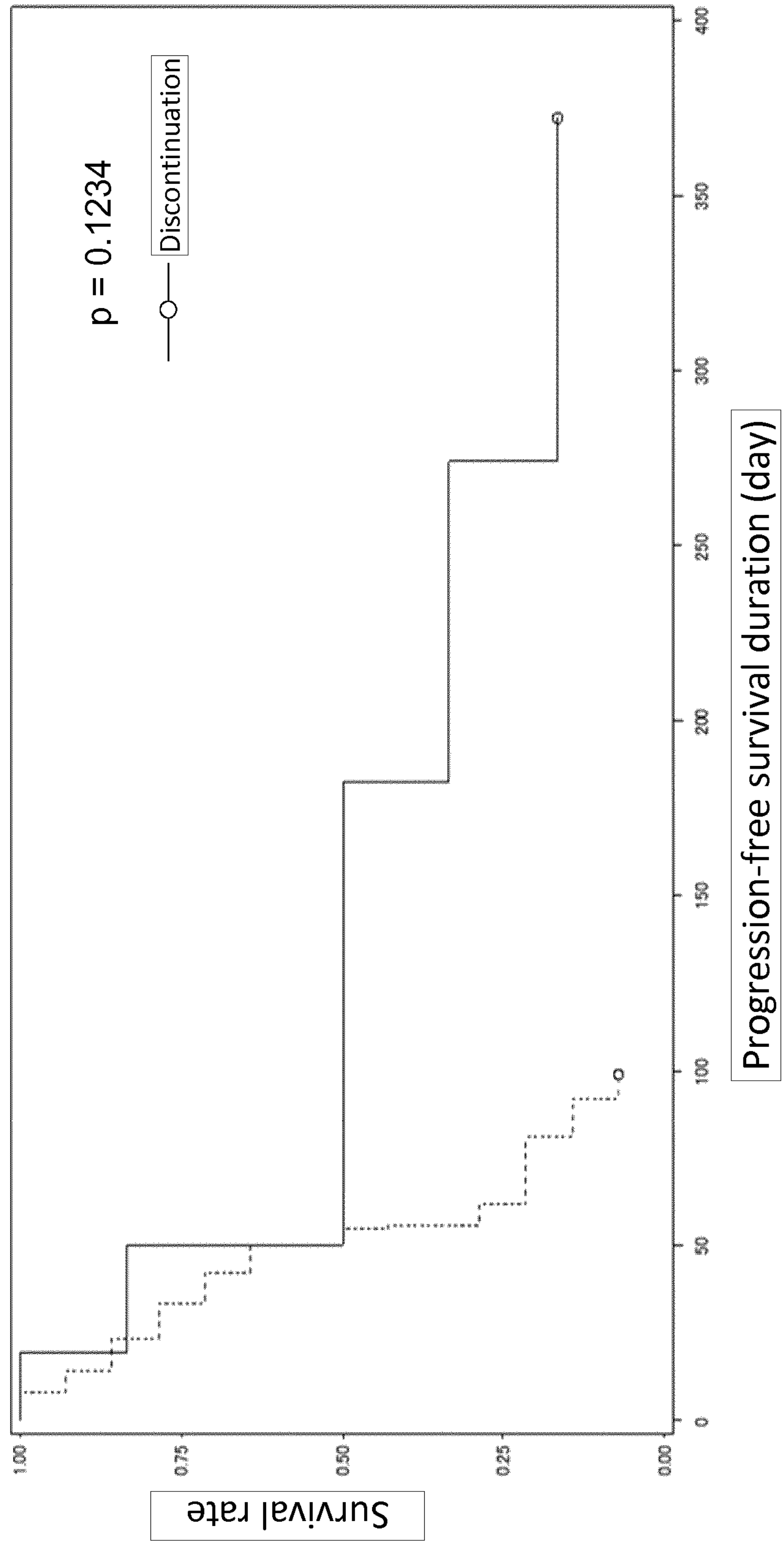
p = 0.1234
Discontinuation
Survival rate
1.00
0.75
0.50
0.25
0.00
Progression-free survival duration (day)
0
50
100
150
200
250
300
350
400

FIG.5B

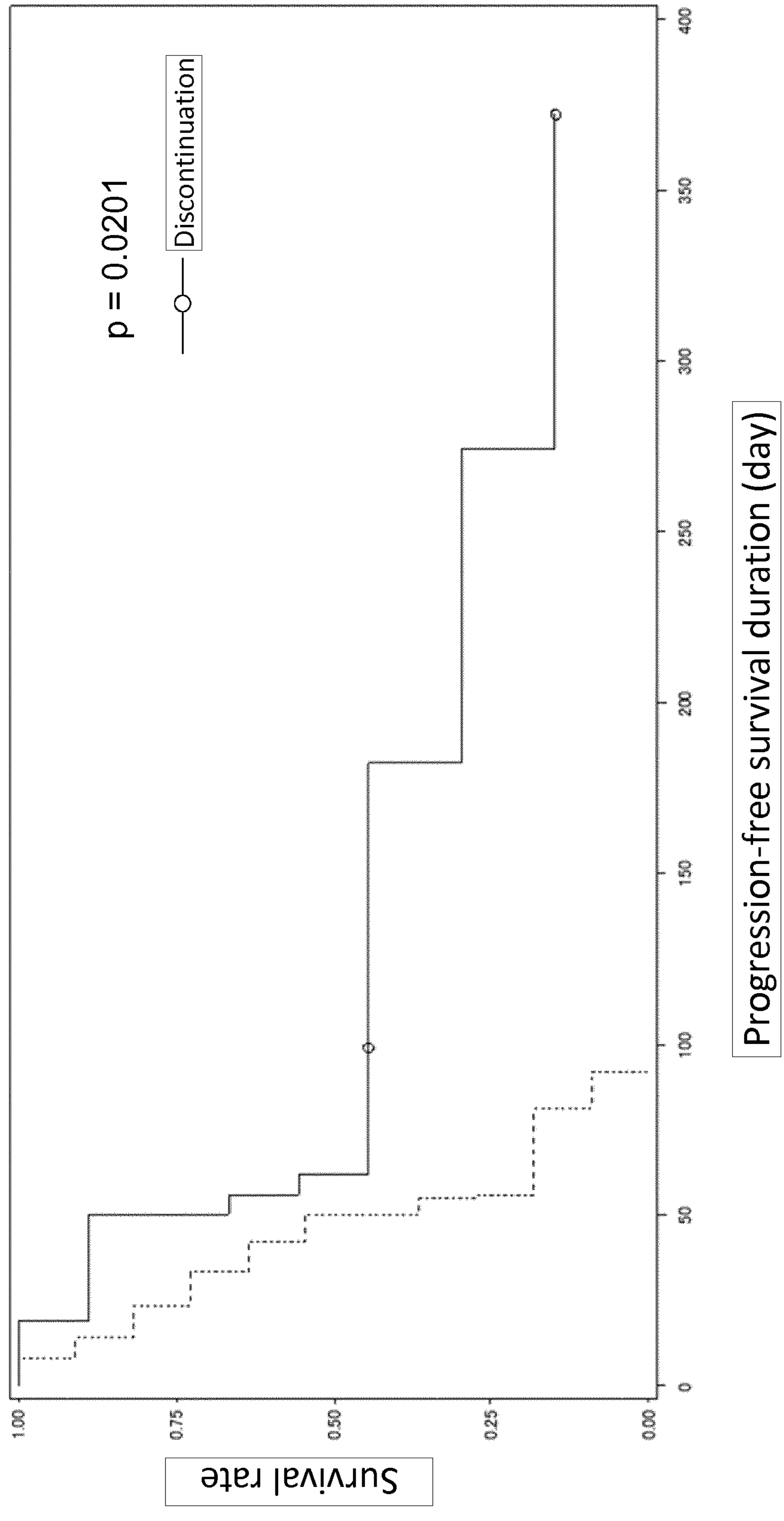
p = 0.0201
Discontinuation
Survival rate
1.00
0.75
0.50
0.25
0.00
Progression-free survival duration (day)
0
50
100
150
200
250
300
350
400

FIG.6A

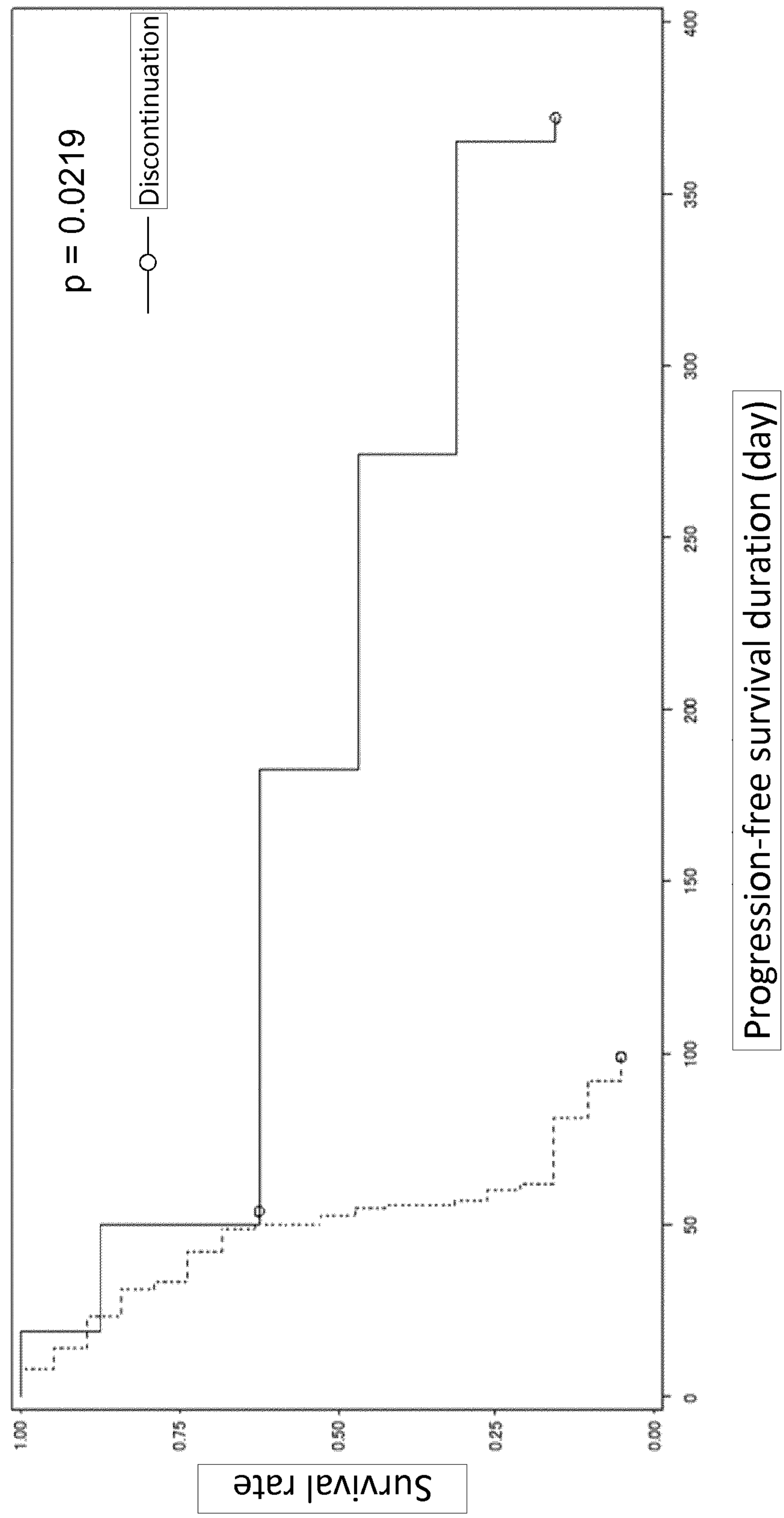

p = 0.0219
Discontinuation
Survival rate
1.00
0.75
0.50
0.25
0.00
0
50
100
150
200
250
300
350
400
Progression-free survival duration (day)

FIG.6B

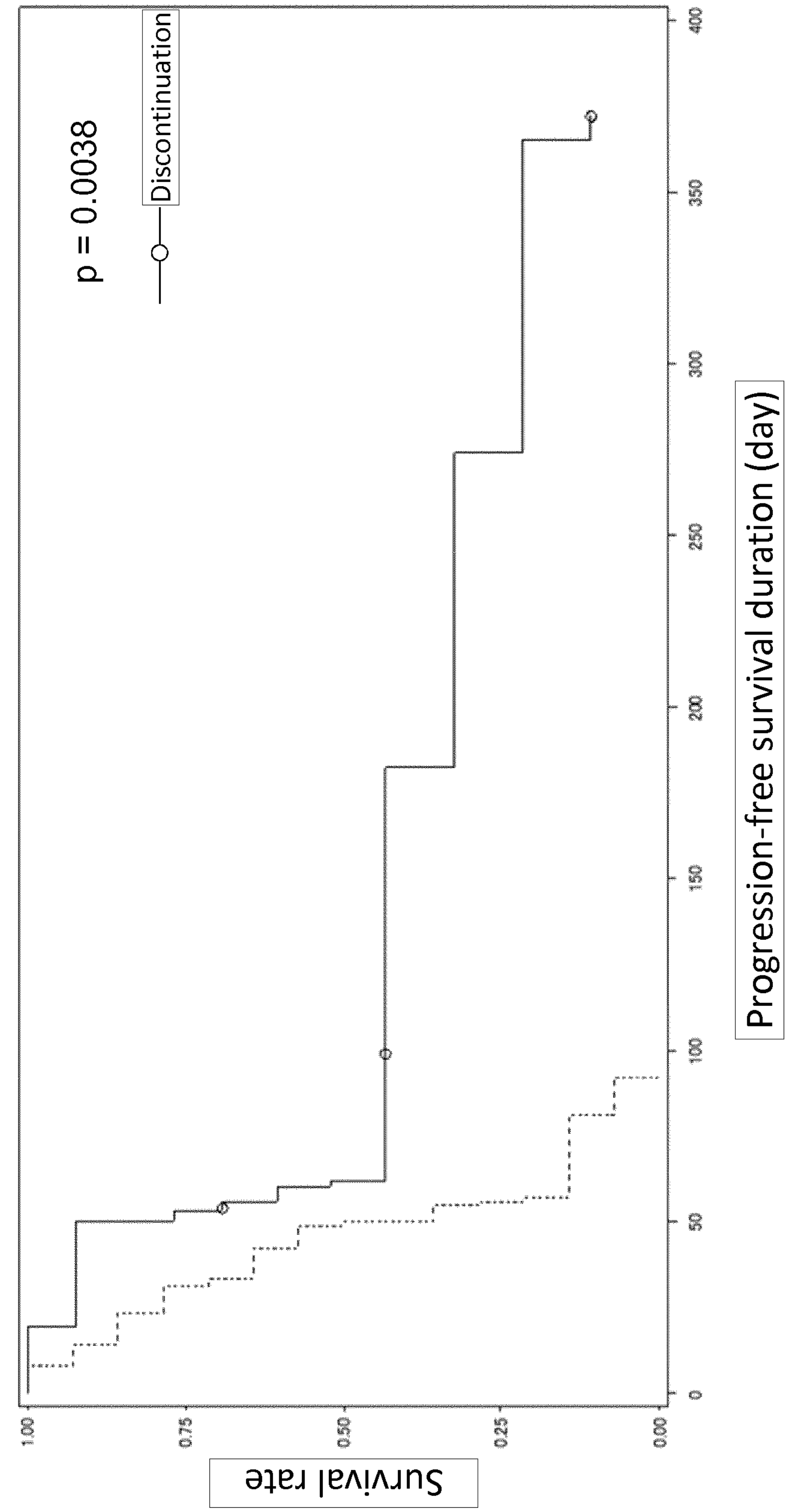
p = 0.0038
Discontinuation
Survival rate
1.00
0.75
0.50
0.25
0.00
0
50
100
150
200
250
300
350
400
Progression-free survival duration (day)

FIG.7A

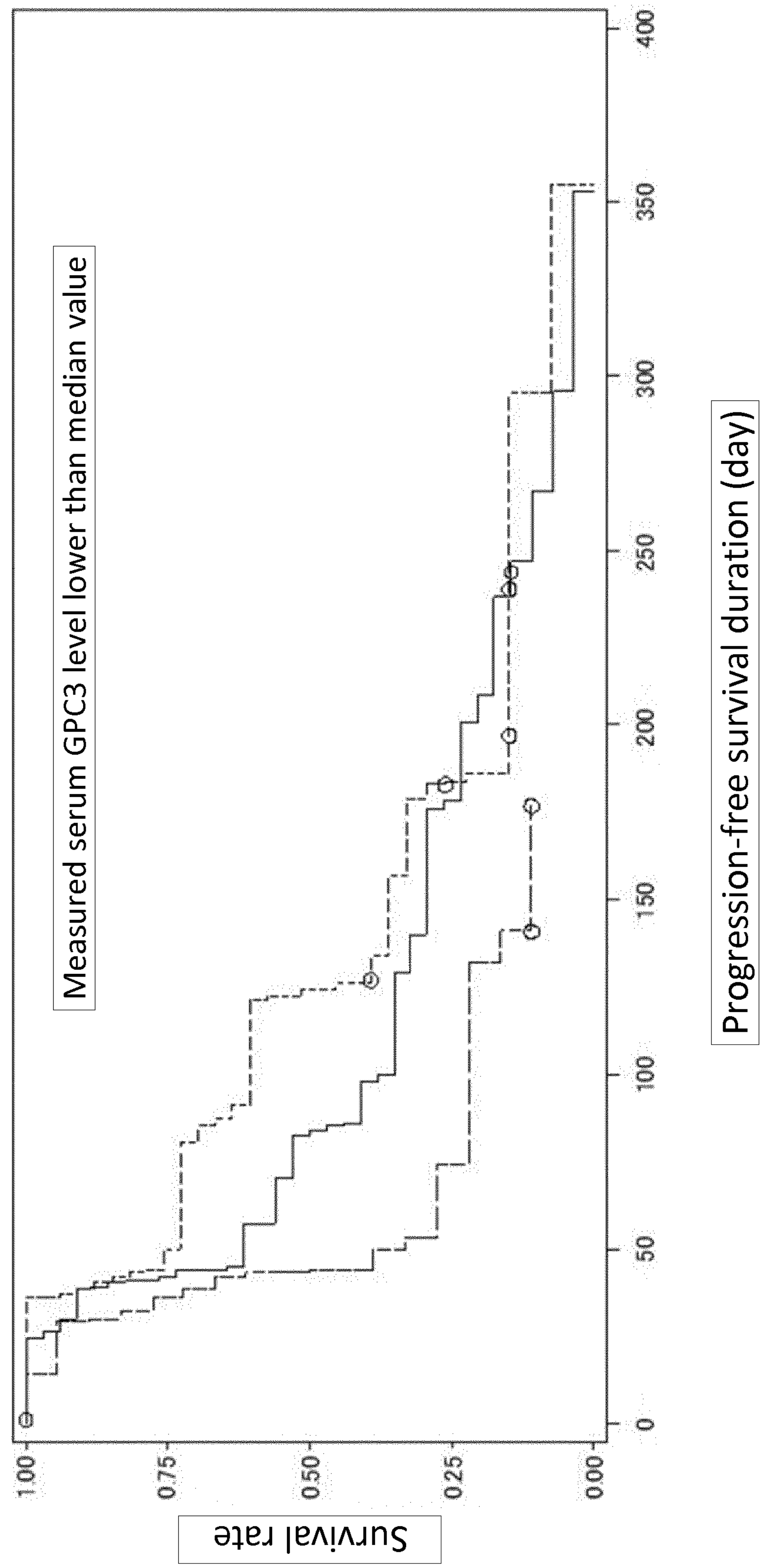
Measured serum GPC3 level lower than median value
Survival rate
1.00
0.75
0.50
0.25
0.00
0
50
100
150
200
250
300
350
400
Progression-free survival duration (day)

FIG.7B

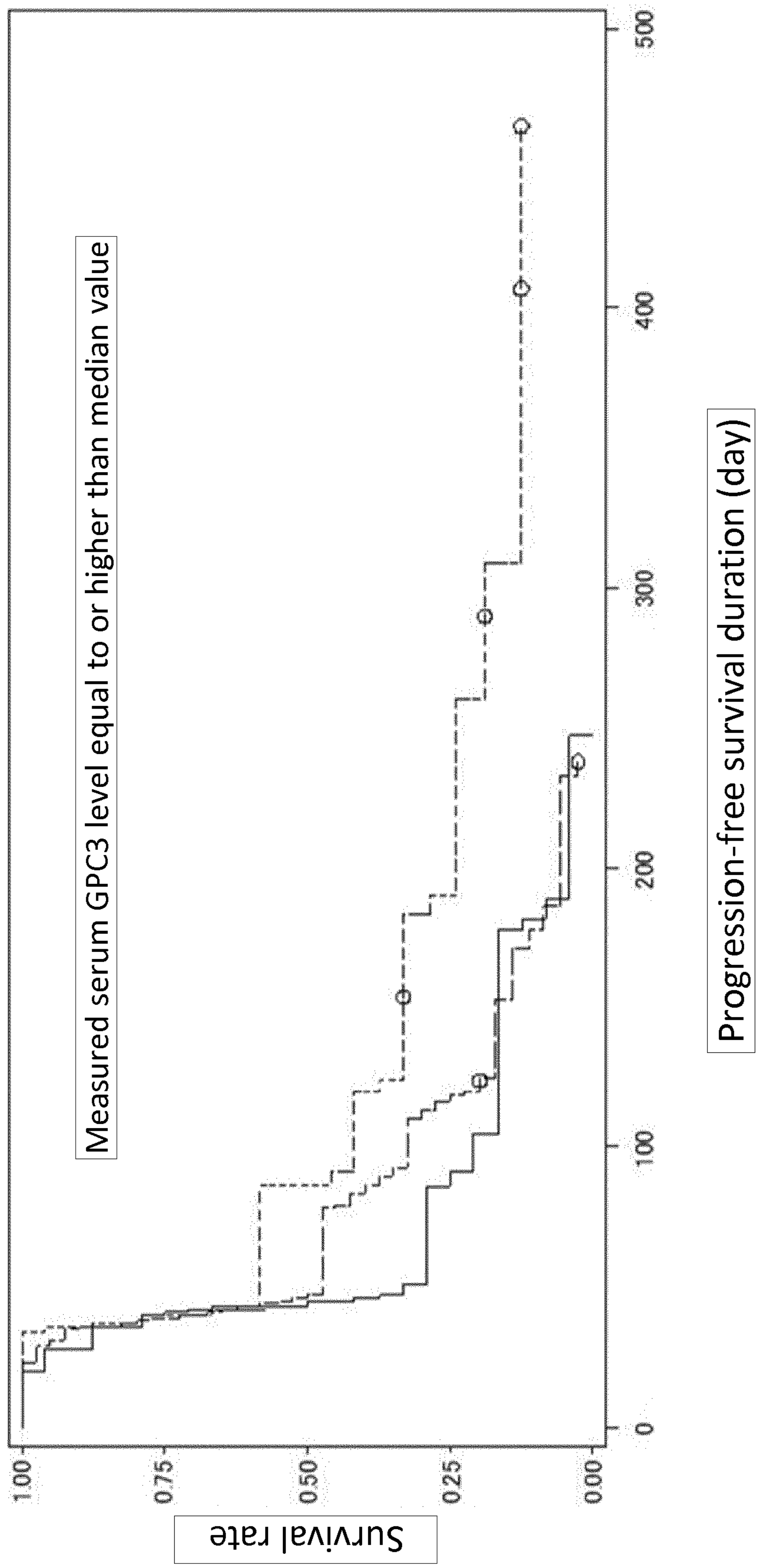
Measured serum GPC3 level equal to or higher than median value
Survival rate
1.00
0.75
0.50
0.25
0.00
0
100
200
300
400
500
Progression-free survival duration (day)

FIG.7C

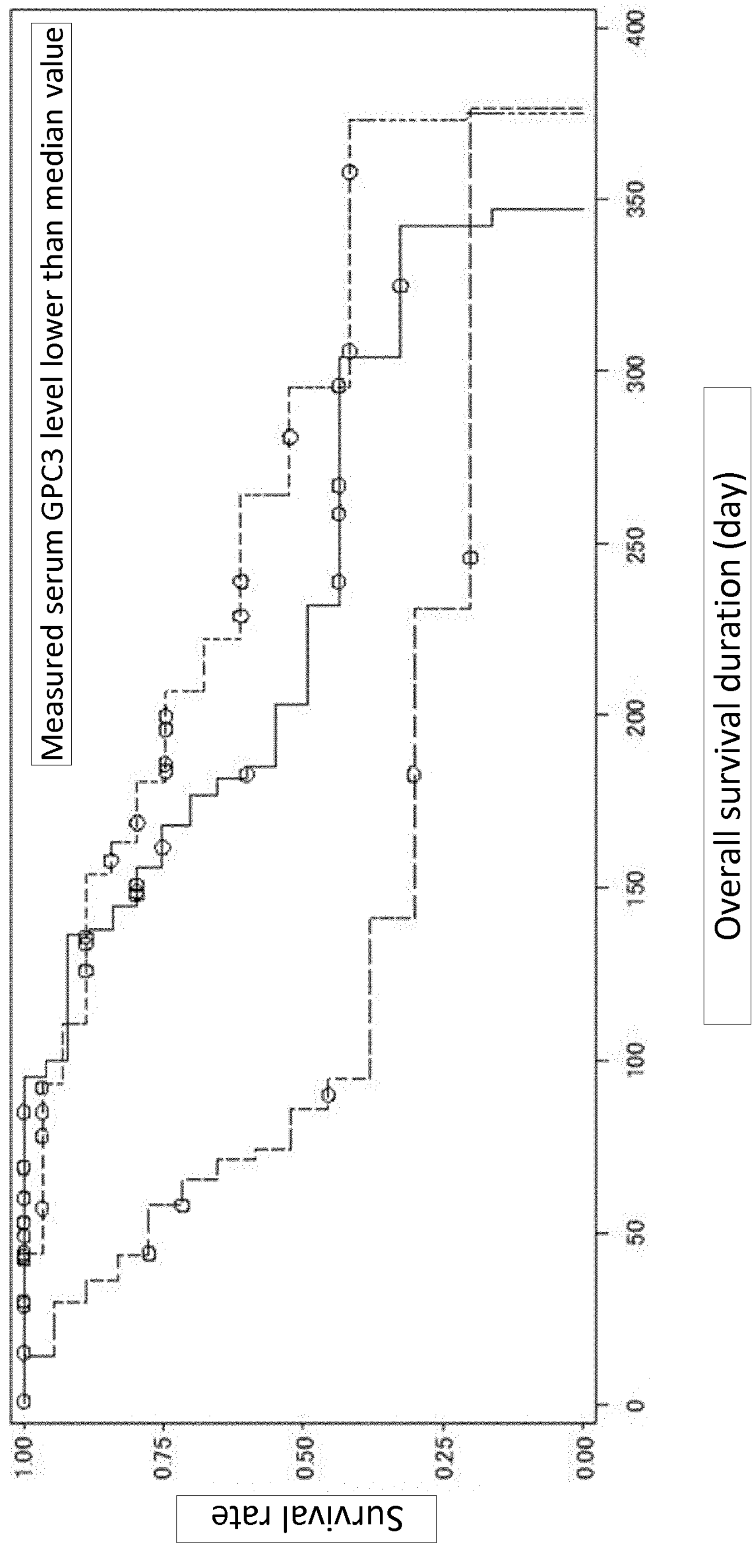
Measured serum GPC3 level lower than median value
Overall survival duration (day)
Survival rate
1.00
0.75
0.50
0.25
0.00
0
50
100
150
200
250
300
350
400

FIG.7D

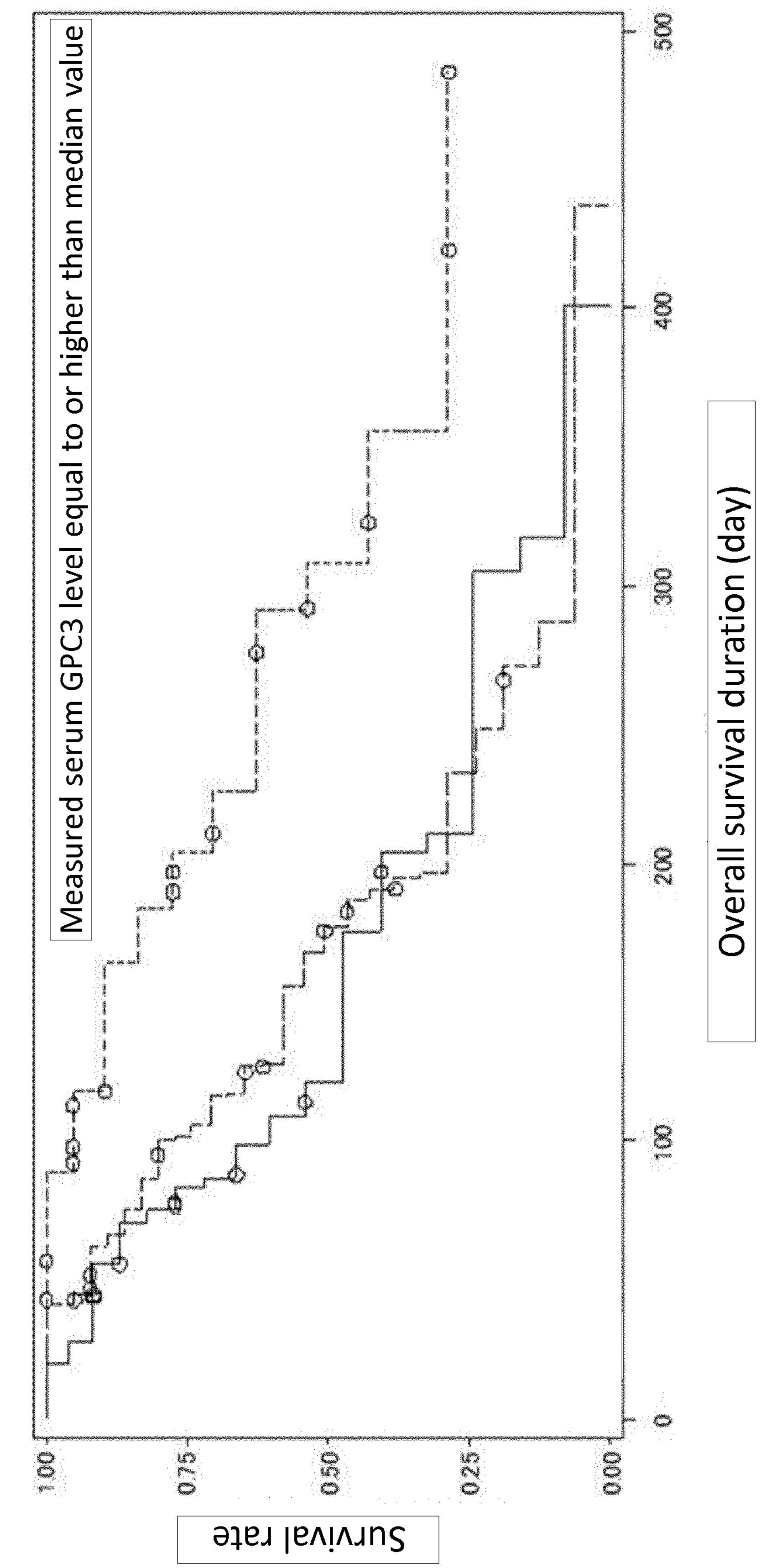
Measured serum GPC3 level equal to or higher than median value
Survival rate
1.00
0.75
0.50
0.25
0.00
0
100
200
300
400
500
Overall survival duration (day)

FIG.7E

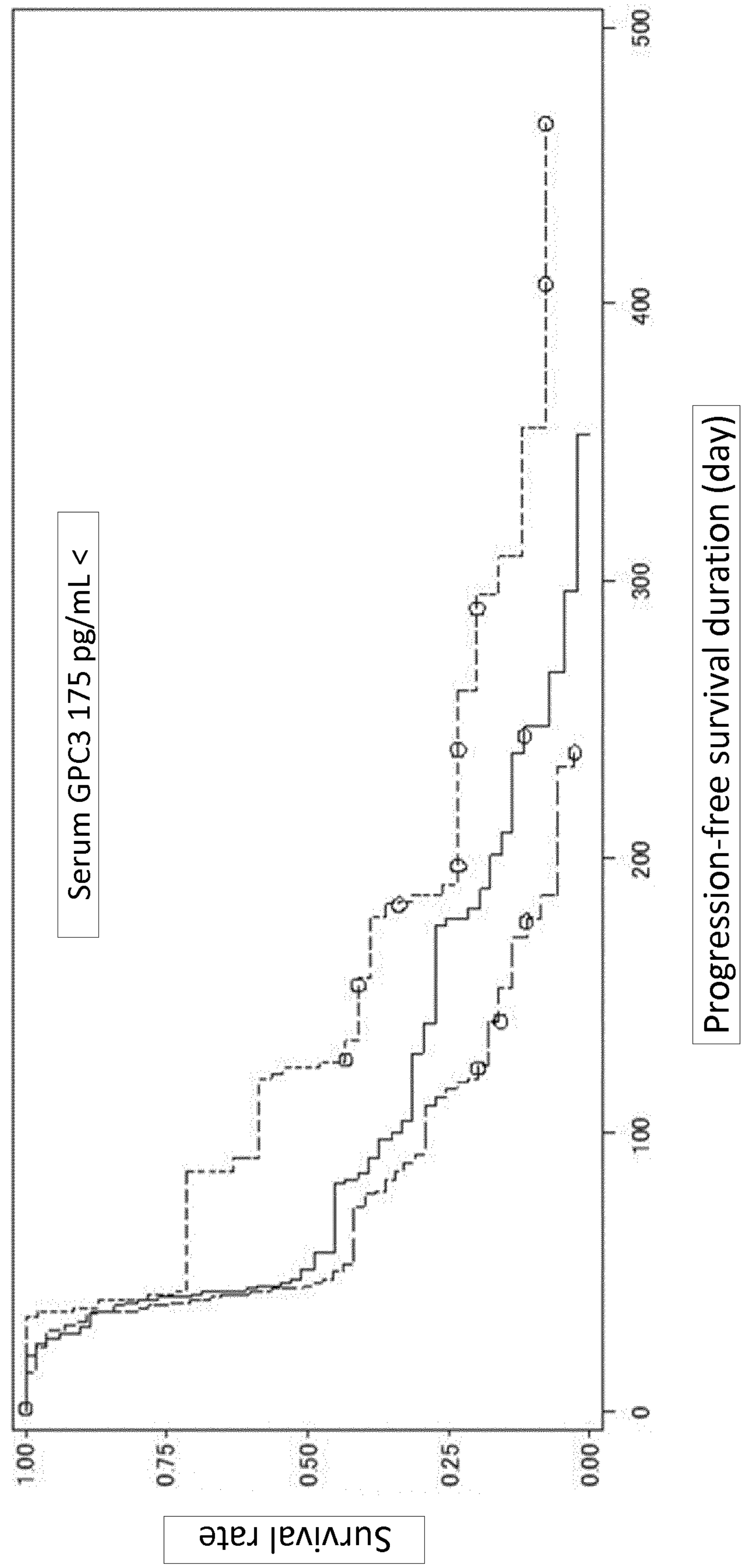
Serum GPC3 175 pg/mL <
Survival rate
1.00
0.75
0.50
0.25
0.00
0
100
200
300
400
500
Progression-free survival duration (day)

FIG.7F

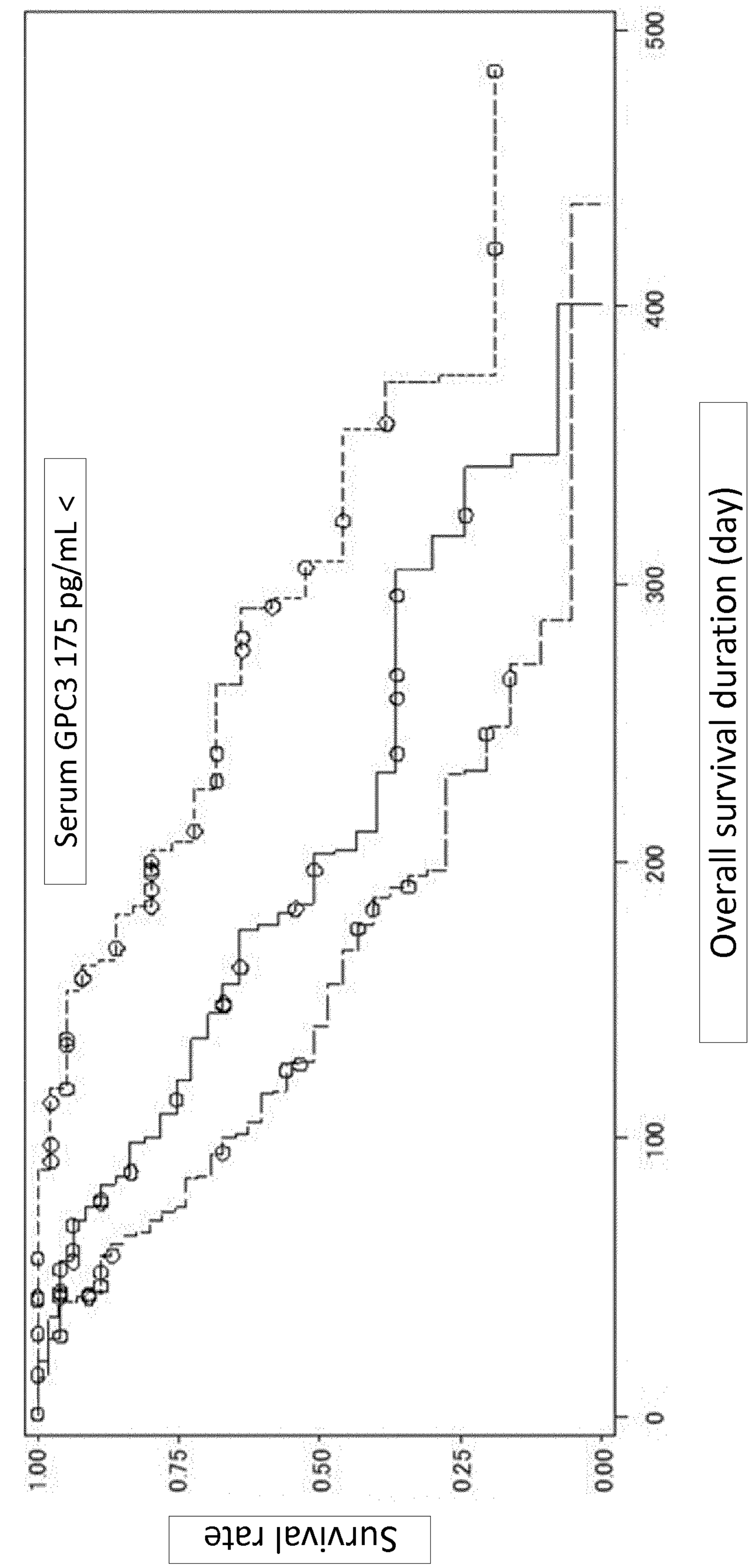
Serum GPC3 175 pg/mL <
Overall survival duration (day)
Survival rate
0
100
200
300
400
500
0.00
0.25
0.50
0.75
1.00

FIG.8A

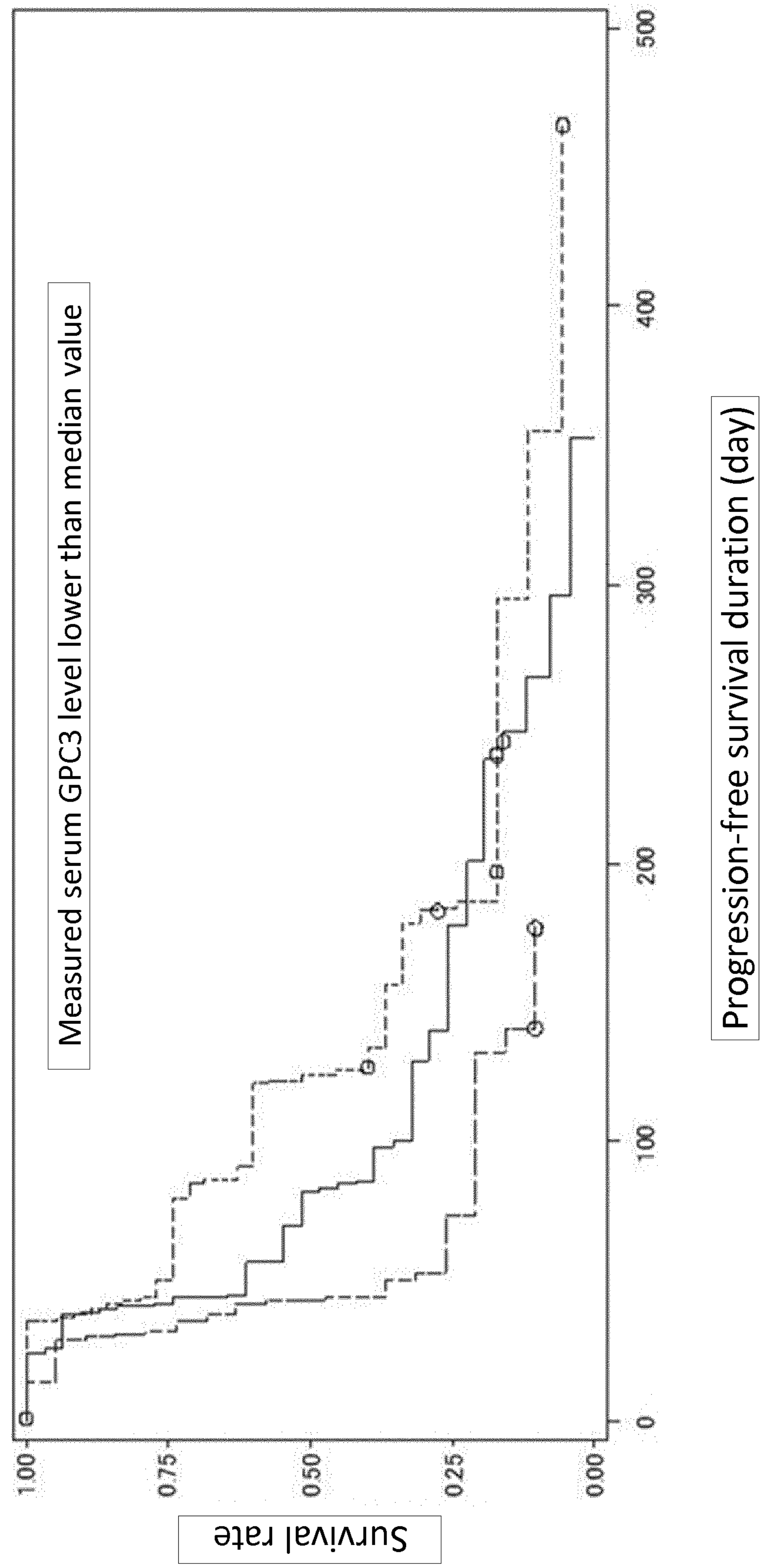
Measured serum GPC3 level lower than median value
Survival rate
1.00
0.75
0.50
0.25
0.00
0
100
200
300
400
500
Progression-free survival duration (day)

FIG.8B

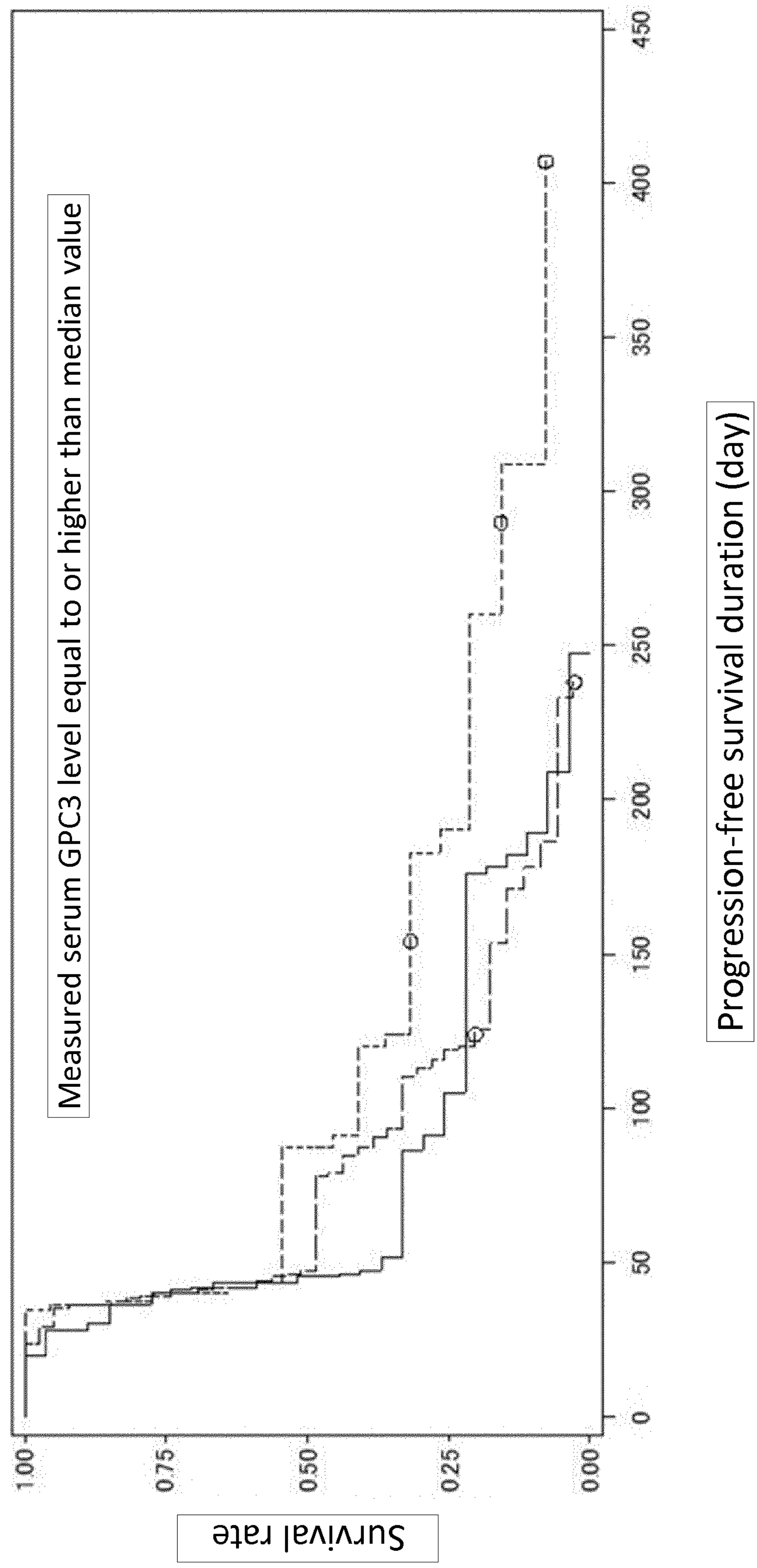
Measured serum GPC3 level equal to or higher than median value
Survival rate
1.00
0.75
0.50
0.25
0.00
0
50
100
150
200
250
300
350
400
450
Progression-free survival duration (day)

FIG.8C

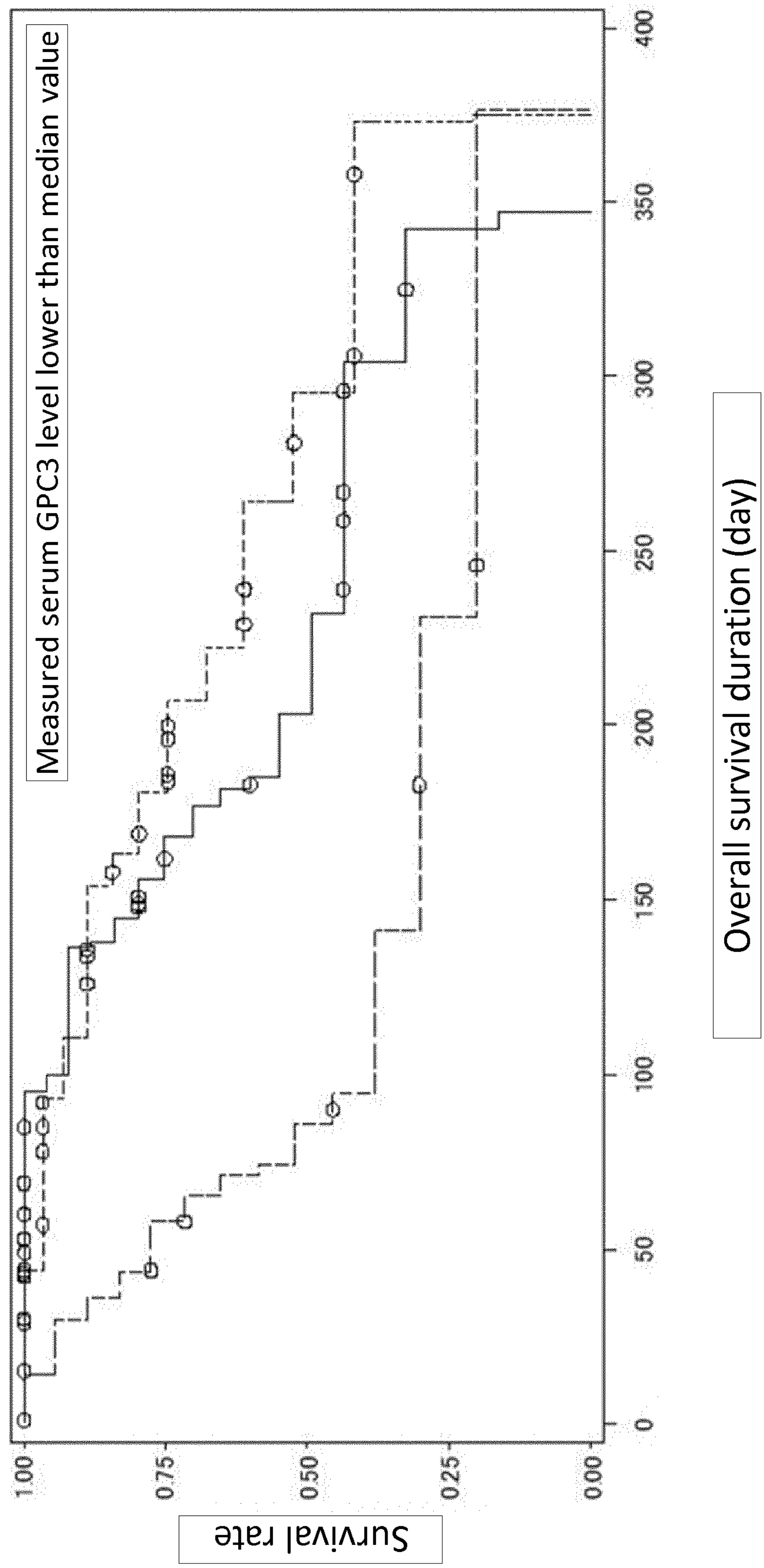
Measured serum GPC3 level lower than median value
Survival rate
1.00
0.75
0.50
0.25
0.00
0
50
100
150
200
250
300
350
400
Overall survival duration (day)

FIG.8D

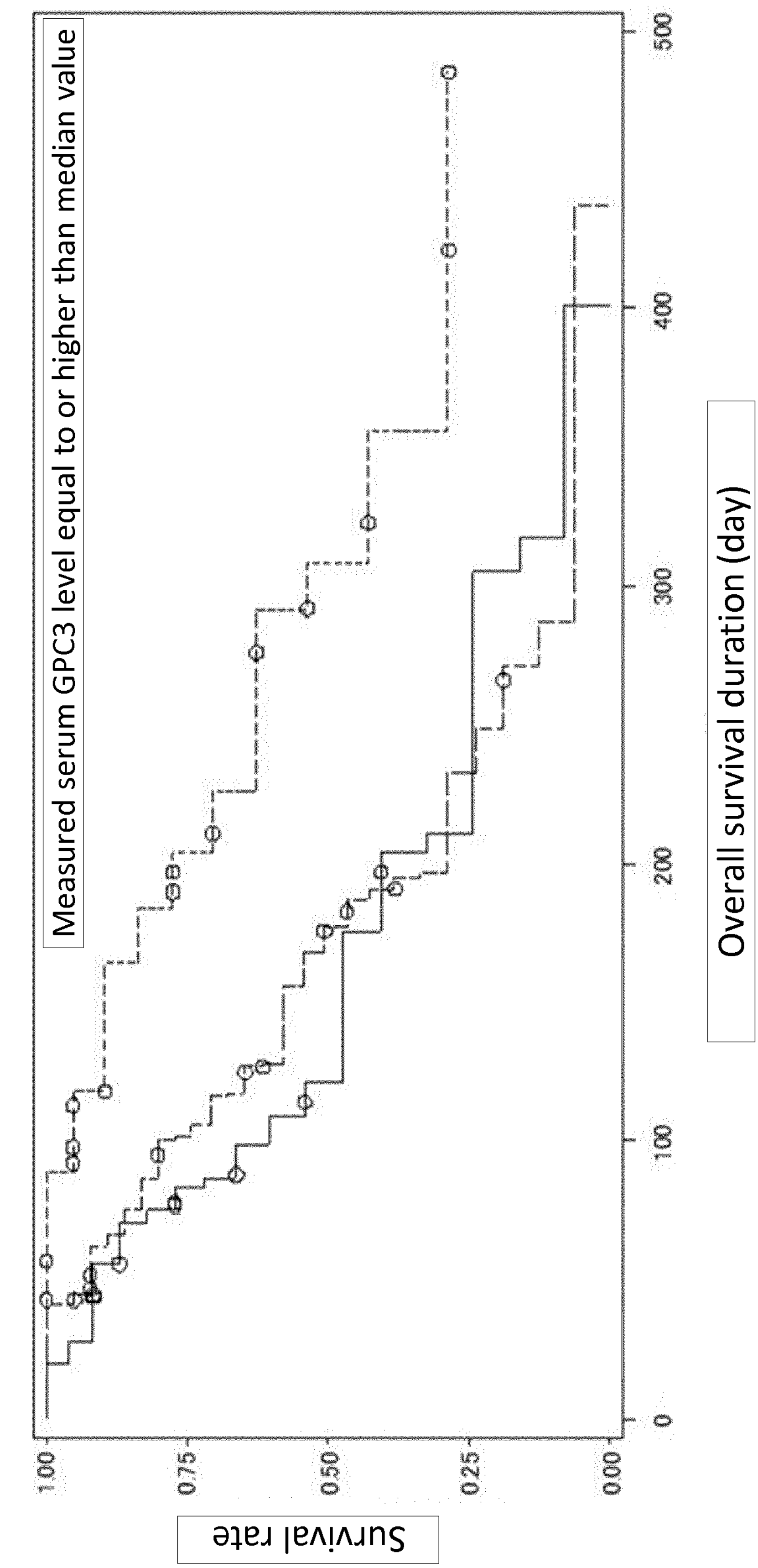
Measured serum GPC3 level equal to or higher than median value
Survival rate
1.00
0.75
0.50
0.25
0.00
0
100
200
300
400
500
Overall survival duration (day)

FIG.8E

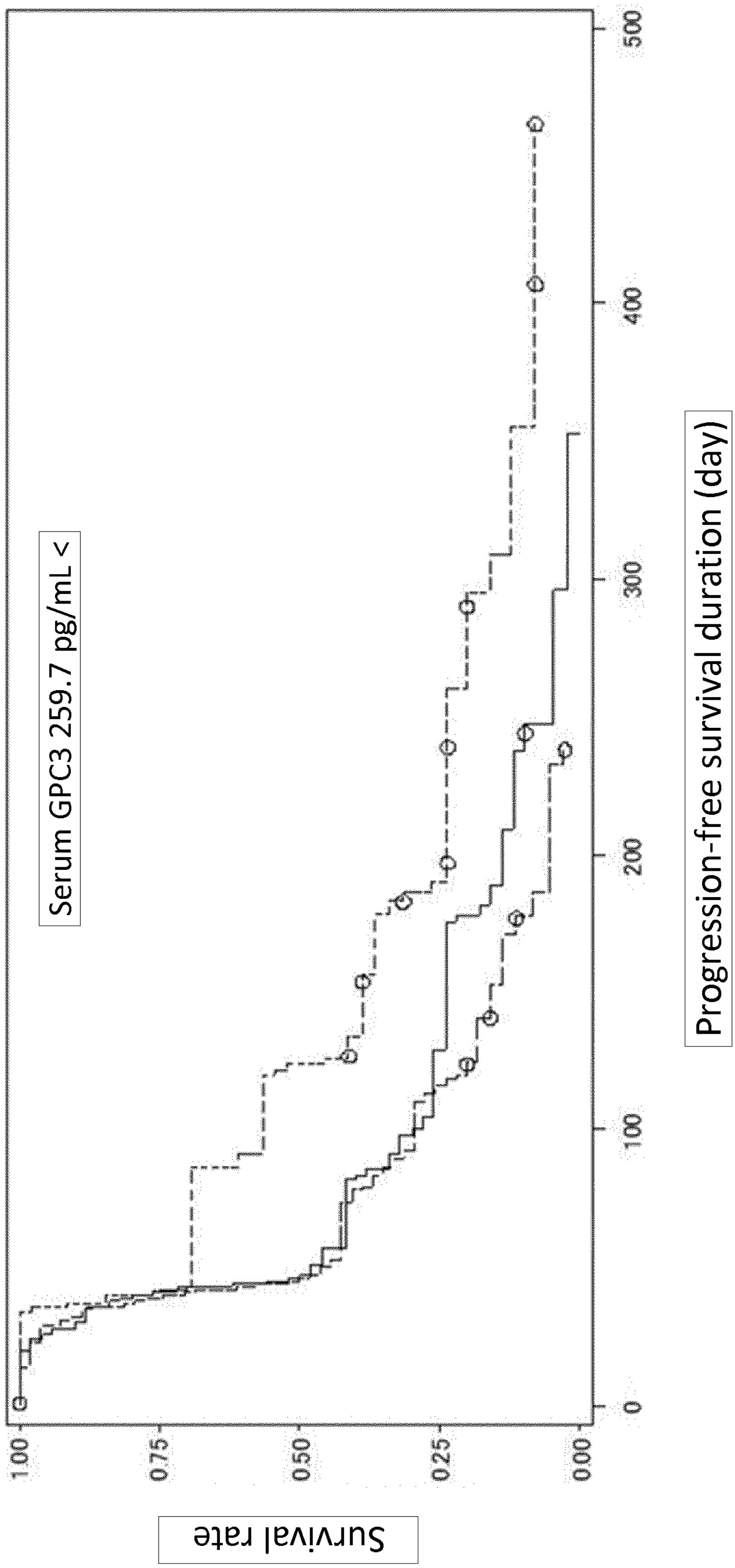
Serum GPC3 259.7 pg/mL <
Survival rate
1.00
0.75
0.50
0.25
0.00
0
100
200
300
400
500
Progression-free survival duration (day)

FIG.8F

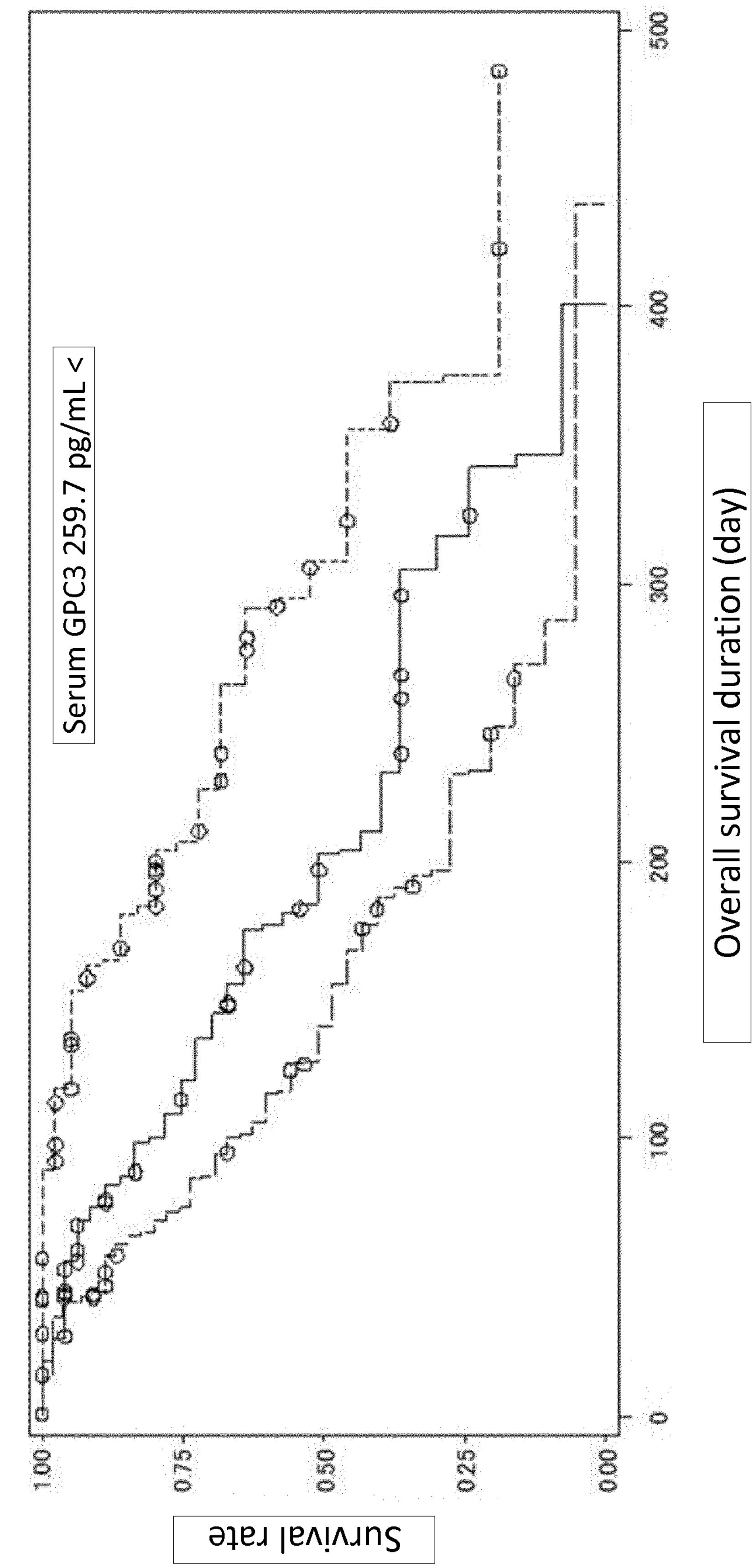
Serum GPC3 259.7 pg/mL <
Survival rate
1.00
0.75
0.50
0.25
0.00
0
100
200
300
400
500
Overall survival duration (day)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2003000883 A
- WO 2006006693 A
- WO 2006046751 A
- WO 2007047291 A
- WO 2009041062 A
- WO 2009122667 A
- WO 2004038420 A
- WO 2009116659 A
- US 20080138827 A1
- JP 2007093274 A
- WO 2012145469 A1
- WO 2008046085 A
- WO 2007137170 A
- WO 2003100429 A
- WO 2009140242 A
- WO 2009086320 A
- WO 2008092117 A
- WO 2007041635 A
- WO 2006105338 A
- WO 2007024249 A
- WO 2007021841 A
- WO 2006031370 A
- WO 2000042072 A
- WO 2004029207 A
- WO 2004099249 A
- WO 2005070963 A
- WO 2006020114 A
- WO 2006116260 A
- WO 2006023403 A
- WO 1999054342 A
- WO 2000061739 A
- WO 2002031140 A
- WO 2006067913 A
- WO 2002079255 A
- WO 2004065540 A
- WO 2004022739 A
- JP 2012280304 A

### Non-patent literature cited in the description

- **LLOVET JM ; BURROUGHS A ; BRUIX J.** *Lancet,* 2003, vol. 362, 1907-17
- **BOSCH FX ; RIBES J ; CLERIES R.** *Gastroenterology,* 2004, vol. 127, 5-16
- **TAKENAKA K ; KAWAHARA N ; YAMAMOTO K ; KAJIYAMA K ; MAEDA T ; ITASAKA H ; SHIRABE K ; NISHIZAKI T ; YANAGA K ; SUGIMACHI K.** *Arch Surg,* 1996, vol. 131, 71-6
- **YEO W ; MOK TS ; ZEE B ; LEUNG TW ; LAI PB ; LAU WY ; KOH J ; MO FK ; YU SC ; CHAN AT.** *J Natl Cancer Inst,* 2005, vol. 97, 1532-8
- **LLOVET J ; RICCI S ; MAZZAFERRO V ; HILGARD P ; GANE E et al.** Sorafenib in advanced hepatocellular carcinoma. *New Eng. J. Med.,* 2008, vol. 359, 378-90
- **CHENG AL ; CHEN Z ; TSAO CJ ; QIN S ; KIM JS et al.** Efficacy and safety of sorefanib in patients in the Asia-Pacific region with advanced hepatocellular carcinoma: a phase III randomized, doubleblind, placebo- controlled trial. *Lancet Oncol,* 2009, vol. 10, 25-34
- **DE CAT B ; MUYLDERMANS S-Y ; COOMANS C ; DEGEEST G ; VANDERSCHUEREN B et al.** Processing by proprotein convertases is required for glypican-3 modulation of cell survival, Wnt signaling, and gastrulation movements. *J. Cell. Biol.,* 2003, vol. 163, 625-635
- **TRAISTER A ; SHI W ; FILMUS J.** Mammalian Notum induces the release of glypicans and other GPI-anchored proteins from the cell surface. *Biochem. J.,* 2008, vol. 410, 503-511
- **MITCHELL et al.** *European Journal of Cancer,* 2010, vol. 47 (3), 333-338
- **KUNKEL et al.** *Proc. Natl. Acad. Sci. USA,* 1985, vol. 82, 488-492
- *Annu. Rev. Biophys. Biomol. Struct.,* 2006, vol. 35, 225-249
- *Proc. Natl. Acad. Sci. U.S.A.,* 2003, vol. 100 (11), 6353-6357
- **KABAT.** Sequences of Proteins of Immunological Interest. National Institute of Health, 1987
- *J. Cell. Biol.,* 2003, vol. 163 (3), 625-635
- **KS. MCCARTY JR. et al.** Use of a monoclonal anti-Estrogen receptor antibody in the immunohistochemical evaluation of human tumors. *Cancer Res. Suppl.,* 1986, vol. 46, 4244s-4248s
- *Cancer Res.,* 2005, vol. 65, 6245-6254

- *Carcinogenesis,* 2008, vol. 29 (7), 1319-1326
- *Int. J. Cancer,* 2003, vol. 103 (4), 455-465
- *Clin. Cancer Res.,* 2006, vol. 12 (9), 2689-2697
- Current protocols in Immunology. **COLIGAN et al.** Immunologic studies in humans. 1993
- **ALLEY et al.** *Curr. Opin. Chem. Biol.,* 2010, vol. 14, 529-537
- **LANGONE et al.** *Methods in Enzymology,* 1983, vol. 93, 307-308
- *Nature Medicine,* 1996, vol. 2, 350-353
- **FULTON et al.** *J. Biol. Chem.,* 1986, vol. 261, 5314-5319
- **SIVAM et al.** *Cancer Res,* 1987, vol. 47, 3169-3173
- **CUMBER et al.** *J. Immunol. Methods,* 1990, vol. 135, 15-24
- **WAWRZYNCZAK et al.** *Cancer Res,* 1990, vol. 50, 7519-7562
- **GHEEITE et al.** *J. Immunol. Methods,* 1991, vol. 142, 223-230
- **THORPE et al.** *Cancer Res,* 1987, vol. 47, 5924-5931
- **WAWRZYNCZAK et al.** *Br. J. Cancer,* 1992, vol. 66, 361-366
- **WAWRZYNCZAK et al.** *Cancer Res.,* 1990, vol. 50, 7519-7562
- **BOLOGNESI et al.** *Clin. exp. Immunol.,* 1992, vol. 89, 341-346
- **STIRPE F. ; BARBIERI L.** *FEBS letter,* 1986, vol. 195, 1-8
- **CASELLAS et al.** *Eur. J. Biochem.,* 1988, vol. 176, 581-588
- *Proc. Natl. Acad. Sci. U.S.A.,* 2006, vol. 103 (11), 4005-4010
- **LAZOR et al.** *Proc. Natl. Acad. Sci. U.S.A.,* 2006, vol. 103 (11), 4005-4010
- *Curr. Opin. Biotechnol.,* 2009, vol. 20 (6), 685-91
- *Curr. Opin. Immunol.,* 2008, vol. 20 (4), 460-470
- *Protein Eng. Des. Sel.,* 2010, vol. 23 (4), 195-202
- **VAUPEL et al.** *Cancer Res,* 1989, vol. 49, 6449-6665
- **WEITZHANDLER et al.** *J. Pharma. Sciences,* 1994, vol. 83 (12), 1670-1675
- **SCHENK et al.** *J. Clin. Investigation,* 2001, vol. 108 (11), 1687-1695
- **BIGGE et al.** *Anal. Biochem.,* 1995, vol. 230 (2), 229-238
- **SATO et al.** *Expert Opin. Biol. Ther.,* 2006, vol. 6 (11), 1161-1173
- **HIPPO et al.** *Cancer Res,* 2004, vol. 64, 2418-2423